# EUROPEAN PATENT APPLICATION

(11) **EP 4 670 793 A1**
(43) Date of publication of application: **31.12.2025**
(21) Application number: 24184026.3
(22) Date of filing: 24.06.2024
(51) Int. Cl.: A61P 37/00, C07K 16/18

(54) **C1Q ANTIGEN-BINDING MOLECULES**

(71) Applicant: Montis Biosciences BV, 3001 Leuven (BE)
(72) Inventor: BENKEIL, Mohammed, 3001 Leuven (BE); CASAZZA, Andrea, 3001 Leuven (BE); DRIEU LA ROCHELLE, Armand, 3001 Leuven (BE)
(74) Representative: Mewburn Ellis LLP

(57) **Abstract**

Antigen binding molecules that bind to C1q are disclosed herein. Also disclosed are compositions comprising such antigen binding molecules, and uses and methods using the same.

## Description

### Technical field

The present disclosure relates to the field of molecular biology, more specifically antibody technology. The present disclosure also relates to methods of medical treatment and prophylaxis.

### Background

C1q is the first recognition subcomponent of the complement classical pathway, which when associated with C1r and C1s, forms a C1 complex, allowing the activation of the complement cascade. The complement cascade plays a vital role in immune regulation, *e.g.* protecting against infections, in support of the innate immune system (Reis et al., Nat Rev Immunol. 2019 Aug;19(8):503-516).

Disruption of complement function, and particularly the function of C1q, has been identified as a key contributing factor in the onset and progression of various diseases, including neurodegenerative and autoimmune diseases, and cancer. In autoimmunity, abnormal C1q expression and bioactivity has been shown to contribute to disease onset and progression. In neurodegenerative disorders, abnormal C1q activity is linked to synaptic pruning though engulfment by microglia (Son etal., Immunol Res. 2015 Dec; 63(1-3): 101-106). In cancer biology, C1q expression is associated with poor prognosis (Winslow et al., Breast Cancer Res. 2015; 17(1): 23).

To date, therapeutic approaches targeting C1q have focused on inhibiting activation of the classical complement cascade.

### Summary

In a first aspect, the present disclosure provides an antigen-binding molecule that binds to C1q for treating or preventing a disease in which M2 macrophages and/or M2-like macrophages are pathologically-implicated.

The present disclosure also provides the use of an antigen-binding molecule that binds to C1q in the manufacture of a medicament for treating or preventing a disease in which M2 macrophages and/or M2-like macrophages are pathologically-implicated.

The present disclosure also provides a method of treating or preventing a disease in which M2 macrophages and/or M2-like macrophages are pathologically-implicated, wherein the method comprises administering to a subject a therapeutically- or prophylactically-effective amount of an antigen-binding molecule that binds to C1q.

In some embodiments in accordance with the various aspects of the present disclosure, method of treating or preventing a disease in which M2 macrophages and/or M2-like macrophages are pathologically-implicated comprises inhibiting polarization of macrophages to an M2 or M2-like phenotype.

In some embodiments, the disease in which M2 macrophages and/or M2-like macrophages are pathologically-implicated is selected from: a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome and multiple sclerosis.

In some embodiments, the antigen-binding molecule inhibits polarization of macrophages to an M2 or M2-like phenotype, inhibits IL-10 production by macrophages and/or inhibits macrophage efferocytosis.

In some embodiments, the antigen-binding molecule does not substantially inhibit activation of the complement cascade.

The present disclosure also provides a chimeric antigen receptor (CAR) comprising an antigen-binding molecule according to the present disclosure.

The present disclosure also provides a nucleic acid, or a plurality of nucleic acids, optionally isolated, encoding an antigen-binding molecule or CAR according to the present disclosure.

The present disclosure also provides an expression vector, or a plurality of expression vectors, comprising a nucleic acid or a plurality of nucleic acids according to the present disclosure.

The present disclosure also provides a cell comprising an antigen-binding molecule, CAR, nucleic acid or a plurality of nucleic acids or expression vector or a plurality of expression vectors according to the present disclosure.

The present disclosure also provides a method comprising culturing a cell according to the present disclosure under conditions suitable for expression of an antigen-binding molecule or CAR by the cell.

The present disclosure also provides an antigen-binding molecule, CAR, nucleic acid or a plurality of nucleic acids, expression vector or a plurality of expression vectors or cell, and a pharmaceutically acceptable carrier, diluent, excipient or adjuvant.

The present disclosure also provides an antigen-binding molecule, CAR, nucleic acid or a plurality of nucleic acids, expression vector or a plurality of expression vectors, cell or composition according to the present disclosure, for use in a method of medical treatment or prophylaxis.

The present disclosure also provides an antigen-binding molecule, CAR, nucleic acid or a plurality of nucleic acids, expression vector or a plurality of expression vectors, cell or composition according to the present disclosure, for treating or preventing: a disease/condition characterized by an increased level of C1q, C1q nephropathy, cryoglobulinemia, rheumatoid arthritis, systemic sclerosis, dermatomyositis, obesity, metabolic syndrome, non-small cell lung cancer, idiopathic pulmonary fibrosis, a neurological disease, Alzheimer's disease, amyotrophic lateral sclerosis, Huntington's disease, multiple sclerosis, Guillain-Barré syndrome, glioma, a disease in which M2 macrophages and/or M2-like macrophages are pathologically-implicated, a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome or multiple sclerosis.

The present disclosure also provides the use of an antigen-binding molecule, CAR, nucleic acid or a plurality of nucleic acids, expression vector or a plurality of expression vectors, cell or composition according to the present disclosure, in the manufacture of a medicament for treating or preventing: a disease/condition characterized by an increased level of C1q, C1q nephropathy, cryoglobulinemia, rheumatoid arthritis, systemic sclerosis, dermatomyositis, obesity, metabolic syndrome, non-small cell lung cancer, idiopathic pulmonary fibrosis, a neurological disease, Alzheimer's disease, amyotrophic lateral sclerosis, Huntington's disease, multiple sclerosis, Guillain-Barré syndrome, glioma, a disease in which M2 macrophages and/or M2-like macrophages are pathologically-implicated, a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome or multiple sclerosis.

The present disclosure also provides a method of treating or preventing a disease/condition characterized by an increased level of C1q, C1q nephropathy, cryoglobulinemia, rheumatoid arthritis, systemic sclerosis, dermatomyositis, obesity, metabolic syndrome, non-small cell lung cancer, idiopathic pulmonary fibrosis, a neurological disease, Alzheimer's disease, amyotrophic lateral sclerosis, Huntington's disease, multiple sclerosis, Guillain-Barré syndrome, glioma, a disease in which M2 macrophages and/or M2-like macrophages are pathologically-implicated, a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome or multiple sclerosis, wherein the method comprises administering to a subject a therapeutically- or prophylactically-effective amount of an antigen-binding molecule, CAR, nucleic acid or a plurality of nucleic acids, expression vector or a plurality of expression vectors, cell or composition according to the present disclosure.

The present disclosure also provides an *in vitro* complex, optionally isolated, comprising an antigen-binding molecule according to the present disclosure bound to C1q.

The present disclosure also provides a method for detecting C1q in a sample, comprising contacting a sample containing, or suspected to contain, C1q with an antigen-binding molecule according to the present disclosure, and detecting the formation of a complex of the antigen-binding molecule with C1q.

The present disclosure also provides the use of an antigen-binding molecule according to the present disclosure as an *in vitro* or *in vivo* diagnostic or prognostic agent.

Other objects and embodiments of the invention will be apparent from the detailed description that follows.

### Description

The present disclosure relates broadly to C1q-binding molecules, and their use in therapeutic and prophylactic applications.

The disclosure is based on the inventors' identification of C1q-binding molecules that inhibit the non-complement-related functions of C1q, enabling the treatment and prevention of diseases in which the non-complement-related functions of C1q are pathologically-implicated.

Specifically, the inventors have identified C1q-binding antibodies capable of: inhibiting C1q-mediated polarization of macrophages to an M2/M2-like phenotype, inhibiting IL-10 secretion by macrophages, inhibiting efferocytosis by macrophages, inhibiting binding of C1q to activated endothelial cells and inhibiting binding of C1q to its receptors (*e.g*., CALR and LAIR1).

Moreover, the inventors identified (i) C1q-binding antibodies that inhibit both non-complement-related and complement-related activities of C1q, and (ii) C1q-binding antibodies that inhibit only non-complement-related activities of C1q (and not complement-related activities). The latter class of antibodies are useful for selectively inhibiting non-complement-related functions of C1q while preserving complement-related activities.

### C1q and its biology

C1q is the first subcomponent of the classical complement pathway and is a 460 kDa polypeptide complex composed of 18 polypeptide chains, comprising 6 heterotrimers. The structure and function of human C1q is described e.g. in Sellar et al. Biochem J. 1991 Mar 1;274 (Pt 2):481-90, and Kouser etal., Front Immunol. 2015; 6: 317, which are hereby incorporated by reference in their entirety. The C1q polypeptide complex comprises a C1q head and a C1q tail. The C1q head and C1q tail enable interaction with multiple binding partners in circulation, and at the cell surface to influence local and systemic immune functions. Where used alone in this specification, 'C1q' refers to the C1q polypeptide complex.

C1q is a polypeptide complex comprising 6 heterotrimers. Each heterotrimer comprises a C1qA polypeptide, a C1qB polypeptide and a C1qC polypeptide. C1qA, C1qB and C1qC polypeptides each comprise: (i) a short N-terminal region, comprising a cysteine through which the polypeptide associates with others via inter-chain disulfide bonds; (ii) a collagen-like region (CLR), comprising ~81 residues: and (iii) a C-terminal globular region, also called the gC1q domain, of ~135 residues (Sellar et al. Biochem J. 1991 Mar 1 ;274 (Pt 2)(Pt 2):481-90).

C1qA, C1qB and C1qC associate through their CLRs to form a triple-helical structural unit having the composition ABC-CBA, which is held together by both covalent and non-covalent interactions between its constituent polypeptides. This subunit structure eventually yields a hexameric C1q molecule comprising the C1q tail and C1q head.

The C1q tail comprises the CLRs of C1qA, C1qB and C1qC, which together form a collagen stem. The C1q head is a globular portion of the C1q polypeptide complex which is formed by interaction of C-terminal globular regions of the C1qA, C1qB and C1qC polypeptides. The C1q head binds to the C1q globular head receptor gC1qR (also named P32, C1qBP, and/or HABP1), and the C1q tail binds cC1qR (also named calreticulin; CALR; CRT).

The C-terminal globular region of C1qA, a C1qB and a C1qC polypeptides is important both for the correct folding and alignment of the triple-helical structural unit and for protein-protein recognition events. There are two well-conserved regions within C-terminal globular region: an aromatic motif is located within the first half of the domain, the other conserved region is located near the C-terminal extremity.

Human C1qA (also known as C1q subcomponent subunit A or C1QA) is the protein identified by UniProt: P02745. The canonical isoform of C1qA has the amino acid sequence shown in SEQ ID NO:1. The canonical isoform of C1qA comprises a signal peptide (SEQ ID NO:2), an N-terminal region (SEQ ID NO:3), a CLR (SEQ ID NO:4), and a C-terminal globular region (SEQ ID NO:5). The mature form of human C1qA is shown in SEQ ID NO:6.

Human C1qB (also known as C1q subcomponent subunit B or C1QB) is the protein identified by UniProt: P02746. The canonical isoform of C1qB has the amino acid sequence shown in SEQ ID NO:7. The canonical isoform of C1qB comprises a signal peptide (SEQ ID NO:8), an N-terminal region (SEQ ID NO:9), two CLRs (SEQ ID NOs:10 and 11), and a C-terminal globular region (SEQ ID NO:12). The mature form of human C1qB is shown in SEQ ID NO:13.

Human C1qC (also known as C1q subcomponent subunit C or C1QC) is the protein identified by UniProt: P02747. The canonical isoform of C1qC has the amino acid sequence shown in SEQ ID NO:14. The canonical isoform of C1qC comprises a signal peptide (SEQ ID NO:15), an N-terminal region (SEQ ID NO:16), a CLR (SEQ ID NO:17), and a C-terminal globular region (SEQ ID NO:18). The mature form of human C1qC is shown in SEQ ID NO:19.

Reference herein to 'C1qA', 'C1qB' and 'C1qC' generally refer, respectively, to the canonical isoforms of the human proteins, but also contemplate isoforms, fragments, variants (including mutants) and homologues thereof (i.e. from other species, *e.g.* non-human mammalian species (*e.g.* a non-human primate, *e.g.* rhesus, cynomolgous; *e.g.* a rodent, *e.g.* rat or mouse).

As used herein, a 'fragment', 'variant' or 'homologue' of a protein may optionally be characterized as having at least 60% amino acid sequence identity, *e.g.* one of ≥70%, ≥75%, ≥80%, ≥85%, ≥86%, ≥87%, ≥88%, ≥89%, ≥90%, ≥91%, ≥92%, ≥93%, ≥94%, ≥95%, ≥96%, ≥97%, ≥98% or ≥99% amino acid sequence identity to the amino acid sequence of the reference protein (*e.g.* the canonical isoform of the human protein). In some embodiments fragments/variants/ isoforms/homologues may be characterized by an ability to perform a function performed by the reference protein.

A 'fragment' generally refers to a fraction of the reference protein. A 'variant' generally refers to a protein having an amino acid sequence comprising one or more amino acid substitutions, insertions, deletions or other modifications relative to the amino acid sequence of the reference protein, but retaining a considerable degree of sequence identity (*e.g.* at least 60%) to the amino acid sequence of the reference protein. An 'isoform' generally refers to a variant of the reference protein expressed by the same species as the species of the reference protein. A 'homologue' generally refers to a variant of the reference protein produced by a different species as compared to the species of the reference protein.

A 'fragment' may be of any length (by number of amino acids), although may optionally be at least 20% of the length of the reference protein (that is, the protein from which the fragment is derived) and may have a maximum length of one of 50%, 75%, 80%, 85%, 90%, 91%, 92%, 93%, 94%, 95%, 96%, 97%, 98%, or 99% of the length of the reference protein.

Isoforms, fragments, variants or homologues may optionally be functional isoforms, fragments, variants or homologues, *e.g.* having a functional property/activity of the reference protein, as determined by analysis by a suitable assay for the functional property/activity.

In some embodiments, the C1qA/C1qB/C1qC is from a mammal (*e.g.* a primate (rhesus, cynomolgous, non-human primate or human) and/or a rodent (*e.g.* rat or murine)). Isoforms, fragments, variants or homologues of C1qA/C1qB/C1qC may optionally be characterized as having at least 60% amino acid sequence identity, *e.g.* one of ≥70%, ≥75%, ≥80%, ≥85%, ≥86%, ≥87%, ≥88%, ≥89%, ≥90%, ≥91 %, ≥92%, ≥93%, ≥94%, ≥95%, ≥96%, ≥97%, ≥98%, ≥99% or 100% amino acid sequence identity to the amino acid sequence of an immature or mature C1qA/C1qB/C1qC isoform from a given species, *e.g.* human.

It will be appreciated that 'C1q' refers to a polypeptide complex formed by association between monomeric C1qA, C1qB and C1qC polypeptides as described herein.

In some embodiments, C1q according to the present disclosure is a polypeptide complex formed by association between: (i) 6 polypeptides, each comprising an amino acid sequence having at least 60% amino acid sequence identity, *e.g.* one of ≥70%, ≥75%, ≥80%, ≥85%, ≥86%, ≥87%, ≥88%, ≥89%, ≥90%, ≥91%, ≥92%, ≥93%, ≥94%, ≥95%, ≥96%, ≥97%, ≥98%, ≥99% or 100% amino acid sequence identity to SEQ ID NO:6; (ii) 6 polypeptides, each comprising an amino acid sequence having at least 60% amino acid sequence identity, *e.g.* one of ≥70%, ≥75%, ≥80%, ≥85%, ≥86%, ≥87%, ≥88%, ≥89%, ≥90%, ≥91%, ≥92%, ≥93%, ≥94%, ≥95%, ≥96%, ≥97%, ≥98%, ≥99% or 100% amino acid sequence identity to SEQ ID NO:13; and (iii) 6 polypeptides, each comprising an amino acid sequence having at least 60% amino acid sequence identity, *e.g.* one of ≥70%, ≥75%, ≥80%, ≥85%, ≥86%, ≥87%, ≥88%, ≥89%, ≥90%, ≥91%, ≥92%, ≥93%, ≥94%, ≥95%, ≥96%, ≥97%, ≥98%, ≥99% or 100% amino acid sequence identity to SEQ ID NO:19.

In some embodiments, C1q according to the present disclosure is a polypeptide complex formed by association between: (i) 6 polypeptides, each consisting of the amino acid of SEQ ID NO:6; (ii) 6 polypeptides, each consisting of the amino acid of SEQ ID NO:13; and (iii) 6 polypeptides, each consisting of the amino acid of SEQ ID NO:19.

C1q is produced by macrophages and immature dendritic cells and released into systemic circulation, and is also produced locally by macrophages. The biology of C1q is complex, with C1q having roles in numerous different cellular processes and biological responses, including the complement cascade, immune modulation, angiogenesis, tissue remodeling and immunologic tolerance. C1q is reported to have both pro- and anti-inflammatory properties, immune-activating and immunosuppressive activities, and has been reported to both enhance and inhibit cell proliferation (and thus promote or attenuate the development of cancers). The various activities of C1q are described in detail *e.g.* in Nayak et al., Innate Immunity (2012)18(2):350-363 and Son etal., Experimental & Molecular Medicine (2022) 54:567-572, both of which are hereby incorporated by reference in their entirety.

The best-understood functions of C1q relate to its roles in the processes of the complement system.

The complement system is a central part of the innate immunity that serves as a first line of defence against foreign and altered host cells. The complement system is activated upon infection with microorganisms to induce inflammation and promote elimination of the pathogens. The complement system is composed of plasma proteins produced mainly by the liver or membrane proteins expressed on cell surface. Complement operates in plasma, in tissues, or within cells. For a review of the complement system, see *e.g.* Merle NS et al., Front Immunol. 2015 Jun 2;6:262, which is hereby incorporated by reference in its entirety. Dysregulation of complement can contribute to inflammatory, immune-related, and age-related conditions. As a result, inappropriate regulation of the complement system has been implicated in a wide variety of diseases in humans *e.g.* diseases of the eye, kidney, neurological diseases and cancer (Morgan, B.P., Semin Immunopathol, 2018. 40(1): p. 113-124; Halbgebauer, R., etal., Semin Immunol, 2018. 37: p. 12-20; Ma, Y., etal., Aging Dis, 2019. 10(2): p. 429-462; and Kleczko, E.K., et al., Front Immunol, 2019. 10: p. 954).

The complement system can be activated via three distinct pathways: the classical pathway (CP), alternative pathway (AP) and lectin binding pathway (LP). The three pathways converge into the generation of a C3 convertase, which cleaves the central complement component C3 into activation products C3b, a large fragment that acts as an opsonin (binds to foreign microorganisms to increase their susceptibility to phagocytosis), and C3a, an anaphylatoxin that promotes inflammation. Along with factor B (FB), C3b forms the C3 convertase (C3bBb) which cleaves further C3 molecules, generates more C3b and C3a, and amplifies C3b deposition on cell surfaces. This is the complement amplification loop.

As well as binding IgG and IgM, C1q may bind other proteins to activate the complement classical complement pathway (Nayak etal., Innate Immunity. 2012;18(2):350-363). C1q is a pattern recognition molecule that binds to structures and ligands on microbial surfaces, apoptotic cells, or indirectly via antibodies and C-reactive protein (CRP), through recognition of charged patterns/clusters. In addition to IgG and IgM, C1q displays binding to *e.g.* short leucine-rich glycoproteins, pentraxins (*e.g.* CRP, SAP, PTX3), bacterial proteins/glycoproteins (*e.g.* phosphatidylserine), β-amyloid, viral proteins (*e.g.* proteins of HIV-1, HTLV-1; e.g. human astrovirus coat protein), fibromodulin, osteoadherin, and chondroadherin.

C1q is the first recognition subcomponent of the classical complement pathway, and interacts with the serine proteases C1s and C1r to form the C1 complex (which is sometimes also referred to as C1qC1r2C1s2). The C1 complex is a large, multimolecular complex which cleaves C4 and C2 to initiate the complement cascade (Zwarthoff *et al.,* 2021, 118 (26) e2102787118). The classical complement pathway is activated upon binding of the C1 complex via the C1q head to the Fc regions of antibodies (complexed with antigens), or upon direct binding to pathogenic factors. Such binding results in a conformational change in the C1 complex, which leads to activation of C1r autocatalytic enzymatic activity. The active form of C1rthen cleaves C1s to generate an active serine protease. C1s cleaves C4 and then C2 to generate two large fragments, C4b and C2b, which together form the C3 convertase of the classical pathway.

While the complement-related activities of C1q are well-studied, other activities of C1q have been investigated less extensively. To date, therapeutic approaches targeting C1q have focused largely on the inhibition of the activation of the classical complement cascade.

C1q also exerts functional effects through interaction with C1q receptors. C1q receptors are a diverse group of cell surface and intracellular proteins that bind to C1q and either initiate or promote various cellular responses. C1q receptors include gC1qR (also known as C1qBP), cC1qR (calreticulin; CALR; CRT), CR1, leukocyte-associated immunoglobulin-like receptor (LAIR)1, LAIR2, discoidin domain receptor (DDR)1 and DDR2.

gC1qR is a multifunctional protein that binds to C1q via the C1q head (see *e.g.* Lei et al., Front Immunol. 2023; 14: 1095943), and is expressed by various different cell types. gC1qR is highly-expressed at the cell surface of activated endothelial cells, and may promote tumor angiogenesis.

cC1qR is a highly-conserved chaperone protein. While it resides primarily in the endoplasmic reticulum, it is expressed at the cell surface of apoptotic cells where binding by C1q 'marks' the apoptotic cell for phagocytosis.

C1q has also been shown to bind to CR1, which is expressed by erythrocytes and leukocytes, among other cell types. CR1 plays an important role in removal of immune complexes and pathogens coated with complement components C3b and C4b.

Leukocyte-associated Ig-like receptor-1 (LAIR1) and its splice variant LAIR2 are expressed by most hematopoietic cells. LAIR1 is an immune inhibitory receptor containing two immunoreceptor tyrosine-based inhibitory motifs (ITIMs). Binding of C1q to LAIR-1 and activation of LAIR-1-mediated signalling inhibits proinflammatory M1-like differentiation and promotes polarization of macrophages to the M2 phenotype (Son et al., Experimental & Molecular Medicine (2022) 54:567-572).

DDR1 and DDR2 are collagen receptors and members of a class of receptor tyrosine kinases (RTKs) characterized by a 155-amino acid discoidin homology domain in their extracellular regions. DDR consists of two types of non-integrin collagen receptors, DDR1 and DDR2, and is activated by receptor-specific collagen binding. DDRs are activated by binding to collagen. It has been reported that C1q binds directly to DDR1, and that this interaction induces the migration and invasion of liver cancer HepG2 cells. C1q has been reported to exhibit sequence and structural similarities with collagen VIII and X and it has been shown the collagen receptors α2β1 and LAIR-1, are also receptors for C1q collagen-like repeat regions. C1q directly activates DDR1 in liver cancer cells and enhances migratory and invasive phenotypes and suggests that the interaction between the two promotes tumor aggressiveness. (Lee et al., Scientific Reports (2018) 8, 4908). Dysregulated DDR function is associated with progression of various human diseases, including fibrosis, arthritis, and cancer (Leitinger. Int Rev Cell Mol Biol. 2014:310:39-87).

### Macrophages

Aspects and embodiments of the present disclosure pertain to the use of the articles of the present disclosure to modulate the phenotype and/or activity of macrophages.

The biology of macrophages and their role in health and disease is reviewed e.g. in Ross et al., Front Immunol. (2021) 12:708186 and Zhang etal., Front Immunol. (2021) 12:620510 and Lendeckel etal. ChemTexts. (2022) 8(2): 12, all of which are hereby incorporated by reference in their entirety.

Macrophages are mature monocytes that play a critical role in the body's defense against infections and tissue repair. They form part of the innate immune system and are found in various tissues, where they detect and engulf pathogens, dead cells, and debris. Macrophages also contribute to the regulation of inflammation and tissue homeostasis. They exhibit remarkable phenotypic plasticity, adapting their function to different microenvironments; M1 macrophages promote inflammation and pathogen elimination, while M2 macrophages facilitate tissue repair and resolution of inflammation. This duality highlights their crucial role in maintaining overall health and immune response balance.

Macrophages are broadly categorized as 'M1' or 'M2' macrophages based on the Th1/Th2 classification system, which distinguishes between two primary macrophage phenotypes: the 'pro-inflammatory' or 'classically-activated' macrophage (M1), and the 'pro-resolving' or 'alternatively-activated' macrophage (M2). Differentiation of macrophages into M1 and M2 phenotypes is linked to the expression of various genes, including those involved in metabolizing arginine. M2 macrophages express arginase (Arg1), leading to the production of ornithine, which supports cell proliferation and tissue repair. By contrast, M1 macrophages express inducible nitric oxide synthase (iNOS), resulting in the production of nitric oxide, which is an important molecule in the innate immune response.

M1 macrophages express pro-inflammatory cytokines such as TNFα, IL-1β, IL-12 and IL-18, and exert cytotoxic activity against infected cells remove pathogens during infection. M1 macrophages also possess advanced antigen-presenting capability for engaging/amplifying the cell-mediated adaptive immune response. High expression of iNOS is accompanied by production of large amounts of NO.

M2 macrophages are characterized by production of anti-inflammatory cytokines such as IL-10 and TGFβ. M2 macrophages play an important role in debris clearance through phagocytic activity (in particular efferocytotic activity) and promote tissue regeneration and repair.

Efferocytosis is reviewed *e.g.* in Doran et al., Nature Reviews Immunology (2020) 20: 254-267, which is hereby incorporated by reference in its entirety. Efferocytosis refers to clearance of apoptotic cells by professional and non-professional phagocytes by phagocytosis, and is important for tissue homeostasis. Defective/aberrant efferocytosis is implicated in the pathology of a variety of diseases. Phagocytosis is a type of endocytosis in which cells internalize (engulf) large particles (>0.5 µm) within a plasmalemma-derived envelope. In efferocytosis, phagocytosis is of apoptotic cells.

Macrophages are highly-adaptable, and can switch between M1 and M2 phenotypes in response to external signals. Signals promoting polarization to the M1 phenotype include Th1 cytokines such as IFNγ and TNFα, and pathogen-associated molecular patterns (PAMPs). Signals promoting polarization to the M1 phenotype include Th2 cytokines such as IL-4 and IL-13.

Markers of M1 macrophages and M2 macrophages are described *e.g.* in Jayasingam etal., Front. Oncol., Sec. Radiation Oncology (2020) 9:2019, which is hereby incorporated by reference in its entirety.

Herein, a macrophage may be characterized by expression (*e.g.* surface expression) of CD14, CD16, CD64, CD68, CD71, CCR5, CD11b and/or CD15.

Herein, a macrophage having an M2 or M2-like phenotype may be characterized by: expression/secretion of IL-10, TGFβ, IL-4, IL-13, CCL1, CCL17, CCL18, CCL22, CCL24, CXCL13 and/or VEGF; expression (*e.g.* surface expression) of CD200R, CD163, CD206, CD209, CD204, CD301, CXCR1, CXCR2, CCR2, Dectin-1, and/or TGM2; expression of Arg1, cMaf, cMyc, MERTK, STAT3, STAT6, IRF4, KLF4, JMJD3, PPARδ and/or PPARγ; and/or phagocytic (*e.g.* efferocytotic) activity. In some embodiments, a macrophage having an M2 or M2-like phenotype may be characterized by expression/secretion of IL-10.

In preferred embodiments, a macrophage having an M2 or M2-like phenotype according to the present disclosure is characterized by: surface expression of CD200R, CD163, CD206, CD209, CD301, Dectin-1, CXCR1, CXCR2, and/or TGM2.

Herein, a macrophage having an M1 or M1-like phenotype may be characterized by: expression/secretion of TNFα, IL-1α, IL-1β, IL-6, IL-12, IL-18, IL-23, CXCL9, CXCL10, CXCL1 1, CXCL16, CCL2 and/or CCL5; expression (*e.g.* surface expression) of MHC Class II (*e.g.* HLA-DR), CD11c, iNOS, TLR4, TLR2, CD32, CD36, CD64, CD86 and/or CD80; expression of NFκB, STAT1, STATS, IRF3, IRF5 and/or HIF1α.

In preferred embodiments, a macrophage having an M1 or M1-like phenotype according to the present disclosure is characterized by: surface expression of CD32, CD64, CD80, CD86, and/or MHC Class II.

M2 macrophages may be further classified into M2a, M2b, M2c and M2d subtypes (see *e.g.* Abdelaziz et al., Journal of Translational Medicine (2020) 18:58). M2a macrophages are involved in tissue repair and wound healing, and promote anti-inflammatory responses for the resolution of inflammation and tissue regeneration. M2b macrophages are associated with dampening excessive inflammation, and help to control immune responses to prevent damage to healthy tissue. Excessive/aberrant M2b macrophage activity can promote infection. M2c macrophages participate in resolving inflammation, suppressing immune responses and promoting tissue remodeling and debris clearance (*e.g.* through efferocytosis) after immune challenge/tissue injury. M2d macrophages are associated with angiogenesis and tumor growth.

Herein, a macrophage having an M2a or M2a-like phenotype may be characterized by: expression/secretion of IL-10 and/or TGFβ; expression (*e.g.* surface expression) of CD206; and/or expression of Arg1. Herein, a macrophage having an M2b or M2b-like phenotype may be characterized by: expression/secretion of IL-10, IL-1β, IL-6 and/or TNFα; and/or expression (*e.g.* surface expression) of CD206. Herein, a macrophage having an M2c or M2c-like phenotype may be characterized by: expression/secretion of IL-10 and/or TGFβ; expression (*e.g.* surface expression) of CD206 and/or CD163; phagocytic (*e.g.* efferocytotic) activity; and/or expression of MERTK. Herein, a macrophage having an M2d or M2d-like phenotype may be characterized by: expression/secretion of IL-10, TGFβ and/or VEGF.

In some embodiments, a macrophage having an M2 or M2-like phenotype according to the present disclosure is a macrophage having an M2c or M2c-like phenotype.

In some embodiments, a macrophage having an M2 or M2-like phenotype according to the present disclosure is a perivascular macrophage. In some embodiments, a macrophage having an M2c or M2c-like phenotype is a perivascular macrophage. In some embodiments, a macrophage characterized by expression/secretion of IL-10 is a perivascular macrophage.

Perivascular macrophages (PVMs) are reviewed *e.g.* in Lapenna et al., Nature Reviews Immunology (2018) 18: 689-702, which is hereby incorporated by reference in its entirety. PVMs lie on, or close to, the outer (abluminal) surface of blood vessels and perform several crucial activities at this interface between the tissue and the blood. PVMs can be found in different tissues such as CNS, lung, kidney, liver, and breast tissue.

PVMs are macrophages located within, or in close proximity to, the perivascular space (also known as the perivascular compartment or Virchow-Robin space). The perivascular space is delimited by the vascular basement membrane on the abluminal side of the vessel wall and by the glia limitans basement membrane on the parenchymal side. Herein, a PVM may be a macrophage located in the perivascular space, or within 15 µm of a blood vessel (*e.g.* an artery, vein, or capillary).

PVMs are localized in close proximity to the endothelial cells in the vasculature. The interplay between endothelial cells (ECs) and perivascular macrophages (PVMs) serves as the vascular immune gatekeeper, responsible for regulating infiltration of immune cells into the adjacent tissue. In steady-state tissues, PVMs maintain tight junctions between endothelial cells and limit vessel permeability, phagocytose potential pathogens before they enter tissues from the blood and limit immune cell infiltration (and thereby inflammation). PVMs have multifaceted roles in diseases such as cancer, Alzheimer disease, multiple sclerosis, and type 1 diabetes, which are reviewed by Lapenna et al. (Nature Reviews Immunology, 18 (11). pp. 689-702), which is hereby incorporated by reference in its entirety.

In some embodiments, a macrophage having an M2 or M2-like phenotype according to the present disclosure is a tumor-associated macrophage. In some embodiments, a macrophage having an M2c or M2c-like phenotype is a tumor-associated macrophage. In some embodiments, a macrophage characterized by expression/secretion of IL-10 is a tumor-associated macrophage.

Tumor-associated macrophages (TAMs) are reviewed *e.g.* in Pitt et al., Nature Reviews Clinical Oncology (2022) 19: 402-421 and Mantovani et al., Nature Reviews Drug Discovery (2022) 21: 799-820 (2022), both of which are hereby incorporated by reference in their entirety. TAMs are macrophages found in solid tumors.

Tumor-associated macrophages are important cellular effectors for establishing and maintaining the immunosuppressive tumor microenvironment (TME), and have a role in the orchestration of angiogenesis, extracellular matrix remodeling, cancer cell proliferation, metastasis and immunosuppression, as well as in resistance to therapeutic intervention for the treatment of cancers.

TAMs can promote tumorigenesis through the production of nitric oxide and reactive oxygen species that contribute to genetic instability, through the production of growth factors such as EGF, through the production of factors that promote the growth and survival of cancer stem cells such as GPNMB, IL-6 and HGF, and promote metastasis through the production of IL-1 and TGFβ. Production of TGFβ and proteases also promotes ECM remodeling and fibrosis, while secretion of IL-1, VEGF and chemokines promotes angiogenesis.

TAMs also promote the development and progression of cancer through immunosuppression. Production of IL-10 and TGFβ promotes the expansion of immunosuppressive Tregs, and skews dendritic cells towards the immature, tolerogenic state. Expression of arginase, prostaglandins and indoleamine 2,3-dioxygenase (IDO) moreover promotes T cell metabolic starvation. TAMs are also characterized by high expression of immune-checkpoint molecules such as PD-L1, PD-L2 and B7-H4 which promote T cell exhaustion (particularly of Th17 cells).

A TAM according to the present disclosure may be a macrophage (*e.g.* a an M2- or M2-like macrophage, *e.g.* an M2c or M2c-like macrophage) located within a solid tumor. A TAM may be characterized by: expression/secretion of IL-10, TGFβ, IL-1, EGF, GPNMB, IL-6, HGF, VEGF, proteases, arginase, IDO, NO and/or prostaglandins; and/or expression (*e.g.* surface expression) of PD-L1, PD-L2 and/or B7-H4.

In some embodiments, a macrophage having an M2 or M2-like phenotype according to the present disclosure is a perivascular tumor-associated macrophage (PvTAM). In some embodiments, a macrophage having an M2c or M2c-like phenotype is a PvTAM. In some embodiments, a macrophage characterized by expression/secretion of IL-10 is a PvTAM.

Perivascular tumor-associated macrophages (PvTAMs) are a highly-specialized, stromal subset of macrophages found within solid tumors in close proximity to the perivascular space. They are both PVMs and TAMs. PvTAMs are described in detail in Bahri et al., Essays Biochem. (2023) 67(6): 919-928, which is hereby incorporated by reference in its entirety.

A PvTAM may be characterized by: expression (*e.g.* surface expression) of TIE2, CD206, FOLR2 and/or LYVE-1; and/or expression of HO-1.

### C1q and macrophage polarization, macrophage activity and vascular dysregulation

The role of C1q in macrophage polarization is reviewed *e.g.* in Bohlson et al., Front Immunol. (2014) 5:402 and Lendeckel et al. (ChemTexts. 2022; 8(2): 12), which are hereby incorporated by reference in their entirety.

C1q has been shown to promote/enhance polarization of macrophages to the M2 phenotype, and to inhibit/reduce polarization of macrophages to the M1 phenotype. Thus, C1q increases the number/proportion of macrophages having the M2 phenotype, and decreases the number/proportion of macrophages having the M1 phenotype.

In Example 1 herein, the inventors demonstrate that expression of genes encoding C1q polypeptides is upregulated in macrophages having an M2 or M2-like phenotype isolated from tissues of various different cancers. This implies the existence of a positive feedback loop, in which the C1q-mediated polarization of macrophages to an M2 or M2-like phenotype promotes the further generation of macrophages having this phenotype.

Example 1 of the present disclosure also demonstrates that the expression of genes encoding C1q polypeptides is upregulated in perivascular macrophages (PVMs) having an M2 or M2-like phenotype, relative to the level of expression by macrophages having an M1 or M1-like phenotype.

C1q has also been shown to enhance/promote efferocytosis by macrophages - see *e.g.* Hulsebus et al., Front Immunol. (2016) 15:7:230 and Pulanco et al., J Immunol. (2017) 198(1): 472-480, which are hereby incorporated by reference in their entirety.

As noted above, C1q directs macrophage polarization towards the generation of M2 macrophages (*e.g.* through binding to LAIR1), which are pro-efferocytotic. C1q also binds to C1q receptors on the surface of apoptotic cells (such as gC1qR and cC1qR), opsonizing them and thereby 'tagging' them for efferocytosis.

Endothelial cells have a barrier function in healthy well-regulated blood vessels. In addition to regulating blood delivery and perfusion, a major function of vascular endothelia, especially those in exchange microvessels (capillaries and postcapillary venules), is to provide a semipermeable barrier that controls blood-tissue exchange of fluids, nutrients, and metabolic wastes while preventing pathogens or harmful materials in the circulation from entering into tissues. This function can be dysregulated in diseased states.

During normal vascular development, macrophages directly interact with, and modulate, the developing vasculature. In adult steady-state tissues, PVMs often have important functions related to their perivascular position, such as the regulation of vascular permeability, the scavenging of blood-borne pathogens and the control of the movement of other leukocytes across the vasculature (Lapenna etal. Nature Reviews Immunology, 18 (11). pp. 689-702).

In some diseases, PVMs acquire an M2 or M2-like phenotype, and ECs show phenotypic and morphological differences, including the loosening of intercellular junctions to neighboring cells (Nagl et al, Frontiers in Cell & Developmental Biology, 2020). This results in compromised vascular immune gatekeeper function, aberrant immune cell filtration, and vascular dysregulation.

In addition to potentiating differentiation of PVMs towards the M2 or M2-like phenotype, C1q binds to gC1 qR expressed by inflamed/activated endothelial cells, and C1q deposits around CD31+ blood vessels, disrupting the vascular immune gatekeeper and lead to increased monocyte adhesion. This may in turn lead to vascular dysregulation and increased tumor immune tolerance.

### C1q-bindinq molecules

The present disclosure provides antigen-binding molecules capable of binding to C1q. An antigen-binding molecule that is capable of binding to C1q may also be described as an antigen-binding molecule that binds to C1q.

An 'antigen-binding molecule' refers to a molecule that binds to a given target antigen. Antigen-binding molecules include antibodies (*i.e.* immunoglobulins (Igs)) and antigen-binding fragments thereof. As used herein, 'antibodies' include monoclonal antibodies, polyclonal antibodies, monospecific and multispecific (*e.g.,* bispecific, trispecific, *etc*.) antibodies, and antibody-derived antigen-binding molecules such as scFv, scFab, diabodies, triabodies, scFv-Fc, minibodies, single domain antibodies (*e.g.* VhH), *etc.* Antigen-binding fragments of antibodies include *e.g.* Fv, Fab, F(ab')2 and F(ab') fragments. In some embodiments, an antigen-binding molecule may be an antibody or an antigen-binding fragment thereof.

Antigen-binding molecules according to the present disclosure also include antibody-derived molecules, *e.g.* molecules comprising an antigen-binding region/domain derived from an antibody. Antibody-derived antigen-binding molecules may comprise an antigen-binding region/domain that comprises, or consists of, the antigen-binding region of an antibody (*e.g.* an antigen-binding fragment of an antibody). In some embodiments, the antigen-binding region/domain of an antibody-derived antigen-binding molecule may be or comprise the Fv (*e.g.* provided as an scFv or dsFv) or the Fab region of an antibody, or the whole antibody. For example, antigen-binding molecules according to the present disclosure include antibody-drug conjugates (ADCs) comprising a (cytotoxic) drug moiety (*e.g.* as described hereinbelow). Antigen-binding molecules according to the present disclosure also include multispecific antigen-binding molecules such as immune cell engager molecules comprising a domain for recruiting (effector) immune cells (reviewed *e.g.* in Goebeler and Bargou, Nat. Rev. Clin. Oncol. (2020) 17: 418-434 and Ellerman, Methods (2019) 154:102-117, both of which are hereby incorporated by reference in their entirety), including BiTEs, BiKEs, and TriKEs. Antigen-binding molecules according to the present disclosure also include chimeric antigen receptors (CARs), which are recombinant receptors providing both antigen-binding and T cell activating functions (CAR structure, function and engineering is reviewed e.g. in Dotti etal., Immunol Rev (2014) 257(1) and Jayaraman et al., EBioMedicine (2020) 58:102931, both of which are hereby incorporated by reference in their entirety).

The antigen-binding molecule of the present disclosure comprises a moiety or moieties capable of binding to a target antigen(s). In some embodiments, the moiety capable of binding to a target antigen comprises an antibody heavy chain variable region (VH) and an antibody light chain variable region (VL) of an antibody capable of specific binding to the target antigen. In some embodiments, the moiety capable of binding to a target antigen comprises or consists of an aptamer capable of binding to the target antigen, *e.g.* a nucleic acid aptamer (reviewed, for example, in Zhou and Rossi Nat Rev Drug Discov. 2017 16(3):181-202). In some embodiments, the moiety capable of binding to a target antigen comprises or consists of an antigen-binding peptide/polypeptide, *e.g.* a peptide aptamer, thioredoxin, monobody, anticalin, Kunitz domain, avimer, knottin, fynomer, atrimer, DARPin, affibody, nanobody (*i.e.* a single-domain antibody (sdAb)), affilin, armadillo repeat protein (ArmRP), OBody or fibronectin - reviewed *e.g.* in Reverdatto et al., Curr Top Med Chem. 2015; 15(12): 1082-1101, which is hereby incorporated by reference in its entirety (see also *e.g.* Boersma etal., J Biol Chem (2011) 286:41273-85 and Emanuel etal., Mabs (2011) 3:38-48).

As used herein, a 'peptide' refers to a chain of two or more amino acid monomers linked by peptide bonds. A peptide typically has a length in the region of about 2 to 50 amino acids. A 'polypeptide' is a polymer chain of two or more peptides. Polypeptides typically have a length greater than about 50 amino acids.

The antigen-binding molecules of the present disclosure generally comprise an antigen-binding domain comprising a VH and a VL of an antibody capable of specific binding to the target antigen. The antigen-binding domain formed by a VH and a VL may also be referred to herein as an Fv region.

An antigen-binding molecule may be, or may comprise, an antigen-binding polypeptide, or an antigen-binding polypeptide complex. An antigen-binding molecule may comprise more than one polypeptide which together form an antigen-binding domain. The polypeptides may associate covalently or non-covalently. In some embodiments, the polypeptides form part of a larger polypeptide comprising the polypeptides (*e.g.* in the case of scFv comprising VH and VL, or in the case of scFab comprising VH-CH1 and VL-CL).

An antigen-binding molecule may refer to a non-covalent or covalent complex of more than one polypeptide (*e.g.* 2, 3, 4, 6, or 8 polypeptides), *e.g.* an IgG-like antigen-binding molecule comprising two heavy chain polypeptides and two light chain polypeptides.

The antigen-binding molecules of the present disclosure may be designed and prepared using the sequences of monoclonal antibodies (mAbs) capable of binding to C1q. Antigen-binding regions of antibodies, such as single chain variable fragment (scFv), Fab and F(ab')2 fragments may also be used/provided. An 'antigen-binding region' is any fragment of an antibody that binds to the target for which the given antibody is specific.

Antibodies generally comprise six complementarity-determining regions CDRs; three in the heavy chain variable (VH) region: HC-CDR1, HC-CDR2 and HC-CDR3, and three in the light chain variable (VL) region: LC-CDR1, LC-CDR2, and LC-CDR3. The six CDRs together define the paratope of the antibody, which is the part of the antibody that binds to the target antigen.

The VH region and VL region comprise framework regions (FRs) either side of each CDR, which provide a scaffold for the CDRs. From N-terminus to C-terminus, VH regions comprise the following structure: N term-[HC-FR1]-[HC-CDR1]-[HC-FR2]-[HC-CDR2]-[HC-FR3]-[HC-CDR3]-[HC-FR4]-C term; and VL regions comprise the following structure: N term-[LC-FR1]-[LC-CDR1]-[LC-FR2]-[LC-CDR2]-[LC-FR3]-[LC-CDR3]-[LC-FR4]-C term.

There are several different conventions for defining antibody CDRs and FRs, such as those described in Kabat et al., Sequences of Proteins of Immunological Interest, 5th Ed. Public Health Service, National Institutes of Health, Bethesda, MD (1991), Chothia et al., J. Mol. Biol. 196:901-917 (1987), and VBASE2, as described in Retter et al., Nucl. Acids Res. (2005) 33 (suppl 1): D671-D674. The CDRs and FRs of the VH regions and VL regions of the antibody clones described herein were defined according to the international IMGT (ImMunoGeneTics) information system (LeFranc etal., Nucleic Acids Res. (2015) 43 (Database issue):D413-22), which uses the IMGT V-DOMAIN numbering rules as described in Lefranc et al., Dev. Comp. Immunol. (2003) 27:55-77. In preferred embodiments, the CDRs and FRs of antigen-binding molecules referred to herein are defined according to the IMGT information system.

In some embodiments, the antigen-binding molecule comprises the CDRs of an antigen-binding molecule that binds to C1q. In some embodiments, the antigen-binding molecule comprises the FRs of an antigen-binding molecule that binds to C1q. In some embodiments, the antigen-binding molecule comprises the CDRs and the FRs of an antigen-binding molecule that binds to C1q. That is, in some embodiments, the antigen-binding molecule comprises the VH region and the VL region of an antigen-binding molecule that binds to C1q.

In some embodiments, the antigen-binding molecule comprises the CDRs, FRs and/or the VH and/or VL regions of a C1q-binding antibody described herein, or CDRs, FRs and/or VH and/or VL regions which are derived from those of a C1q-binding antibody described herein.

In embodiments in accordance with the present disclosure, one or more amino acids are substituted with another amino acid. A substitution comprises substitution of an amino acid residue with a non-identical 'replacement' amino acid residue. A replacement amino acid residue of a substitution according to the present disclosure may be a naturally-occurring amino acid residue (*i.e.* encoded by the genetic code) which is non-identical to the amino acid residue at the relevant position of the equivalent, unsubstituted amino acid sequence, selected from: alanine (Ala), arginine (Arg), asparagine (Asn), aspartic acid (Asp), cysteine (Cys), glutamine (Gln), glutamic acid (Glu), glycine (Gly), histidine (His), isoleucine (Ile): leucine (Leu), lysine (Lys), methionine (Met), phenylalanine (Phe), proline (Pro), serine (Ser), threonine (Thr), tryptophan (Trp), tyrosine (Tyr), and valine (Val). In some embodiments, a replacement amino acid may be a non-naturally-occurring amino acid residue - *i.e.* an amino acid residue other than those recited in the preceding sentence. Examples of non-naturally-occurring amino acid residues include norleucine, ornithine, norvaline, homoserine, aib, and other amino acid residue analogues such as those described in Ellman, et al., Meth. Enzym. 202 (1991) 301-336.

In some embodiments, a substitution may be biochemically conservative. In some embodiments, where an amino acid to be substituted is provided in one of rows 1 to 5 of the table below, the replacement amino acid of the substitution is another, non-identical amino acid provided in the same row:

| **Row** | **Shared property** | **Amino acids** |
|---|---|---|
| 1 | Hydrophobic | Met, Ala, Val, Leu, Ile, Trp, Tyr, Phe, Norleucine |
| 2 | Neutral hydrophilic | Cys, Ser, Thr, Asn, Gln |
| 3 | Acidic or negatively-charged | Asp, Glu |
| 4 | Basic or positively-charged | His, Lys, Arg |
| 5 | Orientation influencing | Gly, Pro |

By way of illustration, in some embodiments wherein substitution is of a Met residue, the replacement amino acid may be selected from Ala, Val, Leu, Ile, Trp, Tyr, Phe and Norleucine.

In some embodiments, a replacement amino acid in a substitution may have the same side chain polarity as the amino acid residue it replaces. In some embodiments, a replacement amino acid in a substitution may have the same side chain charge (at pH 7.4) as the amino acid residue it replaces:

| **Amino Acid** | **Side-chain polarity** | **Side-chain charge (pH 7.4)** |
|---|---|---|
| Alanine | nonpolar | neutral |
| Arginine | basic polar | positive |
| Asparagine | polar | neutral |
| Aspartic acid | acidic polar | negative |
| Cysteine | nonpolar | neutral |
| Glutamic acid | acidic polar | negative |
| Glutamine | polar | neutral |
| Glycine | nonpolar | neutral |
| Histidine | basic polar | positive (10%) |
| | | neutral (90%) |
| Isoleucine | nonpolar | neutral |
| Leucine | nonpolar | neutral |
| Lysine | basic polar | positive |
| Methionine | nonpolar | neutral |
| Phenylalanine | nonpolar | neutral |
| Proline | nonpolar | neutral |
| Serine | polar | neutral |
| Threonine | polar | neutral |
| Tryptophan | nonpolar | neutral |
| Tyrosine | polar | neutral |
| Valine | nonpolar | neutral |

That is, in some embodiments, a nonpolar amino acid is substituted with another, non-identical nonpolar amino acid. In some embodiments, a polar amino acid is substituted with another, non-identical polar amino acid. In some embodiments, an acidic polar amino acid is substituted with another, non-identical acidic polar amino acid. In some embodiments, a basic polar amino acid is substituted with another, non-identical basic polar amino acid. In some embodiments, a neutral amino acid is substituted with another, non-identical neutral amino acid. In some embodiments, a positive amino acid is substituted with another, non-identical positive amino acid. In some embodiments, a negative amino acid is substituted with another, non-identical negative amino acid.

In some embodiments, substitution(s) may be functionally conservative. That is, in some embodiments, the substitution may not affect (or may not substantially affect) one or more functional properties (*e.g.* target binding) of the antigen-binding molecule comprising the substitution as compared to the equivalent unsubstituted molecule.

The VH and VL region of an antigen-binding region of an antibody together constitute the Fv region. In some embodiments, the antigen-binding molecule according to the present disclosure comprises, or consists of, an Fv region that binds to C1q. In some embodiments, the VH and VL regions of the Fv are provided as single polypeptide joined by a linker sequence, i.e. a single chain Fv (scFv). In some embodiments, the VH and VL regions of the Fv are provided as separate polypeptides stabilized by the introduction of one or more cysteine residues for the formation of an interchain disulfide bond, *i.e.* a disulfide stabilized Fv (dsFv).

The VL and light chain constant (CL) region, and the VH region and heavy chain constant 1 (CH1) region of an antigen-binding region of an antibody together constitute the Fab region. In some embodiments, the antigen-binding molecule comprises a Fab region comprising a VH, a CH1, a VL and a CL (*e.g.* Cκ or Cλ). In some embodiments, the Fab region comprises a polypeptide comprising a VH and a CH1 (*e.g*. a VH-CH1 fusion polypeptide), and a polypeptide comprising a VL and a CL (*e.g.* a VL-CL fusion polypeptide). In some embodiments, the Fab region comprises a polypeptide comprising a VH and a CL (*e.g.* a VH-CL fusion polypeptide) and a polypeptide comprising a VL and a CH (*e.g.* a VL-CH1 fusion polypeptide); that is, in some embodiments, the Fab region is a CrossFab region. In some embodiments, the VH, CH1, VL and CL regions of the Fab or CrossFab are provided as single polypeptide joined by linker regions, i.e. as a single chain Fab (scFab) or a single chain CrossFab (scCrossFab).

In some embodiments, the antigen-binding molecule described herein comprises, or consists of, a whole antibody that binds to C1q. As used herein, 'whole antibody' refers to an antibody having a structure which is substantially similar to the structure of an immunoglobulin (Ig). Different kinds of immunoglobulins and their structures are described *e.g.* in Schroeder and Cavacini J Allergy Clin Immunol. (2010) 125(202): S41-S52, which is hereby incorporated by reference in its entirety.

Immunoglobulins of type G (*i.e.* IgG) are -150 kDa glycoproteins comprising two heavy chains and two light chains. From N- to C-terminus, the heavy chains comprise a VH followed by a heavy chain constant region comprising three constant domains (CH1, CH2, and CH3), and similarly the light chains comprise a VL followed by a CL. Depending on the heavy chain, immunoglobulins may be classed as IgG (*e.g.* IgG1, IgG2, IgG3, IgG4), IgA (*e.g.* IgA1, IgA2), IgD, IgE, or IgM. The light chain may be kappa (κ) or lambda (λ).

Herein, a 'CH2 domain' refers to an amino acid sequence corresponding to the CH2 domain of an immunoglobulin (Ig). The CH2 domain is the region of an Ig formed by positions 231 to 340 of the immunoglobulin constant domain, according to the EU numbering system (described in Edelman et al., Proc Natl Acad Sci USA (1969) 63(1): 78-85). A 'CH3 domain' refers to an amino acid sequence corresponding to the CH3 domain of an immunoglobulin (Ig). The CH3 domain is the region of an Ig formed by positions 341 to 447 of the immunoglobulin constant domain, according to the EU numbering system. A 'CH2-CH3 region' refers to an amino acid sequence corresponding to the CH2 and CH3 domains of an immunoglobulin (Ig). The CH2-CH3 region is the region of an Ig formed by positions 231 to 447 of the immunoglobulin constant domain, according to the EU numbering system.

In some embodiments, the antigen-binding molecule described herein comprises, or consists of, an IgG (*e.g.* IgG1, IgG2, IgG3, IgG4), IgA (e*.g.* IgA1, IgA2), IgD, IgE, or IgM that binds to C1q.

In some embodiments, the antigen-binding molecule of the present disclosure comprises one or more regions (*e.g.* CH1, CH2, CH3, *etc.*) of an immunoglobulin heavy chain constant sequence. In some embodiments, the immunoglobulin heavy chain constant sequence is, or is derived from, the heavy chain constant sequence of an IgG (*e.g.* IgG1, IgG2, IgG3, IgG4), IgA (*e.g.* IgA1, IgA2), IgD, IgE or IgM, *e.g.* a human IgG (*e.g.* hlgG1, hIgG2, hIgG3, hIgG4), hlgA (*e.g.* hlgA1, hIgA2), hlgD, hlgE or hlgM. In some embodiments, the immunoglobulin heavy chain constant sequence is, or is derived from, the heavy chain constant sequence of a human IgG1 allotype (*e.g.* G1m1, G1m2, G1m3 or G1m17).

In some embodiments, the antigen-binding molecule comprises an amino acid sequence having at least 60% amino acid sequence identity, *e.g.* one of ≥70%, ≥75%, ≥80%, ≥85%, ≥86%, ≥87%, ≥88%, ≥89%, ≥90%, ≥91 %, ≥92%, ≥93%, ≥94%, ≥95%, ≥96%, ≥97%, ≥98%, ≥99% or 100%, sequence identity to the amino acid sequence of SEQ ID NO:28 or 29.

In some embodiments, the antigen-binding molecule comprises an amino acid sequence having at least 60% amino acid sequence identity, *e.g.* one of ≥70%, ≥75%, ≥80%, ≥85%, ≥86%, ≥87%, ≥88%, ≥89%, ≥90%, ≥91%, ≥92%, ≥93%, ≥94%, ≥95%, ≥96%, ≥97%, ≥98%, ≥99% or 100%, sequence identity to the amino acid sequence of SEQ ID NO:20 or 25.

It will be appreciated that CH2 and/or CH3 regions may be provided with further substitutions in accordance with modification to an Fc region of the antigen-binding molecule as described herein.

In some embodiments, the antigen-binding molecule of the present disclosure comprises one or more regions of an immunoglobulin light chain constant sequence. In some embodiments, the immunoglobulin light chain constant sequence is human immunoglobulin kappa constant (IGKC; Cκ). In some embodiments, the immunoglobulin light chain constant sequence is a human immunoglobulin lambda constant (IGLC; Cλ), *e.g.* IGLC1, IGLC2, IGLC3, IGLC6 or IGLC7.

In some embodiments, the antigen-binding molecule comprises an amino acid sequence having at least 70% sequence identity more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:30, 31, 32, 33, 34 or 35. In preferred embodiments, the antigen-binding molecule comprises an amino acid sequence having at least 70% sequence identity more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:30.

In some embodiments, the antigen-binding molecule is or comprises a monoclonal antibody, or an antigen-binding fragment thereof.

In some embodiments, the antigen-binding molecule is or comprises a fully human antibody/antibody fragment. A fully human antibody/antibody fragment may be encoded by human nucleic acid sequence(s). A fully human antibody/antibody fragment may be devoid of non-human amino acid sequences. Commonly employed techniques for the production of fully human antibodies include (i) phage display, in which human antibody genes are expressed in phage display libraries, and (ii) production of antibodies in transgenic mice engineered to have human antibody genes (described in Park and Smolen, Advances in Protein Chemistry (2001) 56: 369-421). Briefly, in the human antibody gene-phage display technique, genes encoding the VH and VL chains are generated by PCR amplification and cloning from 'naive' human lymphocytes, and assembled into a library from which they can be expressed either as disulfide-linked Fab fragments or as single-chain Fv (scFv) fragments. The Fab- or scFv-encoding genes are fused to a surface coat protein of filamentous bacteriophage and Fab or scFv capable of binding to the target of interest can then be identified by screening the library with antigen. Molecular evolution or affinity maturation procedures can be employed to enhance the affinity of the Fab/scFv fragment. In the transgenic mouse technique, mice in which the endogenous murine Ig gene loci have been replaced by homologous recombination with their human homologues are immunized with antigen, and monoclonal antibody is prepared by conventional hybridoma technology, to yield a fully human monoclonal antibody.

In some embodiments, the antigen-binding molecule of the present disclosure is a mouse antibody/antibody fragment. In some embodiments, the antibody/antibody fragment is obtained from phage display using a human naive antibody gene library.

In some embodiments, the antigen-binding molecule is a mouse/human chimeric antigen-binding molecule (*i.e.* an antigen-binding molecule comprising mouse antibody variable domains and human antibody constant regions). In some embodiments, the antigen-binding molecule is a humanized antigen-binding molecule (*i.e.* an antigen-binding molecule comprising variable domains derived by humanization of the variable domains of an antibody from a non-human animal, *e.g.* a mouse) comprising mouse antibody variable domains and human antibody constant regions. In some embodiments, the antigen-binding molecule comprises mouse antibody CDRs and human antibody framework and constant regions.

Mouse/human chimeric antigen-binding molecules can be prepared from mouse antibodies by the process of chimerisation, *e.g.* as described in Human Monoclonal Antibodies: Methods and Protocols, Michael Steinitz (Editor), Methods in Molecular Biology 1060, Springer Protocols, Humana Press (2014), in Chapter 8 thereof, in particular section 3 of Chapter 8.

Humanized antigen-binding molecules can be prepared from mouse antibodies by the process of humanization, *e.g.* as described in Human Monoclonal Antibodies: Methods and Protocols, Michael Steinitz (Editor), Methods in Molecular Biology 1060, Springer Protocols, Humana Press (2014), in Chapter 7 thereof, in particular section 3.1 of Chapter 7 entitled 'Antibody Humanization'. Techniques for antibody humanization are also described *e.g.* in Safdari et al., Biotechnol Genet Eng Rev (2013) 29:175-86.

Aspects of the present disclosure relate to multispecific antigen-binding molecules. By 'multispecific' it is meant that the antigen-binding molecule displays specific binding to more than one target. In some embodiments, the antigen-binding molecule is a bispecific antigen-binding molecule. In some embodiments, the antigen-binding molecule comprises at least two different antigen-binding domains (*i.e.* at least two antigen-binding domains, *e.g.* comprising non-identical VHs and VLs).

In some embodiments, the antigen-binding molecule binds to C1q and another target (*e.g.* an antigen other than C1q), and so is at least bispecific. The term 'bispecific' means that the antigen-binding molecule is able to bind specifically to at least two distinct antigenic determinants.

It will be appreciated that an antigen-binding molecule according to the present disclosure (*e.g.* a multispecific antigen-binding molecule) may comprise antigen-binding molecules capable of binding to the targets for which the antigen-binding molecule is specific. For example, an antigen-binding molecule that binds to C1q and an antigen other than C1q may comprise: (i) an antigen-binding molecule that binds to C1q, and (ii) an antigen-binding molecule that binds to an antigen other than C1q.

It will also be appreciated that an antigen-binding molecule according to the present disclosure (*e.g.* a multispecific antigen-binding molecule) may comprise antigen-binding polypeptides or antigen-binding polypeptide complexes capable of binding to the targets for which the antigen-binding molecule is specific.

In some embodiments, a component antigen-binding molecule of a larger antigen-binding molecule (*e.g.* a multispecific antigen-binding molecule) may be referred to *e.g.* as an 'antigen-binding domain' or'antigen-binding region' of the larger antigen-binding molecule.

In some embodiments, the antigen other than C1q in a multispecific antigen-binding molecule is an immune cell surface molecule. In some embodiments, the antigen is a cancer cell antigen. In some embodiments, the antigen is a receptor molecule, *e.g.* a cell surface receptor. In some embodiments, the antigen is a cell signalling molecule, *e.g.* a cytokine, chemokine, interferon, interleukin or lymphokine. In some embodiments, the antigen is a growth factor or a hormone.

In some embodiments, the antigen-binding molecule is an immune cell engager. Immune cell engagers are reviewed *e.g.* in Goebeler and Bargou, Nat. Rev. Clin. Oncol. (2020) 17: 418-434 and Ellerman, Methods (2019) 154:102-117, both of which are hereby incorporated by reference in their entirety. Immune cell engager molecules comprise an antigen-binding region for a target antigen of interest, and an antigen-binding region for recruiting/engaging an immune cell of interest. Immune cell engagers recruit/engage immune cells through an antigen-binding region specific for an immune cell surface molecule.

The best studied immune cell engagers are bispecific T cell engagers (BiTEs), which comprise a target antigen binding domain, and a CD3 polypeptide (typically CD3ε)-binding domain, through which the BiTE recruits T cells. Binding of the BiTE to its target antigen and to the CD3 polypeptide expressed by the T cell results in activation of the T cell, and ultimately directs T cell effector activity against cells expressing the target antigen. Other kinds of immune cell engagers are well known in the art, and include natural killer cell engagers such as bispecific killer engagers (BiKEs), which recruit and activate NK cells.

In some embodiments, the immune cell engaged by the immune cell engager is a T cell or an NK cell. In some embodiments, the immune cell engager is a T cell-engager.

Multispecific antigen-binding molecules according to the present disclosure may be provided in any suitable format, such as those formats described in described in Brinkmann and Kontermann, MAbs (2017) 9(2): 182-212, which is hereby incorporated by reference in its entirety. Suitable formats include those shown in Figure 2 of Brinkmann and Kontermann, MAbs (2017) 9(2): 182-212: antibody conjugates, *e.g.* IgG2, F(ab')2 or CovX-Body; IgG or IgG-like molecules, *e.g.* IgG, chimeric IgG, κλ-body common HC; CH1/CL fusion proteins, *e.g.* scFv2-CH1/CL, VHH2-CH1/CL; 'variable domain only' bispecific antigen-binding molecules, *e.g.* tandem scFv (taFV), triplebodies, diabodies (Db), dsDb, Db(kih), DART, scDB, dsFv-dsFv, tandAbs, triple heads, tandem dAb/VHH, tertravalent dAb.VHH; Non-Ig fusion proteins, *e.g.* scFv2-albumin, scDb-albumin, taFv-albumin, taFv-toxin, miniantibody, DNL-Fab2, DNL-Fab2-scFv, DNL-Fab2-lgG-cytokine2, ImmTAC (TCR-scFv); modified Fc and CH3 fusion proteins, *e.g.* scFv-Fc(kih), scFv-Fc(CH3 charge pairs), scFv-Fc (EW-RVT), scFv-fc (HA-TF), scFv-Fc (SEEDbody), taFv-Fc(kih), scFv-Fc(kih)-Fv, Fab-Fc(kih)-scFv, Fab-scFv-Fc(kih), Fab-scFv-Fc(BEAT), Fab-scFv-Fc (SEEDbody), DART-Fc, scFv-CH3(kih), TriFabs; Fc fusions, *e.g.* Di-diabody, scDb-Fc, taFv-Fc, scFv-Fc-scFv, HCAb-VHH, Fab-scFv-Fc, scFv4-Ig, scFv2-Fcab; CH3 fusions, *e.g.* Dia-diabody, scDb-CH3; IgE/IgM CH2 fusions, e.g. scFv-EHD2-scFv, scFvMHD2-scFv; Fab fusion proteins, *e.g.* Fab-scFv (bibody), Fab-scFv2 (tribody), Fab-Fv, Fab-dsFv, Fab-VHH, orthogonal Fab-Fab; non-lg fusion proteins, *e.g.* DNL-Fab3, DNL-Fab2-scFv, DNL-Fab2-IgG-cytokine2; asymmetric IgG or IgG-like molecules, *e.g.* IgG(kih), IgG(kih) common LC, ZW1 IgG common LC, Biclonics common LC, CrossMab, CrossMab(kih), scFab-IgG(kih), Fab-scFab-IgG(kih), orthogonal Fab IgG(kih), DuetMab, CH3 charge pairs + CH1/CL charge pairs, hinge/CH3 charge pairs, SEED-body, Duobody, four-in-one-CrossMab(kih), LUZ-Y common LC; LUZ-Y scFab-IgG, FcFc*; appended and Fc-modified IgGs, *e.g.* IgG(kih)-Fv, IgG HA-TF-Fv, IgG(kih)scFab, scFab-Fc(kih)-scFv2, scFab-Fc(kih)-scFv, half DVD-Ig, DVI-Ig (four-in-one), CrossMab-Fab; modified Fc and CH3 fusion proteins, *e.g.* Fab-Fc(kih)-scFv, Fab-scFv-Fc(kih), Fab-scFv-Fc(BEAT), Fab-scFv-Fc-SEEDbody, TriFab; appended IgGs - HC fusions, *e.g.* IgG-HC, scFv, IgG-dAb, IgG-taFV, IgG-CrossFab, IgG-orthogonal Fab, IgG-(CαCβ) Fab, scFv-HC-IgG, tandem Fab-IgG (orthogonal Fab), Fab-IgG(CαCβ Fab), Fab-IgG(CR3), Fab-hinge-IgG(CR3); appended IgGs - LC fusions, *e.g.* IgG-scFv(LC), scFv(LC)-lgG, dAb-IgG; appended IgGs - HC and LC fusions, *e.g.* DVD-Ig, TVD-Ig, CODV-Ig, scFv4-IgG, Zybody; Fc fusions, *e.g.* Fab-scFv-Fc, scFv4-Ig; F(ab')2 fusions, *e.g.* F(ab')2-scFv2; CH1/CL fusion proteins *e.g.* scFv2-CH1-hinge/CL; modified IgGs, *e.g.* DAF (two-in one-IgG), DutaMab, Mab2; and non-lg fusions, *e.g.* DNL-Fab4-lgG. The skilled person is readily able to design and produce multispecific antigen-binding molecules.

The present disclosure also provides chimeric antigen receptors (CARs). CARs are recombinant receptors that provide both antigen-binding and T cell activating functions. CAR structure and engineering is reviewed, for example, in Dotti et al., Immunol Rev (2014) 257(1), hereby incorporated by reference in its entirety. CARs comprise an antigen-binding region linked to a cell membrane anchor region and a signalling region. An optional hinge region may provide separation between the antigen-binding region and cell membrane anchor region, and may act as a flexible linker.

The antigen-binding domain of a CAR according to the present disclosure comprises or consists of an antigen-binding molecule that binds to C1q as described herein. Accordingly, a CAR according to the present disclosure comprises an antigen-binding molecule as described herein.

It will be appreciated that an antigen-binding molecule according to the present disclosure forms, or is comprised in, the antigen-binding domain of the CAR. Accordingly, in some embodiments, the antigen-binding molecule of the present disclosure is comprised in a CAR. It will also be appreciated that an antigen-binding molecule according to the present disclosure may be a CAR. A CAR having an antigen-binding domain comprising or consisting of an antigen-binding molecule of the present disclosure (*e.g.* a C1q-binding Fv) is an antigen-binding molecule. The antigen-binding domain of the CAR of the present disclosure may be provided with any suitable format, *e.g.* scFv, scFab, *etc.*

The cell membrane anchor region is provided between the antigen-binding region and the signalling region of the CAR and provides for anchoring the CAR to the cell membrane of a cell expressing a CAR, with the antigen-binding region in the extracellular space, and signalling region inside the cell. In some embodiments, the CAR comprises a cell membrane anchor region comprising or consisting of an amino acid sequence which comprises, consists of, or is derived from, the transmembrane region amino acid sequence for one of CD3-ζ, CD4, CD8 or CD28. As used herein, a region which is 'derived from' a reference amino acid sequence comprises an amino acid sequence having at least 60%, *e.g.* one of at least 65%, 70%, 75%, 80%, 85%, 90%, 91%, 92%, 93%, 94%, 95%, 96%, 97%, 98%, 99% or 100% sequence identity to the reference sequence.

The signalling region of a CAR allows for activation of the T cell. The CAR signalling regions may comprise the amino acid sequence of the intracellular domain of CD3-ζ, which provides immunoreceptor tyrosine-based activation motifs (ITAMs) for phosphorylation and activation of the CAR-expressing T cell. Signalling regions comprising sequences of other ITAM-containing proteins such as FcγRl have also been employed in CARs (Haynes etal., 2001 J Immunol 166(1):182-187). Signalling regions of CARs may also comprise co-stimulatory sequences derived from the signalling region of co-stimulatory molecules, to facilitate activation of CAR-expressing T cells upon binding to the target protein. Suitable co-stimulatory molecules include CD28, OX40, 4-1BB, ICOS and CD27. In some cases CARs are engineered to provide for co-stimulation of different intracellular signalling pathways. For example, signalling associated with CD28 costimulation preferentially activates the phosphatidylinositol 3-kinase (PI3K) pathway, whereas the 4-1BB-mediated signalling is through TNF receptor associated factor (TRAF) adaptor proteins. Signalling regions of CARs therefore sometimes contain co-stimulatory sequences derived from signalling regions of more than one co-stimulatory molecule. In some embodiments, the CAR of the present disclosure comprises one or more co-stimulatory sequences comprising or consisting of an amino acid sequence which comprises, consists of, or is derived from, the amino acid sequence of the intracellular domain of one or more of CD28, OX40, 4-1BB, ICOS and CD27.

An optional hinge region may provide separation between the antigen-binding domain and the transmembrane domain, and may act as a flexible linker. Hinge regions may be derived from IgG1 or IgG4. In some embodiments, the CAR of the present disclosure comprises a hinge region comprising or consisting of an amino acid sequence which comprises, consists of, or is derived from, the amino acid sequence of the hinge region of IgG1 or IgG4.

Also provided is a cell comprising a CAR according to the present disclosure. The CAR according to the present disclosure may be used to generate CAR-expressing immune cells, *e.g.* CAR-T or CAR-NK cells. Engineering of CARs into immune cells may be performed during culture, *in vitro.*

### Fc regions

In some embodiments, the antigen-binding molecules of the present disclosure comprise an Fc region.

As used herein, an 'Fc region' refers to a polypeptide complex formed by interaction between two polypeptides, each polypeptide comprising the CH2-CH3 region of an immunoglobulin (Ig) heavy chain constant sequence.

Herein, a 'CH2 region' refers to an amino acid sequence corresponding to the CH2 region of an immunoglobulin (Ig). The CH2 region is the region of an Ig formed by positions 231 to 340 of the immunoglobulin constant region, according to the EU numbering system described in Edelman et al., Proc Natl Acad Sci USA (1969) 63(1): 78-85. A 'CH3 region' refers to an amino acid sequence corresponding to the CH3 region of an immunoglobulin (Ig). The CH3 region is the region of an Ig formed by positions 341 to 447 of the immunoglobulin constant region, according to the EU numbering system described in Edelman et al., Proc Natl Acad Sci USA (1969) 63(1): 78-85. A 'CH2-CH3 region' refers to an amino acid sequence corresponding to the CH2 and CH3 regions of an immunoglobulin (Ig). The CH2-CH3 region is the region of an Ig formed by positions 231 to 447 of the immunoglobulin constant region, according to the EU numbering system described in Edelman et al., Proc Natl Acad Sci USA (1969) 63(1): 78-85.

In some embodiments, a CH2 region, CH3 region and/or a CH2-CH3 region according to the present disclosure corresponds to the CH2 region/CH3 region/CH2-CH3 region of an IgG (*e.g.* IgG1, IgG2, IgG3, IgG4), IgA (*e.g.* IgA1, IgA2), IgD, IgE or IgM. In some embodiments, the CH2 region, CH3 region and/or a CH2-CH3 region corresponds to the CH2 region/CH3 region/CH2-CH3 region of a human IgG (*e.g.* hlgG1, hIgG2, hIgG3, hIgG4), hlgA (*e.g.* hlgA1, hIgA2), hlgD, hlgE or hlgM. In some embodiments, the CH2 region, CH3 region and/or a CH2-CH3 region corresponds to the CH2 region/CH3 region/CH2-CH3 region of a human IgG1 allotype (*e.g.* G1m1, G1m2, G1m3 or G1m17). Fc regions provide for interaction with Fc receptors and other molecules of the immune system to bring about functional effects. Fc-mediated effector functions are reviewed *e.g.* in Jefferis et al., Immunol Rev 1998 163:59-76 (hereby incorporated by reference in its entirety), and are brought about through Fc-mediated recruitment and activation of immune cells (*e.g.* macrophages, dendritic cells, neutrophils, basophils, eosinophils, platelets, mast cells, NK cells and T cells) through interaction between the Fc region and Fc receptors expressed by the immune cells, recruitment of complement pathway components through binding of the Fc region to complement protein C1q, and consequent activation of the complement cascade. Fc-mediated functions include Fc receptor binding, antibody-dependent cellular cytotoxicity (ADCC), antibody-dependent cell-mediated phagocytosis (ADCP), complement-related cytotoxicity (CDC), formation of the membrane attack complex (MAC), cell degranulation, cytokine and/or chemokine production, and antigen processing and presentation.

Modifications to antibody Fc regions that influence Fc-mediated functions are known in the art, such as those described *e.g.* in Wang et al., Protein Cell (2018) 9(1):63-73, which is hereby incorporated by reference in its entirety. Exemplary Fc region modifications known to influence antibody effector function are summarized in Table 1 of Wang et al., Protein Cell (2018) 9(1):63-73. In some embodiments, the antigen-binding molecule of the present disclosure comprises an Fc region comprising modification to increase or reduce an Fc-mediated function as compared to an antigen-binding molecule comprising the corresponding unmodified Fc region.

Where an Fc region/CH2/CH3 is described as comprising modification(s) 'corresponding to' reference substitution(s), equivalent substitution(s) in the homologous Fc/CH2/CH3 are contemplated. By way of illustration, L234A/L235A substitutions in human IgG1 (numbered according to the EU numbering system as described in Kabat et al., Sequences of Proteins of Immunological Interest, 5th Ed. Public Health Service, National Institutes of Health, Bethesda, MD, 1991) correspond to L to A substitutions at positions 117 and 118 of the mouse Ig gamma-2A chain C region (UniProtKB: P01863-1, v1).

Where an Fc region is described as comprising a modification, the modification may be present in one or both of the polypeptide chains which together form the Fc region.

In some embodiments, the antigen-binding molecule of the present disclosure comprises an Fc region comprising modification. In some embodiments, the antigen-binding molecule of the present disclosure comprises an Fc region comprising modification in one or more of the CH2 and/or CH3 regions.

In some embodiments, the Fc region comprises modification to increase an Fc-mediated function. In some embodiments, the Fc region comprises modification to increase ADCC. In some embodiments, the Fc region comprises modification to increase ADCP. In some embodiments, the Fc region comprises modification to increase CDC. An antigen-binding molecule comprising an Fc region comprising modification to increase an Fc-mediated function (*e.g.* ADCC, ADCP, CDC) induces an increased level of the relevant effector function as compared to an antigen-binding molecule comprising the corresponding unmodified Fc region.

In some embodiments, the Fc region comprises modification to increase binding to an Fc receptor. In some embodiments, the Fc region comprises modification to increase binding to an Fcγ receptor. In some embodiments, the Fc region comprises modification to increase binding to one or more of FcγRI, FcγRIIa, FcγRIIb, FcγRIIc, FcγRIIIa and FcγRIIIb. In some embodiments, the Fc region comprises modification to increase binding to FcγRIIIa. In some embodiments, the Fc region comprises modification to increase binding to FcγRIIa. In some embodiments, the Fc region comprises modification to increase binding to FcγRIIb. In some embodiments, the Fc region comprises modification to increase binding to FcRn. In some embodiments, the Fc region comprises modification to increase binding to a complement protein. In some embodiments, the Fc region comprises modification to increase binding to C1q. In some embodiments, the Fc region comprises modification to promote hexamerisation of the antigen-binding molecule. In some embodiments, the Fc region comprises modification to increase antigen-binding molecule half-life. In some embodiments, the Fc region comprises modification to increase co-engagement.

In some embodiments, the Fc region comprises modification corresponding to the combination of substitutions F243L/R292P/Y300L/V305I/P396L as described in Stavenhagen et al. Cancer Res. (2007) 67:8882-8890. In some embodiments, the Fc region comprises modification corresponding to the combination of substitutions S239D/I332E or S239D/I332E/A330L as described in Lazar et al., Proc Natl Acad Sci USA. (2006)103:4005-4010. In some embodiments, the Fc region comprises modification corresponding to the combination of substitutions S298A/E333A/K334A as described in Shields et al., J Biol Chem. (2001) 276:6591-6604. In some embodiments, the Fc region comprises modification to one of heavy chain polypeptides corresponding to the combination of substitutions L234Y/L235Q/G236W/S239M/H268D/D270E/S298A, and modification to the other heavy chain polypeptide corresponding to the combination of substitutions D270E/K326D/A330M/K334E, as described in Mimoto etal., MAbs. (2013): 5:229-236. In some embodiments, the Fc region comprises modification corresponding to the combination of substitutions G236A/S239D/I332E as described in Richards et al., Mol Cancer Ther. (2008) 7:2517-2527.

In some embodiments, the Fc region comprises modification corresponding to the combination of substitutions K326W/E333S as described in Idusogie et al. J Immunol. (2001) 166(4):2571-5. In some embodiments, the Fc region comprises modification corresponding to the combination of substitutions S267E/H268F/S324T as described in Moore et al. MAbs. (2010) 2(2):181-9. In some embodiments, the Fc region comprises modification corresponding to the combination of substitutions described in Natsume et al., Cancer Res. (2008) 68(10):3863-72. In some embodiments, the Fc region comprises modification corresponding to the combination of substitutions E345R/E430G/S440Y as described in Diebolder et al. Science (2014) 343(6176):1260-3.

In some embodiments, the Fc region comprises modification corresponding to the combination of substitutions M252Y/S254T/T256E as described in Dall'Acqua et al. J Immunol. (2002) 169:5171-5180.

In some embodiments, the Fc region comprises a CH2-CH3 region comprising an amino acid difference at one or more of the following positions, relative to the amino acid sequence of a CH2-CH3 region of a reference Fc region: 252, 254 or 256 (according to the EU numbering system). In some embodiments, the Fc region comprises a CH2-CH3 region comprising one or more of the following specified amino acid residues: Y252, T254 or E256 (according to the EU numbering system). In some embodiments, the Fc region comprises a CH2-CH3 region comprising Y252, T254 and E256. In some embodiments, the Fc region comprises a CH2-CH3 region comprising one or more of the following amino acid substitutions, relative to the amino acid sequence of a CH2-CH3 region of the reference Fc region: M252Y, S254T or T256E (according to the EU numbering system).

These so called 'YTE' modifications located at the CH2-CH3 interface of the Fc region have been shown to increase the binding affinity at pH 6.0 to the MHC Class I neonatal Fc receptor (FcRn), localized within the acidic endosomes of endothelial and hematopoietic cells, which increases efficient recycling of administered mAb and longer half-life in the plasma.

In some embodiments, the Fc region comprises modification corresponding to the combination of substitutions M428L/N434S as described in Zalevsky etal. Nat Biotechnol. (2010) 28:157-159.

In some embodiments, the Fc region comprises modification corresponding to the combination of substitutions S267E/L328F as described in Chu et al., Mol Immunol. (2008) 45:3926-3933. In some embodiments, the Fc region comprises modification corresponding to the combination of substitutions N325S/L328F as described in Shang et al. Biol Chem. (2014) 289:15309-15318.

In some embodiments, the Fc region comprises modification to reduce/prevent an Fc-mediated function. In some embodiments, the Fc region comprises modification to reduce/prevent ADCC. In some embodiments, the Fc region comprises modification to reduce/prevent ADCP. In some embodiments, the Fc region comprises modification to reduce/prevent CDC. In some embodiments, an antigen-binding molecule comprising an Fc region comprising modification to reduce/prevent an Fc-mediated function (*e.g.* ADCC, ADCP, CDC) induces a reduced level of the relevant effector function as compared to an antigen-binding molecule comprising the corresponding unmodified Fc region.

In some embodiments, the Fc region comprises modification to reduce/prevent binding to an Fc receptor. In some embodiments, the Fc region comprises modification to reduce/prevent binding to an Fcγ receptor. In some embodiments, the Fc region comprises modification to reduce/prevent binding to one or more of FcγRI, FcγRIIa, FcγRIIb, FcγRIIc, FcγRIIIa and FcγRIIIb. In some embodiments, the Fc region comprises modification to reduce/prevent binding to FcγRIIIa. In some embodiments, the Fc region comprises modification to reduce/prevent binding to FcγRIIa. In some embodiments, the Fc region comprises modification to reduce/prevent binding to FcγRIIb. In some embodiments, the Fc region comprises modification to reduce/prevent binding to a complement protein. In some embodiments, the Fc region comprises modification to reduce/prevent binding to C1q. In some embodiments, the Fc region comprises modification to reduce/prevent glycosylation of the amino acid residue corresponding to N297.

In some embodiments, the Fc region is not able to induce one or more Fc-mediated functions (*i.e.* lacks the ability to elicit the relevant Fc-mediated function(s)). Accordingly, antigen-binding molecules comprising such Fc regions also lack the ability to induce the relevant function(s). Such antigen-binding molecules may be described as being devoid of the relevant function(s).

In some embodiments, the Fc region is not able to induce ADCC. In some embodiments, the Fc region is not able to induce ADCP. In some embodiments, the Fc region is not able to induce CDC. In some embodiments, the Fc region is not able to induce ADCC and/or is not able to induce ADCP and/or is not able to induce CDC.

In some embodiments, the Fc region is not able to bind to an Fc receptor. In some embodiments, the Fc region is not able to bind to an Fcγ receptor. In some embodiments, the Fc region is not able to bind to one or more of FcγRI, FcγRIIa, FcγRIIb, FcγRIIc, FcγRIIIa and FcγRIIIb. In some embodiments, the Fc region is not able to bind to FcγRllla. In some embodiments, the Fc region is not able to bind to FcγRIIa. In some embodiments, the Fc region is not able to bind to FcγRIIb. In some embodiments, the Fc region is not able to bind to FcRn. In some embodiments, the Fc region is not able to bind to a complement protein. In some embodiments, the Fc region is not able to bind to C1q. In some embodiments, the Fc region is not glycosylated at the amino acid residue corresponding to N297.

In some embodiments, the Fc region comprises modification corresponding to N297A or N297Q or N297G as described in Leabman et al., MAbs. (2013) 5:896-903. In some embodiments, the Fc region comprises modification corresponding to L235E as described in Alegre et al., J Immunol. (1992) 148:3461-3468. In some embodiments, the Fc region comprises modification corresponding to the combination of substitutions L234A/L235A or F234A/L235A as described in Xu et al., Cell Immunol. (2000) 200:16-26. In some embodiments, the Fc region comprises modification corresponding to P329A or P329G as described in Schlothauer et al., Protein Engineering, Design and Selection (2016), 29(10):457-466. In some embodiments, the Fc region comprises modification corresponding to the combination of substitutions L234A/L235A/P329G as described in Lo et al. J. Biol. Chem (2017) 292(9):3900-3908. In some embodiments, the Fc region comprises modification corresponding to the combination of substitutions described in Rother et al., Nat Biotechnol. (2007) 25:1256-1264. In some embodiments, the Fc region comprises modification corresponding to the combination of substitutions S228P/L235E as described in Newman et al., Clin. Immunol. (2001) 98:164-174. In some embodiments, the Fc region comprises modification corresponding to the combination of substitutions H268Q/V309L/A330S/P331S as described in An et al., MAbs. (2009) 1:572-579. In some embodiments, the Fc region comprises modification corresponding to the combination of substitutions V234A/G237A/P238S/H268A/V309L/A330S/P331S as described in Vafa etal., Methods. (2014) 65:114-126. In some embodiments, the Fc region comprises modification corresponding to the combination of substitutions L234A/L235E/G237A/A330S/P331S as described in US 2015/0044231 A1.

The combination of substitutions 'L234A/L235A' and corresponding substitutions (such as *e.g.* F234A/L235A in human IgG4) are known to disrupt binding of Fc to Fcγ receptors and inhibit ADCC, ADCP, and also to reduce C1q binding and thus CDC (Schlothauer et al., Protein Engineering, Design and Selection (2016), 29(10):457-466, hereby incorporated by reference in entirety). The substitutions 'P329G' and 'P329A' reduce C1q binding (and thereby CDC). Substitution of 'N297' with 'A', 'G' or 'Q' is known to eliminate glycosylation, and thereby reduce Fc binding to C1q and Fcγ receptors, and thus CDC and ADCC. Lo etal. J. Biol. Chem (2017) 292(9):3900-3908 (hereby incorporated by reference in its entirety) reports that the combination of substitutions L234A/L235A/P329G eliminated complement binding and fixation as well as Fc γ receptor dependent, antibody-dependent, cell-mediated cytotoxicity in both murine IgG2a and human IgG1.

The combination of substitutions L234A/L235E/G237A/A330S/P331S in IgG1 Fc is disclosed in US 2015/0044231 A1 to abolish induction of phagocytosis, ADCC and CDC.

In some embodiments, the Fc region comprises modification corresponding to the substitution S228P as described in Silva et al., J Biol Chem. (2015) 290(9):5462-5469. The substitution S228P in IgG4 Fc reduces Fab-arm exchange (Fab-arm exchange can be undesirable).

In some embodiments, the Fc region comprises modification corresponding to the combination of substitutions L234A/L235A. In some embodiments, the Fc region comprises modification corresponding to the substitution P329G. In some embodiments, the Fc region comprises modification corresponding to the substitution N297Q.

In some embodiments, the Fc region comprises modification corresponding to the combination of substitutions L234A/L235A/P329G.

In some embodiments, the Fc region comprises modification corresponding to the combination of substitutions L234A/L235A/P329G/N297Q.

In some embodiments, the Fc region comprises modification corresponding to the combination of substitutions L234A/L235E/G237A/A330S/P331S.

In some embodiments, the Fc region comprises modification corresponding to the substitution S228P, e.g. in IgG4.

In some embodiments, the antigen-binding molecule does not comprise an Fc region. In some embodiments, the antigen-binding molecule consists essentially of an Fv moiety (*e.g.* is a scFv or a dsFv). In some embodiments, the antigen-binding molecule consists essentially of a Fab moiety (*e.g.* is a Fab, CrossFab, scFab, scCrossFab or F(ab')2).

As used herein, an antigen-binding molecule that 'consists essentially of a reference polypeptide domain(s)/amino acid sequence(s) either (i) consists of the reference domain(s)/amino acid sequence(s), or (ii) comprises the reference domain(s)/amino acid sequence(s), wherein the reference domain(s)/amino acid sequence(s) constitute at least 80% (*e.g.* one of ≥85% ≥86%, ≥87%, ≥88%, ≥89%, ≥90%, ≥91 %, ≥92%, ≥93%, ≥94%, ≥95%, ≥96%, ≥97%, ≥98%, ≥99%) of the molecule. It will be appreciated that an antigen-binding molecule that 'consists essentially of a reference polypeptide domain(s)/amino acid sequence(s) may comprise the reference domain(s)/amino acid sequence(s), and additional amino acid(s) at one or both of the N-terminal and C-terminal ends of the reference domain(s)/amino acid sequence(s), provided that the additional amino acid(s) constitute ≥20% of the molecule. By way of illustration, antigen-binding molecules that consist essentially of an Fv moiety include scFv molecules, which comprise a VH and VL connected via a linker moiety.

In some embodiments - particularly embodiments in which the antigen-binding molecule is a multispecific (*e.g.* bispecific) antigen-binding molecule - the antigen-binding molecule comprises an Fc region comprising modification in one or more of the CH2 and CH3 regions promoting association of the Fc region. Recombinant co-expression of constituent polypeptides of an antigen-binding molecule and subsequent association leads to several possible combinations. To improve the yield of the desired combinations of polypeptides in antigen-binding molecules in recombinant production, it is advantageous to introduce in the Fc regions modification(s) promoting association of the desired combination of heavy chain polypeptides. Modifications may promote *e.g.* hydrophobic and/or electrostatic interaction between CH2 and/or CH3 regions of different polypeptide chains. Suitable modifications are described *e.g.* in Ha et al., Front. Immnol (2016) 7:394, which is hereby incorporated by reference in its entirety.

In some embodiments, the antigen-binding molecule of the present disclosure comprises an Fc region comprising paired substitutions in the CH3 regions of the Fc region according to one of the following formats, as shown in Table 1 of Ha etal., Front. Immnol (2016) 7:394: KiH, KiHs-s, HA-TF, ZW1, 7.8.60, DD-KK, EW-RVT, EW-RVTs-s, SEED or A107.

### Particular exemplary polypeptides and antigen-binding molecules

The present disclosure also provides polypeptide constituents of antigen-binding molecules. The polypeptides may be provided in isolated or substantially purified form.

The antigen-binding molecule of the present disclosure may be, or may comprise, a complex of polypeptides.

In the present specification where a polypeptide comprises more than one domain or region, it will be appreciated that the plural domains/regions are preferably present in the same polypeptide chain. That is, the polypeptide comprising more than one domain or region is a fusion polypeptide comprising the domains/regions.

In some embodiments a polypeptide according to the present disclosure comprises, or consists of, a VH as described herein. In some embodiments a polypeptide according to the present disclosure comprises, or consists of, a VL as described herein.

In some embodiments, the polypeptide additionally comprises one or more antibody heavy chain constant regions (CH). In some embodiments, the polypeptide additionally comprises one or more antibody light chain constant regions (CL). In some embodiments, the polypeptide comprises a CH1, CH2 region and/or a CH3 region of an immunoglobulin (Ig).

In some embodiments, the polypeptide comprises one or more regions of an immunoglobulin heavy chain constant sequence. In some embodiments, the polypeptide comprises a CH1 region as described herein. In some embodiments, the polypeptide comprises a CH1-CH2 hinge region as described herein. In some embodiments, the polypeptide comprises a CH2 region as described herein. In some embodiments, the polypeptide comprises a CH3 region as described herein.

In some embodiments, the polypeptide comprises one or more regions of an immunoglobulin light chain constant sequence. In some embodiments, the polypeptide comprises a CL region as described herein.

In some embodiments, the polypeptide according to the present disclosure comprises a structure from N- to C-terminus according to one of the following:
(i) VH
(ii) VL
(iii) VH-CH1
(iv) VL-CL
(v) VL-CH1
(vi) VH-CL
(vii) VH-CH1-CH2-CH3
(viii) VL-CL-CH2-CH3
(ix) VL-CH1-CH2-CH3
(x) VH-CL-CH2-CH3

Also provided by the present disclosure are antigen-binding molecules composed of the polypeptides of the present disclosure. In some embodiments, the antigen-binding molecule of the present disclosure comprises one of the following combinations of polypeptides:
(A) VH + VL
(B) VH-CH1 + VL-CL
(C) VL-CH1 + VH-CL
(D) VH-CH1-CH2-CH3 + VL-CL
(E) VH-CL-CH2-CH3 + VL-CH1
(F) VL-CH1-CH2-CH3 + VH-CL
(G) VL-CL-CH2-CH3 + VH-CH1
(H) VH-CH1-CH2-CH3 + VL-CL-CH2-CH3
(I) VH-CL-CH2-CH3 + VL-CH1-CH2-CH3

In some embodiments, the antigen-binding molecule comprises more than one of a polypeptide of the combinations shown in (A) to (I) above. By way of example, with reference to (D) above, in some embodiments, the antigen-binding molecule comprises two polypeptides comprising the structure VH-CH1-CH2-CH3, and two polypeptides comprising the structure VL-CL.

In some embodiments, the antigen-binding molecule of the present disclosure comprises one of the following combinations of polypeptides:
(J) VH (anti-C1q) + VL (anti-C1q)
(K) VH (anti-C1q)-CH1 + VL (anti-C1q)-CL
(L) VL (anti-C1q)-CH1 + VH (anti-C1q)-CL
(M) VH (anti-C1q)-CH1-CH2-CH3 + VL (anti-C1q)-CL
(N) VH (anti-C1q)-CL-CH2-CH3 + VL (anti-C1q)-CH1
(O) VL (anti-C1q)-CH1-CH2-CH3 + VH (anti-C1q)-CL
(P) VL (anti-C1q)-CL-CH2-CH3 + VH (anti-C1q)-CH1
(Q) VH (anti-C1q)-CH1-CH2-CH3 + VL (anti-C1q)-CL-CH2-CH3

### Functional properties of the antigen-binding molecules

The antigen-binding molecules described herein may be characterized by reference to certain functional properties. In some embodiments, the antigen-binding molecule described herein may possess one or more of the following properties:
binds to C1q;
inhibits polarization of macrophages to an M2 or M2-like phenotype;
inhibits IL-10 production by macrophages;
inhibits macrophage efferocytosis;
does not substantially inhibit activation of the complement cascade;
inhibits binding of C1q to activated endothelial cells; and/or
inhibits C1q binding to a C1q receptor (*e.g.* cC1qR and/or LAIR1).

It will be appreciated that a given antigen-binding molecule may display more than one of the properties recited in the preceding paragraph. A given antigen-binding molecule may be evaluated for the properties recited in the preceding paragraph using suitable assays. For example, the assays may be *e.g. in vitro* assays, optionally cell-based assays or cell-free assays. In some embodiments, the assays may be *e.g. in vivo* assays, *i.e.* performed in non-human animals. In some embodiments, the assays may be *e.g. ex vivo* assays, *i.e.* performed using cells/tissue/an organ obtained from a subject.

Where assays are cell-based assays, they may comprise treating cells with a given antigen-binding molecule in order to determine whether the antigen-binding molecule displays one or more of the recited properties. Assays may employ species labelled with detectable entities in order to facilitate their detection. Assays may comprise evaluating the recited properties following treatment of cells separately with a range of quantities/concentrations of a given antigen-binding molecule (*e.g.* a dilution series).

Analysis of the results of such assays may comprise determining the concentration at which 50% of the maximal level of the relevant activity is attained. The concentration of a given agent at which 50% of the maximal level of the relevant activity is attained may be referred to as the 'half-maximal effective concentration' of the agent in relation to the relevant activity, which may also be referred to as the 'EC50'. By way of illustration, the EC50 of a given antigen-binding molecule for binding to C1q may be the concentration of the antigen-binding molecule at which 50% of the maximal level of binding to C1q is achieved.

Depending on the property, the EC50 may also be referred to as the 'half-maximal inhibitory concentration' or 'IC50', this being the concentration of the agent at which 50% of the maximal level of inhibition of a given property is observed.

The antigen-binding molecules described herein bind to C1q. In some embodiments, the antigen-binding molecule binds to human C1q.

The antigen-binding molecules and antigen-binding domains described herein preferably display specific binding to C1q. As used herein, 'specific binding' refers to binding which is selective for the antigen, and which can be discriminated from non-specific binding to non-target antigen. An antigen-binding molecule/domain that specifically binds to a target molecule preferably binds the target with greater affinity, and/or with greater duration than it binds to other, non-target molecules.

The ability of a given antigen-binding molecule to bind specifically to C1q can be determined by analysis according to methods known in the art, such as by ELISA, Surface Plasmon Resonance (SPR; see *e.g.* Hearty et al., Methods Mol Biol (2012) 907:411-442), Bio-Layer Interferometry (BLI; see *e.g.* Lad et al., (2015) J Biomol Screen 20(4): 498-507), flow cytometry, immunoblot (*e.g.* western blot), immunoprecipitation or by radiolabelled antigen-binding assay (RIA). Through such analysis binding to C1q can be detected and/or quantified. In some embodiments, the binding may be the response detected in a given assay.

In some embodiments, the extent of binding of the antigen-binding molecule to a non-target molecule is less than about 10% of the binding of the antibody to the target molecule as measured, *e.g.* by ELISA, SPR, BLI or by RIA. Alternatively, binding specificity may be reflected in terms of binding affinity where the antigen-binding molecule binds with a dissociation constant (KD) that is at least 0.1 order of magnitude (*i.e*. 0.1 x 10n, where n is an integer representing the order of magnitude) greater than the KD of the antigen-binding molecule towards a non-target molecule. This may optionally be one of at least 0.2, 0.3, 0.4, 0.5, 0.6, 0.7, 0.8, 0.9, 1.0, 1.5, or 2.0.

In some embodiments, the antigen-binding molecule described herein binds to C1q with an affinity in the micromolar range, i.e. K_{D} = 9.9 x 10⁻⁴ to 1 x 10⁻⁶ M. In some embodiments, the antigen-binding molecule described herein binds to C1q with sub-micromolar affinity, i.e. K_{D} < 1 x 10⁻⁶ M. In some embodiments, the antigen-binding molecule described herein binds to C1q with an affinity in the nanomolar range, i.e. K_{D} = 9.9 x 10⁻⁷ to 1 x 10⁻⁹ M. In some embodiments, the antigen-binding molecule described herein binds to C1q with sub-nanomolar affinity, i.e. K_{D} < 1 x 10⁻⁹ M. In some embodiments, the antigen-binding molecule described herein binds to C1q with an affinity in the picomolar range, i.e. K_{D} = 9.9 x 10⁻¹⁰ to 1 x 10⁻¹² M. In some embodiments, the antigen-binding molecule described herein binds to C1q with sub-picomolar affinity, i.e. K_{D} < 1 x 10⁻¹² M.

The antigen-binding molecules of the present disclosure may bind to a particular region of interest of the C1q polypeptide complex, or a particular region of a constituent polypeptide of a C1q polypeptide complex.

The region of a given target molecule to which an antigen-binding molecule binds can be determined by the skilled person using various methods well known in the art, including X-ray co-crystallography analysis of antibody-antigen complexes, peptide scanning, mutagenesis mapping, hydrogen-deuterium exchange analysis by mass spectrometry, phage display, competition ELISA and proteolysis-based 'protection' methods. Such methods are described, for example, in Gershoni et al., BioDrugs, 2007, 21(3):145-156, which is hereby incorporated by reference in its entirety.

In some embodiments, the antigen-binding molecule inhibits C1q-mediated polarization of macrophages to an M2 or M2-like phenotype (*e.g.* as described hereinabove). In some embodiments, the antigen-binding molecule inhibits C1q-mediated development and/or maturation of macrophages to an M2 or M2-like phenotype. In some embodiments, the antigen-binding molecule reduces the number of macrophages having an M2 or M2-like phenotype following treatment with C1q. In some embodiments, the antigen-binding molecule reduces the proportion of macrophages having an M2 or M2-like phenotype (*i.e.* within a given population of macrophages) following treatment with C1q.

In some embodiments, the antigen-binding molecule promotes polarization of macrophages to an M1 or M1-like phenotype (*e.g.* as described hereinabove). In some embodiments, the antigen-binding molecule promotes development and/or maturation of macrophages to an M1 or M1-like phenotype. In some embodiments, the antigen-binding molecule increases the number of macrophages having an M1 or M1-like phenotype following treatment with C1q. In some embodiments, the antigen-binding molecule increases the proportion of macrophages having an M1 or M1-like phenotype (i.e. within a given population of macrophages) following treatment with C1q.

The ability of a given antigen-binding molecule to inhibit polarization of macrophages to an M2 or M2-like phenotype/inhibit development and/or maturation of macrophages to an M2 or M2-like phenotype/ reduce the number of macrophages having an M2 or M2-like phenotype/reduce the proportion of macrophages having an M2 or M2-like phenotype/promote polarization of macrophages to an M1 or M1-like phenotype/promote development and/or maturation of macrophages to an M1 or M1-like phenotype/ increase the number of macrophages having an M1 or M1-like phenotype/increase the proportion of macrophages having an M1 or M1-like phenotype can be evaluated *e.g.* in an appropriate *in vitro* assay of macrophage polarization. For example, a method may comprise culturing macrophages in the presence of C1q and the antigen-binding molecule, and subsequently (i.e. after a sufficient period of time for an effect on macrophage polarization/differentiation/maturation to be observed) evaluating the number/proportion of macrophages having an M2 or M2-like phenotype, and/or the number/proportion of macrophages having an M1 or M1-like phenotype. An antigen-binding molecule may be determined to inhibit polarization of macrophages to an M2 or M2-like phenotype/inhibit development and/or maturation of macrophages to an M2 or M2-like phenotype/ reduce the number of macrophages having an M2 or M2-like phenotype/reduce the proportion of macrophages having an M2 or M2-like phenotype/promote polarization of macrophages to an M1 or M1-like phenotype/promote development and/or maturation of macrophages to an M1 or M1-like phenotype/ increase the number of macrophages having an M1 or M1-like phenotype/increase the proportion of macrophages having an M1 or M1-like phenotype where culture in the presence of the antigen-binding molecule is found to result in a decrease in the number/proportion of macrophages having an M2 or M2-like phenotype and/or an increase in the number/proportion of macrophages having an M1 or M1-like phenotype, as compared to the number/proportion of such macrophages observed in an appropriate control condition, *e.g.* wherein macrophages are cultured with C1q in the absence of an antigen-binding molecule, or wherein macrophages are cultured with C1q in the presence of an isotype-matched control antigen-binding molecule known not to affect M1/M2 macrophage polarization.

In accordance with such methods, M1/M1-like and/or M2/M2-like macrophages may be identified on the basis of one or more markers of the relevant cell type. For example, in Example 4 of the present disclosure, expression of IL-10 is used as a correlate of the M2/M2-like phenotype.

In some embodiments, the ability of an antigen-binding molecule to inhibit polarization of macrophages to an M2 or M2-like phenotype/inhibit development and/or maturation of macrophages to an M2 or M2-like phenotype/reduce the number of macrophages having an M2 or M2-like phenotype/reduce the proportion of macrophages having an M2 or M2-like phenotype/promote polarization of macrophages to an M1 or M1-like phenotype/promote development and/or maturation of macrophages to an M1 or M1-like phenotype/increase the number of macrophages having an M1 or M1-like phenotype/increase the proportion of macrophages having an M1 or M1-like phenotype is evaluated essentially as described in Example 4 of the present disclosure.

In some embodiments, in an appropriate assay of macrophage polarization, the antigen-binding molecule reduces the number/proportion of macrophages having an M2 or M2-like phenotype to less than 1 times, *e.g.* ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the number/proportion observed in the absence of the antigen-binding molecule (or in the presence of an appropriate control antigen-binding molecule, *e.g.* an antigen-binding molecule known not to affect M1/M2 macrophage polarization).

In some embodiments, in an appropriate assay of macrophage polarization, the antigen-binding molecule increases the number/proportion of macrophages having an M1 or M1-like phenotype to greater than 1 times, *e.g.* one of ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times or ≥10 times the number/proportion observed in the absence of the antigen-binding molecule (or in the presence of an appropriate control antigen-binding molecule, *e.g.* an antigen-binding molecule known not to affect M1/M2 macrophage polarization).

In some embodiments, the antigen-binding molecule inhibits IL-10 expression/production by macrophages, *e.g.* following treatment with C1q. In some embodiments, the antigen-binding molecule inhibits gene and/or protein expression of IL-10 by macrophages. In some embodiments, the antigen-binding molecule inhibits secretion of IL-10 by macrophages, *i.e.* into cell culture medium of macrophages cultured *in vitro.* In some embodiments, the antigen-binding molecule reduces the number/proportion of IL-10-producing/expressing macrophages.

*IL10* gene expression can be determined *e.g.* by detection of mRNA encoding IL-10, for example by quantitative real-time PCR (qRT-PCR). IL-10 protein expression can be determined *e.g.* by antibody-based methods, for example by western blot, immunohistochemistry, immunocytochemistry, flow cytometry, or ELISA. IL-10 protein expression may be evaluated by detection of the protein within the macrophages, or in cell culture medium in which the macrophages have been cultured.

The ability of a given antigen-binding molecule to inhibit IL-10 expression/production by macrophages can be evaluated *e.g.* in a method comprising culturing macrophages in the presence of C1q and the antigen-binding molecule, and subsequently (i.e. after a sufficient period of time for an effect on expression/production of IL-10 to be observed) evaluating the level of (gene or protein) expression of IL-10 by the macrophages, and/or the level of IL-10 in the cell culture medium. An antigen-binding molecule may be determined to inhibit IL-10 expression/production where culture in the presence of the antigen-binding molecule is found to result in a decrease in the level of IL-10 expression by the cells and/or a decrease in the level of IL-10 in the cell culture medium, as compared to the level observed in an appropriate control condition, *e.g.* wherein macrophages are cultured with C1q in the absence of an antigen-binding molecule, or wherein macrophages are cultured with C1q in the presence of an isotype-matched control antigen-binding molecule known not to affect expression/production of IL-10 by macrophages.

In some embodiments, the ability of an antigen-binding molecule to inhibit IL-10 expression/production by macrophages is evaluated essentially as described in Example 4 of the present disclosure.

In some embodiments, in an appropriate assay of IL-10 expression/production by macrophages, the antigen-binding molecule decreases the expression/production of IL-10 by macrophages to less than 1 times, *e.g.* ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level observed in the absence of the antigen-binding molecule (or in the presence of an appropriate control antigen-binding molecule, *e.g.* an antigen-binding molecule known not to affect IL-10 expression/production by macrophages). In some embodiments, the antigen-binding molecule inhibits more than 10%, *e.g.* ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥95%, ≥96%, ≥97%, ≥98% or ≥99% of the IL-10 expression/production by macrophages observed in the absence of the antigen-binding molecule (or in the presence of an appropriate control antigen-binding molecule, *e.g.* an antigen-binding molecule known not to affect IL-10 expression/production by macrophages).

In some embodiments, the antigen-binding molecule inhibits more than 15%, *e.g.* ≥30%, ≥35% or ≥40% of the IL-10 expression/production by macrophages observed in the absence of the antigen-binding molecule (or in the presence of an appropriate control antigen-binding molecule, *e.g.* an antigen-binding molecule known not to affect IL-10 expression/production by macrophages). In some embodiments, the antigen-binding molecule inhibits more than 50%, *e.g.* ≥55%, ≥60%, ≥65%, ≥70%, ≥75%, ≥80%, ≥85%, ≥90% or ≥95% of the IL-10 expression/production by macrophages observed in the absence of the antigen-binding molecule (or in the presence of an appropriate control antigen-binding molecule, *e.g.* an antigen-binding molecule known not to affect IL-10 expression/production by macrophages).

In some embodiments, the antigen-binding molecule inhibits macrophage efferocytosis. In some embodiments, the antigen-binding molecule inhibits efferocytosis by macrophages of cells (*e.g.* apoptotic cells) coated with C1q. In some embodiments, the antigen-binding molecule reduces the number/proportion of efferocytotic macrophages.

The ability of a given antigen-binding molecule to inhibit macrophage efferocytosis can be evaluated *e.g.* in a method comprising co-culturing macrophages with apoptotic cells coated with C1q in the presence of the antigen-binding molecule, and subsequently (i.e. after a sufficient period of time for an effect on efferocytosis of the apoptotic cells to be observed) evaluating the level of efferocytosis of the apoptotic cells by the macrophages. An antigen-binding molecule may be determined to inhibit macrophage efferocytosis where culture in the presence of the antigen-binding molecule is found to result in a decrease in the level of efferocytosis, as compared to the level observed in an appropriate control condition, *e.g.* wherein macrophages are co-cultured with apoptotic cells coated with C1q in the absence of an antigen-binding molecule, or wherein macrophages are co-cultured with apoptotic cells coated with C1q in the presence of an isotype-matched control antigen-binding molecule known not to affect macrophage efferocytosis.

Efferocytosis of the apoptotic cells may be evaluated through detection of an appropriate marker; for example, in Example 3 herein, apoptotic cells are labelled with a pH-sensitive dye which is only fluorescent following internalization by the macrophages, and exposure to the acidic environment of the endosomes and lysosomes. Further methods for evaluating phagocytosis and efferocytosis are described *e.g.* in Evans et al. Methods Mol Biol. (2017) 1519:25-41, Taefehshokr and Heit Methods in Molecular Biology; MIMB, volume 2692. Springer. Electronic ISSN 1940-6029, and Parv et al. Front. Endocrinol. (2021) 12: 606175.

In some embodiments, the ability of an antigen-binding molecule to inhibit macrophage efferocytosis is evaluated essentially as described in Example 3 of the present disclosure.

In some embodiments, in an appropriate assay of macrophage efferocytosis, the antigen-binding molecule decreases the level of efferocytosis to less than 1 times, *e.g.* ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level observed in the absence of the antigen-binding molecule (or in the presence of an appropriate control antigen-binding molecule, *e.g.* an antigen-binding molecule known not to affect macrophage efferocytosis). In some embodiments, the antigen-binding molecule inhibits more than 10%, *e.g.* ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥95%, ≥96%, ≥97%, ≥98% or ≥99% of the efferocytosis observed in the absence of the antigen-binding molecule (or in the presence of an appropriate control antigen-binding molecule, *e.g.* an antigen-binding molecule known not to affect macrophage efferocytosis).

In some embodiments, the antigen-binding molecule inhibits more than 25%, *e.g.* ≥30%, ≥35%, ≥40%, ≥45%, ≥50%, ≥55%, ≥60%, ≥65%, ≥70%, ≥75%, ≥80%, ≥85%, ≥90%, ≥95%, ≥96%, ≥97%, ≥98% or ≥99% of the efferocytosis observed in the absence of the antigen-binding molecule (or in the presence of an appropriate control antigen-binding molecule, *e.g.* an antigen-binding molecule known not to affect macrophage efferocytosis).

In some embodiments, the antigen-binding molecule inhibits more than 15%, *e.g.* ≥30%, ≥35% or ≥40% of the efferocytosis observed in the absence of the antigen-binding molecule (or in the presence of an appropriate control antigen-binding molecule, *e.g.* an antigen-binding molecule known not to affect macrophage efferocytosis). In some embodiments, the antigen-binding molecule inhibits more than 50%, *e.g.* ≥55%, ≥60%, ≥65%, ≥70%, ≥75%, ≥80%, ≥85%, ≥90% or ≥95% of the efferocytosis observed in the absence of the antigen-binding molecule (or in the presence of an appropriate control antigen-binding molecule, *e.g.* an antigen-binding molecule known not to affect macrophage efferocytosis).

In some embodiments, the antigen-binding molecule does not substantially inhibit activation of the complement cascade. In some embodiments, the antigen-binding molecule does not substantially inhibit the activity of the C1 complex.

Whether a given antigen-binding molecule inhibits activation of the complement cascade and/or activity of the C1 complex can be evaluated *e.g.* in an assay of complement activity. Many such assays are known, and include assays which involve detecting and quantifying the level or activity of a polypeptide or polypeptide complex whose expression and/or activity is upregulated following activation of the complement cascade, or whose expression and/or activity is upregulated as a consequence of an activity of the C1 complex. For example, the method may comprise incubating activated human sera with the antigen-binding molecule, and subsequently (i.e. after a sufficient period of time for an effect on activation of the complement cascade and/or activity of the C1 complex to be observed) evaluating the level of a correlate of activation of the complement cascade and/or activity of the C1 complex. An antigen-binding molecule may be determined not to substantially inhibit activation of the complement cascade and/or activity of the C1 complex if the level of the relevant correlate is similar to (i.e. ≥0.5 times and ≤2 times, *e.g.* one of ≥0.6 times and ≤1.8 times, ≥0.7 times and ≤1.6 times, ≥0.8 times and ≤1.4 times, ≥0.9 times and ≤1.2 times, or ≤0.95 times and ≤1.1 times) the level of the level observed in an appropriate control condition, *e.g.* wherein activated human sera is incubated in the absence of an antigen-binding molecule, or wherein activated human sera is incubated in the presence of an isotype-matched control antigen-binding molecule known not to affect activation of the complement cascade and/or activity of the C1 complex.

Activation of the complement cascade and/or activity of the C1 complex can be evaluated by measuring any appropriate correlate thereof. By way of illustration, in Example 5 herein, activation of the complement cascade is evaluated through quantification of the level of the C5b-9 terminal complement complex by ELISA. Further assays for investigating activation of the complement cascade are described in Kirschfink and Mollnes, Clin Diagn Lab Immunol. (2003) Nov; 10(6): 982-989.

In some embodiments, whether a given antigen-binding molecule substantially inhibits activation of the complement cascade and/or activity of the C1 complex is evaluated essentially as described in Example 5 of the present disclosure.

In some embodiments, in an appropriate assay of activation of the complement cascade and/or activity of the C1 complex, the antigen-binding molecule inhibits less than 20%, *e.g.* ≤15%, ≤10% or ≤5% of the activation of the complement cascade and/or activity of the C1 complex observed in the absence of the antigen-binding molecule (or in the presence of an appropriate control antigen-binding molecule, *e.g.* an antigen-binding molecule known not to affect activation of the complement cascade and/or activity of the C1 complex).

In some embodiments, the antigen-binding molecule inhibits activation of the complement cascade. In some embodiments, the antigen-binding molecule inhibits the activity of the C1 complex.

In some embodiments, in an appropriate assay of activation of the complement cascade and/or activity of the C1 complex, the antigen-binding molecule decreases the level of a correlate of activation of the complement cascade and/or activity of the C1 complex to less than 1 times, *e.g.* ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level observed in the absence of the antigen-binding molecule (or in the presence of an appropriate control antigen-binding molecule, *e.g.* an antigen-binding molecule known not to affect activation of the complement cascade and/or activity of the C1 complex). In some embodiments, the antigen-binding molecule inhibits more than 10%, *e.g.* ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥95%, ≥96%, ≥97%, ≥98% or ≥99% of the activation of the complement cascade and/or activity of the C1 complex observed in the absence of the antigen-binding molecule (or in the presence of an appropriate control antigen-binding molecule, *e.g.* an antigen-binding molecule known not to affect activation of the complement cascade and/or activity of the C1 complex).

In some embodiments, the antigen-binding molecule inhibits more than 80%, *e.g.* ≥85%, ≥80% or ≥95% of the activation of the complement cascade and/or activity of the C1 complex observed in the absence of the antigen-binding molecule (or in the presence of an appropriate control antigen-binding molecule, *e.g.* an antigen-binding molecule known not to affect activation of the complement cascade and/or activity of the C1 complex).

In some embodiments, the antigen-binding molecule inhibits binding of C1q to activated endothelial cells.

The ability of a given antigen-binding molecule to inhibit binding of C1q to activated endothelial cells can be evaluated *e.g.* in a method comprising incubating activated endothelial cells (*e.g.* endothelial cells activated by treatment with TNFα) with C1q in the presence of the antigen-binding molecule, and subsequently (i.e. after a sufficient period of time for binding of C1q to the activated endothelial cells to be observed) evaluating the level of C1q bound to the activated endothelial cells. An antigen-binding molecule may be determined to inhibit binding of C1q to activated endothelial cells where incubation in the presence of the antigen-binding molecule is found to result in a decrease in the level of C1q bound to the activated endothelial cells, as compared to the level observed in an appropriate control condition, *e.g.* wherein the activated endothelial cells are incubated with C1q in the absence of an antigen-binding molecule, or wherein the activated endothelial cells are incubated with C1q in the presence of an isotype-matched control antigen-binding molecule known not to affect interaction between C1q and activated endothelial cells.

Techniques for evaluating the level of a given protein bound to a given cell type are well known in the art, and include *e.g.* ELISA- and flow cytometry-based methodologies. By way of illustration, in Example 6 herein, binding of C1q to activated endothelial cells is evaluated by ELISA. In some embodiments, the ability of an antigen-binding molecule to inhibit binding of C1q to activated endothelial cells is evaluated essentially as described in Example 6 of the present disclosure.

In some embodiments, in an appropriate assay of binding of C1q to activated endothelial cells, the antigen-binding molecule decreases the level of binding to less than 1 times, *e.g*. ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level observed in the absence of the antigen-binding molecule (or in the presence of an appropriate control antigen-binding molecule, *e.g.* an antigen-binding molecule known not to affect interaction between C1q and activated endothelial cells). In some embodiments, the antigen-binding molecule inhibits more than 10%, *e.g.* ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥95%, ≥96%, ≥97%, ≥98% or ≥99% of the binding of C1q to activated endothelial cells observed in the absence of the antigen-binding molecule (or in the presence of an appropriate control antigen-binding molecule, *e.g.* an antigen-binding molecule known not to affect interaction between C1q and activated endothelial cells).

In some embodiments, the antigen-binding molecule inhibits more than 20%, *e.g.* ≥25%, ≥30% or ≥35% of the binding of C1q to activated endothelial cells observed in the absence of the antigen-binding molecule (or in the presence of an appropriate control antigen-binding molecule, e.g. an antigen-binding molecule known not to affect interaction between C1q and activated endothelial cells). In some embodiments, the antigen-binding molecule inhibits more than 80%, *e.g.* ≥85%, ≥80% or ≥95% of the binding of C1q to activated endothelial cells observed in the absence of the antigen-binding molecule (or in the presence of an appropriate control antigen-binding molecule, *e.g.* an antigen-binding molecule known not to affect interaction between C1q and activated endothelial cells).

In some embodiments, the antigen-binding molecule inhibits binding of C1q to a C1q receptor (*e.g.* a C1q receptor selected from cC1qR, LAIR1, LAIR2, gC1qR, CR1, DDR1, and DDR2). In some embodiments, the antigen-binding molecule inhibits binding of C1q to cC1qR. In some embodiments, the antigen-binding molecule inhibits binding of C1q to LAIR1. In some embodiments, the antigen-binding molecule inhibits binding of C1q to cC1qR and LAIR1.

The ability of a given antigen-binding molecule to inhibit binding of C1q to a given C1q receptor (*e.g.* cC1qR, LAIR1) can be evaluated *e.g.* in a method comprising contacting C1q with the C1q receptor in the presence of the antigen-binding molecule, and subsequently (i.e. after a sufficient period of time for binding of C1q to the C1q receptor to be observed) evaluating the level of binding of C1q to the C1q receptor. An antigen-binding molecule may be determined to inhibit binding of C1q to a given C1q receptor where incubation in the presence of the antigen-binding molecule is found to result in a decrease in the level of binding of C1q to the C1q receptor, as compared to the level observed in an appropriate control condition, *e.g.* wherein C1q and the C1q receptor are incubated in the absence of an antigen-binding molecule, or wherein C1q and the C1q receptor are incubated in the presence of an isotype-matched control antigen-binding molecule known not to affect interaction between C1q and the C1q receptor.

Techniques for evaluating the level of interaction between given proteins are well known in the art, and include *e.g.* resonance energy transfer techniques such as fluorescence resonance energy transfer (FRET) and Bioluminescence Resonance Energy Transfer (BRET), using appropriate labelled interaction partners, *e.g.* as described in Ciruela, Curr. Opin. Biotechnol. (2008) 19(4):338-43. Other suitable technologies include ELISA, SPR, BLI, flow cytometry, immunoblot, immunoprecipitation, *etc.*

By way of illustration, in Example 7 herein, binding of C1q to cC1qRand LAIR1 is evaluated by ELISA. In some embodiments, the ability of an antigen-binding molecule to inhibit binding of C1q to a C1q receptor (*e.g.* cC1qR or LAIR1) is evaluated essentially as described in Example 7 of the present disclosure.

In some embodiments, in an appropriate assay of binding of C1q to cC1qR, the antigen-binding molecule decreases the level of binding to less than 1 times, *e.g.* ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level observed in the absence of the antigen-binding molecule (or in the presence of an appropriate control antigen-binding molecule, *e.g.* an antigen-binding molecule known not to affect interaction between C1q and cC1qR). In some embodiments, the antigen-binding molecule inhibits more than 10%, *e.g.* ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥95%, ≥96%, ≥97%, ≥98% or ≥99% of the binding of C1q to cC1qR observed in the absence of the antigen-binding molecule (or in the presence of an appropriate control antigen-binding molecule, *e.g.* an antigen-binding molecule known not to affect interaction between C1q and cC1qR).

In some embodiments, the antigen-binding molecule inhibits more than 10%, *e.g.* ≥20%, ≥25%, ≥30%, ≥35%, ≥40%, ≥45% or ≥50% of the binding of C1q to cC1qR observed in the absence of the antigen-binding molecule (or in the presence of an appropriate control antigen-binding molecule, *e.g.* an antigen-binding molecule known not to affect interaction between C1q and cC1qR). In some embodiments, the antigen-binding molecule inhibits more than 80%, *e.g.* ≥85%, ≥90% or ≥95% of the binding of C1q to cC1 qR observed in the absence of the antigen-binding molecule (or in the presence of an appropriate control antigen-binding molecule, *e.g.* an antigen-binding molecule known not to affect interaction between C1q and cC1qR).

In some embodiments, in an appropriate assay of binding of C1q to LAIR, the antigen-binding molecule decreases the level of binding to less than 1 times, *e.g*. ≤0.99 times, ≤0.95 times, ≤0.9 times, ≤0.85 times, ≤0.8 times, ≤0.75 times, ≤0.7 times, ≤0.65 times, ≤0.6 times, ≤0.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level observed in the absence of the antigen-binding molecule (or in the presence of an appropriate control antigen-binding molecule, *e.g.* an antigen-binding molecule known not to affect interaction between C1q and LAIR). In some embodiments, the antigen-binding molecule inhibits more than 10%, *e.g.* ≥20%, ≥25%, ≥30%, ≥35%, ≥40%, ≥45%, ≥50%, ≥55%, ≥60%, ≥65%, ≥70%, ≥75%, ≥80%, ≥85%, ≥90%, ≥95%, ≥96%, ≥97%, ≥98% or ≥99% of the binding of C1q to LAIR observed in the absence of the antigen-binding molecule (or in the presence of an appropriate control antigen-binding molecule, *e.g.* an antigen-binding molecule known not to affect interaction between C1q and LAIR).

In some embodiments, the antigen-binding molecule inhibits more than 80%, *e.g.* ≥85%, ≥90% or ≥95% of the binding of C1q to LAIR1 observed in the absence of the antigen-binding molecule (or in the presence of an appropriate control antigen-binding molecule, *e.g.* an antigen-binding molecule known not to affect interaction between C1q and LAIR1).

### Linkers and additional sequences

The antigen-binding molecules and polypeptides of the present disclosure may additionally comprise further amino acids or sequences of amino acids.

The antigen-binding molecules and polypeptides of the present disclosure may comprise one or more linker sequences between sequences of amino acids. For example, a linker sequence may be provided between a VH sequence and a VL sequence, providing linkage between the VH and VL (*e.g.* as in an scFv molecule).

Linker sequences are known to the skilled person, and are described, for example in Chen et al., Adv Drug Deliv Rev (2013) 65(10): 1357-1369, which is hereby incorporated by reference in its entirety. In some embodiments, a linker sequence may be a flexible linker sequence. Flexible linker sequences allow for relative movement of the amino acid sequences which are linked by the linker sequence. Flexible linkers are known to the skilled person, and several are identified in Chen et al., Adv Drug Deliv Rev (2013) 65(10): 1357-1369. Flexible linker sequences often comprise high proportions of glycine and/or serine residues.

In some embodiments, the linker sequence comprises at least one glycine residue and/or at least one serine residue. In some embodiments, the linker sequence comprises or consists of glycine and serine residues. In some embodiments, the linker sequence has the structure: (GxS)n or (GxS)nGm; wherein G = glycine, S = serine, x = 3 or 4, n = 2, 3, 4, 5 or 6, and m = 0, 1, 2 or 3. In some embodiments, the linker sequence comprises one or more (*e.g.* 1, 2, 3, 4, 5 or 6) copies (*e.g.* in tandem) of the sequence motif G₄S. In some embodiments, the linker sequence comprises or consists of (G₄S)₄ or (G₄S)₆. In some embodiments, the linker sequence has a length of 1-2, 1-3, 1-4, 1-5, 1-10, 1-15, 1-20, 1-25, or 1-30 amino acids.

The antigen-binding molecules and polypeptides of the present disclosure may comprise amino acid sequence(s) to facilitate expression, folding, trafficking, processing, purification or detection of the antigen-binding molecule/polypeptide. For example, antigen-binding molecules and polypeptides of the present disclosure may additionally comprise a sequence of amino acids forming a detectable moiety, *e.g.* as described hereinbelow.

The antigen-binding molecules and polypeptides of the present disclosure may additionally comprise a signal peptide (also known as a leader sequence or signal sequence). Signal peptides normally consist of a sequence of 5-30 hydrophobic amino acids, which form a single alpha helix. Secreted proteins and proteins expressed at the cell surface often comprise signal peptides. Signal peptides are known for many proteins, and are recorded in databases such as GenBank, UniProt and Ensembl, and/or can be identified/predicted *e.g.* using amino acid sequence analysis tools such as SignalP (Petersen et al., 2011 Nature Methods 8: 785-786) or Signal-BLAST (Frank and Sippl, 2008 Bioinformatics 24: 2172-2176).

The signal peptide may be present at the N-terminus of the antigen-binding molecule/polypeptide, and may be present in the newly synthesized antigen-binding molecule/polypeptide. The signal peptide provides for efficient trafficking of the antigen-binding molecule/polypeptide. Signal peptides are often removed by cleavage, and thus are not comprised in the mature antigen-binding molecule/polypeptide.

Signal peptides are known for many proteins, and are recorded in databases such as GenBank, UniProt, Swiss-Prot, TrEMBL, Protein Information Resource, Protein Data Bank, Ensembl, and InterPro, and/or can be identified/predicted *e.g.* using amino acid sequence analysis tools such as SignalP (Petersen et al., 2011 Nature Methods 8: 785-786) or Signal-BLAST (Frank and Sippl, 2008 Bioinformatics 24: 2172-2176).

Strategies to facilitate the transport (*i.e.,* crossing) of a given polypeptide/polypeptide complex across a blood-brain barrier are discussed in Bellettato and Scarpa (Italian Journal of Pediatrics volume 44, Article number: 131. 2018), which is hereby incorporated by reference in its entirety. Methods of engineering antibody and protein therapeutics to facilitate the crossing the of blood-brain barrier are reviewed by Zhao et al. (Antibody Therapeutics, 5(4), 2022, 311-331), which is hereby incorporated by reference in its entirety.

In some embodiments, antigen-binding molecules and polypeptides of the present disclosure may additionally comprise a moiety that facilitates the crossing of the blood-brain barrier into the brain, i.e., from the luminal side of the blood-brain barrier to the abluminal side of the blood-brain barrier. Such moieties may enhance/improve the ability of an antigen-binding molecule/polypeptide comprising such a moiety to cross the blood-brain barrier (*i.e.,* from the luminal side of the blood-brain barrier to the abluminal side of the blood-brain barrier). A moiety that facilitates the crossing of the blood-brain barrier may be effective to increase the number/proportion of antigen-binding molecules/polypeptides comprising the moiety that cross the blood-brain barrier, *e.g.,* compared to the number/proportion of the antigen-binding molecules/polypeptides that cross the blood-brain barrier in the absence of such a moiety.

In some embodiments, a moiety facilitating the crossing of the blood-brain barrier is a peptide/polypeptide moiety. In some embodiments, a moiety facilitating the crossing of the blood-brain barrier comprises or consists of an amino acid sequence. In some embodiments, the antigen-binding molecule/polypeptide of the present disclosure comprises a blood-brain barrier penetrating peptide, a blood-brain barrier-shuttle peptide, a branched blood-brain barrier-shuttle peptide, and/or a receptor-targeting sequence. In some embodiments, the antigen-binding molecule/polypeptide of the present disclosure comprises a blood-brain barrier penetrating peptide. In some embodiments, the antigen-binding molecule/polypeptide of the present disclosure comprises a blood-brain barrier-shuttle peptide. In some embodiments, the antigen-binding molecule/polypeptide of the present disclosure comprises a branched blood-brain barrier-shuttle peptide. In some embodiments, the antigen-binding molecule/polypeptide of the present disclosure comprises a receptor-targeting sequence.

Blood-brain barrier penetrating peptides (also known as brain penetrating peptides) are known to facilitate the crossing of the blood-brain barrier. Blood-brain barrier penetrating peptides are described in Chen et al, (Front. Genet., Vol 13. 2022) and Zhou et al. (Wiley Interdiscip Rev Nanomed Nanobiotechnol. 2021 Jul;13(4):e1695) which are both hereby incorporated by reference in their entirety. In some embodiments, blood-brain barrier penetrating peptides are identified based on the BBPpredict method of Chen et al, (Front. Genet., Vol 13. 2022). In some embodiments, the moiety facilitating the crossing of the blood-brain barrier is a blood-brain barrier penetrating peptide. In some embodiments, the moiety facilitating the crossing of the blood-brain barrier is a blood-brain barrier-shuttle peptide. In some embodiments, the moiety facilitating the crossing of the blood-brain barrier is a branched blood-brain barrier-shuttle peptide, such as a branched THRre peptide described in Diaz-Perlas et al. (Chem. Sci., 2018, 9, 8409), which is hereby incorporated by reference in its entirety.

In some embodiments, the moiety facilitating the crossing of the blood-brain barrier is a receptor-targeting moiety. In some embodiments, the receptor-targeting moiety is a receptor-targeting amino acid sequence moiety. In some embodiments, the receptor-targeting moiety is a receptor-targeting peptide/polypeptide moiety. In some embodiments, the receptor-targeting moiety facilitates receptor mediated transcytosis. In some embodiments, the receptor-targeting moiety interacts with a receptor capable of receptor mediated transcytosis. In some embodiments, a target receptor is a receptor capable of receptor mediated transcytosis. In some embodiments, a target receptor is a receptor expressed on the luminal side of the blood-brain barrier which is capable of receptor mediated transcytosis. In some embodiments, the receptor-targeting moiety is the ligand of a target receptor, an antigen-binding molecule which interacts with a target receptor, an antibody which interacts with a target receptor, and/or a mimetic which interacts with a target receptor.

In some embodiments, the receptor-targeting moiety binds/interacts with a receptor selected from the group consisting of: transferrin receptor (TfR), CD98hc, FC5 antibody binding receptor, insulin receptor (InsR), and LDL receptor. In some embodiments, the receptor-targeting sequence and/or receptor-targeting moiety interacts with TfR, CD98hc, FC5 antibody binding receptor, InsR, and/or LDL receptor. In some embodiments, the receptor-targeting sequence and/or receptor-targeting moiety binds TfR, CD98hc, FC5 antibody binding receptor, InsR, and/or LDL receptor. Receptor-targeting sequences are discussed in Pulgar (Front. Neurosci. 12:1019. 2019), which is hereby incorporated by reference in its entirety. In some embodiments, the receptor-targeting moiety binds/interacts with TfR. In some embodiments, the receptor-targeting moiety binds/interacts with CD98hc. In some embodiments, the receptor-targeting moiety binds/interacts with FC5 antibody binding receptor. In some embodiments, the receptor-targeting moiety binds/interacts with InsR. In some embodiments, the receptor-targeting moiety binds/interacts with LDL receptor.

### Labels and conjugates

In some embodiments, the antigen-binding molecule or polypeptide of the present disclosure comprises a detectable moiety.

In some embodiments, a detectable moiety is a fluorescent label, phosphorescent label, luminescent label, immuno-detectable label (*e.g.* an epitope tag), radiolabel, chemical, nucleic acid or enzymatic label. The antigen-binding molecule or polypeptide may be covalently or non-covalently labelled with the detectable moiety.

Fluorescent labels include *e.g.* fluorescein, rhodamine, allophycocyanin, eosine and NDB, green fluorescent protein (GFP), chelates of rare earths such as europium (Eu), terbium (Tb) and samarium (Sm), tetramethyl rhodamine, Texas Red, 4-methyl umbelliferone, 7-amino-4-methyl coumarin, Cy3, and Cy5. Radiolabels include radioisotopes such as Hydrogen³, Sulfur³⁵, Carbon¹⁴, Phosphorus³², Iodine¹²³, Iodine¹²⁵, Iodine¹²⁶, Iodine¹³¹, Iodine¹³³, Bromine⁷⁷, Technetium^{99m}, Indium¹¹¹, Indium^{113m}, Gallium⁶⁷, Gallium⁶⁸, Ruthenium⁹⁵, Ruthenium⁹⁷, Ruthenium¹⁰³, Ruthenium¹⁰⁵, Mercury²⁰⁷, Mercury²⁰³, Rhenium^{99m}, Rhenium¹⁰¹, Rhenium¹⁰⁵, Scandium⁴⁷, Tellurium^{121m}, Tellurium^{122m}, Tellurium^{125m,} Thulium¹⁶⁵, Thuliuml¹⁶⁷, Thulium¹⁶⁸, Copper, Fluorine¹⁸, Yttrium⁹⁰, Palladium¹⁰⁰, Bismuth²¹⁷ and Antimony²¹¹. Luminescent labels include as radioluminescent, chemiluminescent (*e.g*. acridinium ester, luminol, isoluminol) and bioluminescent labels. Immuno-detectable labels include haptens, peptides/polypeptides, antibodies, receptors and ligands such as biotin, avidin, streptavidin or digoxigenin. Nucleic acid labels include aptamers.

In some embodiments, the antigen-binding molecule/polypeptide comprises an epitope tag, *e.g.* a His, (*e.g.* 6XHis), FLAG, c-Myc, StrepTag, haemagglutinin, E, calmodulin-binding protein (CBP), glutathione-s-transferase (GST), maltose-binding protein (MBP), thioredoxin, S-peptide, T7 peptide, SH2 domain, avidin, streptavidin, and haptens (*e.g.* biotin, digoxigenin, dinitrophenol), optionally at the N- or C- terminus of the antigen-binding molecule/polypeptide.

In some embodiments, the antigen-binding molecule/polypeptide comprises a moiety having a detectable activity, *e.g.* an enzymatic moiety. Enzymatic moieties include *e.g.* luciferases, glucose oxidases, galactosidases (*e.g.* beta-galactosidase), glucorinidases, phosphatases (*e.g.* alkaline phosphatase), peroxidases (*e.g.* horseradish peroxidase) and cholinesterases.

In some embodiments, the antigen-binding molecule or polypeptide of the present disclosure comprises a chemical moiety. In some embodiments, the antigen-binding molecule/polypeptide of the present disclosure is conjugated to a chemical moiety.

The chemical moiety may be a moiety for providing a therapeutic effect, *i.e.* a drug moiety. A drug moiety may be a small molecule (*e.g.* a low molecular weight (< 1000 daltons, typically between ~300-700 daltons) organic compound). Drug moieties are described *e.g.* in Parslow et al., Biomedicines. 2016 Sep; 4(3):14 (hereby incorporated by reference in its entirety). In some embodiments, a drug moiety may be or comprise a cytotoxic agent. In some embodiments, a drug moiety may be or comprise a chemotherapeutic agent. Drug moieties include *e.g.* calicheamicin, DM1, DM4, monomethylauristatin E (MMAE), monomethylauristatin F (MMAF), SN-38, doxorubicin, duocarmycin, D6.5 and PBD.

In some embodiments, antigen-binding molecules and polypeptides of the present disclosure may be conjugated to a moiety that facilitates the crossing of the blood-brain barrier to the brain, *i.e.,* from the luminal side of the blood-brain barrier to the abluminal side of the blood-brain barrier. Such moieties may enhance/improve the ability of an antigen-binding molecule/polypeptide comprising such a moiety to cross the blood-brain barrier (*i.e.,* from the luminal side of the blood-brain barrier to the abluminal side of the blood-brain barrier). A moiety that facilitates the crossing of the blood-brain barrier may be effective to increase the number/proportion of antigen-binding molecules/polypeptides conjugated to the moiety that cross the blood-brain barrier, *e.g.,* compared to the number/proportion of the antigen-binding molecules/polypeptides that cross the blood-brain barrier in the absence of such a moiety.

In some embodiments, the antigen-binding molecule/polypeptide of the present disclosure is conjugated to a moiety which facilitates the crossing of the blood-brain barrier. In some embodiments, the antigen-binding molecule/polypeptide of the present disclosure is conjugated to a peptide/polypeptide which facilitates the crossing of the blood-brain barrier. In some embodiments, the antigen-binding molecule/polypeptide of the present disclosure is conjugated to a blood-brain barrier penetrating peptide, a blood-brain barrier-shuttle peptide, a branched blood-brain barrier-shuttle peptide, a branched THRre peptide, and/or a receptor-targeting moiety. In some embodiments, the antigen-binding molecule/polypeptide of the present disclosure is conjugated to a blood-brain barrier penetrating peptide. In some embodiments, the antigen-binding molecule/polypeptide of the present disclosure is conjugated to a blood-brain barrier-shuttle peptide. In some embodiments, the antigen-binding molecule/polypeptide of the present disclosure is conjugated to a branched blood-brain barrier-shuttle peptide. In some embodiments, the antigen-binding molecule/polypeptide of the present disclosure is conjugated to a branched THRre peptide. In some embodiments, the antigen-binding molecule and/or polypeptide of the present disclosure is conjugated to a receptor-targeting moiety.

### Nucleic acids and vectors

The present disclosure provides a nucleic acid, or a plurality of nucleic acids, encoding an antigen-binding molecule or polypeptide according to the present disclosure. In some embodiments, the nucleic acid(s) comprise or consist of DNA and/or RNA.

An antigen-binding molecule or polypeptide according to the present disclosure may be produced within a cell by translation of RNA encoding the polypeptide(s). An antigen-binding molecule or polypeptide according to the present disclosure may be produced within a cell by transcription from nucleic acid encoding the polypeptide(s), and subsequent translation of the transcribed RNA.

In some embodiments, the nucleic acid(s) may be, or may be comprised/contained in, a vector, or a plurality of vectors. A 'vector' as used herein is a nucleic acid molecule used as a vehicle to transfer exogenous nucleic acid into a cell.

Accordingly, the present disclosure also provides a vector, or plurality of vectors, comprising the nucleic acid or plurality of nucleic acids according to the present disclosure. The vector may facilitate delivery of the nucleic acid(s) encoding a polypeptide according to the present disclosure to a cell. The vector may be an expression vector comprising elements required for expressing a polypeptide according to the present disclosure. The vector may comprise elements facilitating integration of the nucleic acid(s) into the genomic DNA of cell into which the vector is introduced.

Nucleic acids and vectors according to the present disclosure may be provided in purified or isolated form, i.e. from other nucleic acid, or naturally-occurring biological material.

A vector may be a vector for expression of the nucleic acid in the cell (*i.e.* an expression vector). Such vectors may include a promoter sequence operably linked to a nucleotide sequence encoding an antigen-binding molecule or polypeptide according to the present disclosure. A vector may also include a termination codon (*i.e.* 3' in the nucleotide sequence of the vector to the nucleotide sequence encoding the polypeptide(s)) and expression enhancers. Any suitable vectors, promoters, enhancers and termination codons known in the art may be used to express a peptide or polypeptide from a vector according to the present disclosure.

The term 'operably linked' may include the situation where nucleic acid encoding a polypeptide according to the present disclosure and regulatory nucleic acid sequence(s) (*e.g.* a promoter and/or enhancers) are covalently linked in such a way as to place the expression of the nucleic acid encoding a polypeptide under the influence or control of the regulatory nucleic acid sequence(s) (thereby forming an expression cassette). Thus, a regulatory sequence is operably linked to the selected nucleic acid sequence if the regulatory sequence is capable of effecting transcription of the nucleic acid sequence. The resulting transcript(s) may then be translated into the desired polypeptide(s).

Vectors contemplated in connection with the present disclosure include DNA vectors, RNA vectors, plasmids (*e.g.* conjugative plasmids (*e.g.* F plasmids), non-conjugative plasmids, R plasmids, col plasmids, episomes), viral vectors (*e.g.* retroviral vectors, *e.g.* gammaretroviral vectors (*e.g.* murine Leukemia virus (MLV)-derived vectors, *e.g.* SFG vector), lentiviral vectors, adenovirus vectors, adeno-associated virus vectors, vaccinia virus vectors and herpesvirus vectors), transposon-based vectors, and artificial chromosomes (*e.g.* yeast artificial chromosomes), *e.g.* as described in Maus etal., Annu Rev Immunol (2014) 32:189-225 and Morgan and Boyerinas, Biomedicines (2016) 4:9, which are both hereby incorporated by reference in their entirety. In some embodiments, a vector according to the present disclosure is a lentiviral vector.

In some embodiments, the vector may be a eukaryotic vector, *i.e.* a vector comprising the elements necessary for expression of protein from the vector in a eukaryotic cell. In some embodiments, the vector may be a mammalian vector, *e.g.* comprising a cytomegalovirus (CMV) or SV40 promoter to drive protein expression.

Constituent polypeptides of an antigen-binding molecule according to the present disclosure may be encoded by different nucleic acids of the plurality of nucleic acids, or by different vectors of the plurality of vectors.

### Producing the antigen-binding molecules and polypeptides

Antigen-binding molecules and polypeptides according to the present disclosure may be prepared according to methods for the production of polypeptides known to the skilled person.

Antigen-binding molecules and polypeptides may be prepared by chemical synthesis, *e.g.* liquid or solid phase synthesis. For example, peptides/polypeptides can be synthesized using the methods described in, for example, Chandrudu etal., Molecules (2013), 18: 4373-4388, which is hereby incorporated by reference in its entirety.

Alternatively, antigen-binding molecules and polypeptides may be produced by recombinant expression. Molecular biology techniques suitable for recombinant production of polypeptides are well known in the art, such as those set out in Green and Sambrook, Molecular Cloning: A Laboratory Manual (4th Edition), Cold Spring Harbor Press, 2012, and in Nat Methods. (2008); 5(2): 135-146 both of which are hereby incorporated by reference in their entirety. Methods for the recombinant production of antigen-binding molecules are also described in Frenzel et al., Front Immunol. (2013); 4: 217 and Kunert and Reinhart, Appl Microbiol Biotechnol. (2016) 100: 3451-3461, both of which are hereby incorporated by reference in their entirety.

In some cases, the antigen-binding molecules of the present disclosure are comprised of more than one polypeptide chain. In such cases, production of the antigen-binding molecule may comprise transcription and translation of more than one polypeptide, and subsequent association of the polypeptide chains to form the antigen-binding molecule.

For recombinant production according to the present disclosure, any cell suitable for the expression of polypeptides may be used. The cell may be a prokaryote or eukaryote. In some embodiments, the cell is a prokaryotic cell, such as a cell of archaea or bacteria. In some embodiments, the bacteria may be Gram-negative bacteria such as bacteria of the family Enterobacteriaceae, for example *Escherichia coli.* In some embodiments, the cell is a eukaryotic cell such as a yeast cell, a plant cell, insect cell or a mammalian cell, *e.g.* a cell described hereinabove.

In some cases, the cell is not a prokaryotic cell because some prokaryotic cells do not allow for the same folding or post-translational modifications as eukaryotic cells. In addition, very high expression levels are possible in eukaryotes and proteins can be easier to purify from eukaryotes using appropriate tags. Specific plasmids may also be utilized which enhance secretion of the protein into the media.

In some embodiments polypeptides may be prepared by cell-free-protein synthesis (CFPS), *e.g.* according to a system described in Zemella et al. Chembiochem (2015) 16(17): 2420-2431, which is hereby incorporated by reference in its entirety.

Production may involve culture or fermentation of a eukaryotic cell modified to express the polypeptide(s) of interest. The culture or fermentation may be performed in a bioreactor provided with an appropriate supply of nutrients, air/oxygen and/or growth factors. Secreted proteins can be collected by partitioning culture media/fermentation broth from the cells, extracting the protein content, and separating individual proteins to isolate secreted polypeptide(s). Culture, fermentation and separation techniques are well known to those of skill in the art, and are described, for example, in Green and Sambrook, Molecular Cloning: A Laboratory Manual (4th Edition; incorporated by reference herein above).

Bioreactors include one or more vessels in which cells may be cultured. Culture in the bioreactor may occur continuously, with a continuous flow of reactants into, and a continuous flow of cultured cells from, the reactor. Alternatively, the culture may occur in batches. The bioreactor monitors and controls environmental conditions such as pH, oxygen, flow rates into and out of, and agitation within the vessel such that optimum conditions are provided for the cells being cultured.

Following culturing the cells that express the polypeptide(s), the polypeptide(s) of interest may be isolated. Any suitable method for separating proteins from cells known in the art may be used. In order to isolate the polypeptide, it may be necessary to separate the cells from nutrient medium. If the polypeptide(s) are secreted from the cells, the cells may be separated by centrifugation from the culture media that contains the secreted polypeptide(s) of interest. If the polypeptide(s) of interest collect within the cell, protein isolation may comprise centrifugation to separate cells from cell culture medium, treatment of the cell pellet with a lysis buffer, and cell disruption *e.g.* by sonification, rapid freeze-thaw or osmotic lysis.

It may then be desirable to isolate the polypeptide(s) of interest from the supernatant or culture medium, which may contain other protein and non-protein components. A common approach to separating protein components from a supernatant or culture medium is by precipitation. Proteins of different solubilities are precipitated at different concentrations of precipitating agent such as ammonium sulfate. For example, at low concentrations of precipitating agent, water soluble proteins are extracted. Thus, by adding different increasing concentrations of precipitating agent, proteins of different solubilities may be distinguished. Dialysis may be subsequently used to remove ammonium sulfate from the separated proteins.

Other methods for distinguishing different proteins are known in the art, for example ion exchange chromatography and size chromatography. These may be used as an alternative to precipitation or may be performed subsequently to precipitation.

Once the polypeptide(s) of interest have been isolated from culture it may be desired or necessary to concentrate the polypeptide(s). A number of methods for concentrating proteins are known in the art, such as ultrafiltration or lyophilisation.

### Cells comprising/expressing the antigen-binding molecules and polypeptides

The present disclosure also provides a cell comprising or expressing an antigen-binding molecule or polypeptide according to the present disclosure. Also provided is a cell comprising or expressing a nucleic acid, a plurality of nucleic acids, a vector or a plurality of vectors according to the present disclosure.

It will be appreciated that where cells are referred to herein in the singular (*i.e.* 'a/the cell'), pluralities/populations of such cells are also contemplated.

The cell may be a eukaryotic cell, *e.g.* a mammalian cell. The mammal may be a primate (rhesus, cynomolgous, non-human primate or human) or a non-human mammal (*e.g.* rabbit, guinea pig, rat, mouse or other rodent (including any animal in the order Rodentia), cat, dog, pig, sheep, goat, cattle (including cows, *e.g.* dairy cows, or any animal in the order Bos), horse (including any animal in the order Equidae), donkey, and non-human primate).

In some embodiments, the cell is, or is derived from, a cell type commonly used for the expression of polypeptides for use in therapy in humans. Exemplary cells are described *e.g.* in Kunert and Reinhart, Appl Microbiol Biotechnol. (2016) 100:3451-3461 (hereby incorporated by reference in its entirety), and include *e.g.* CHO, HEK 293, PER.C6, NS0 and BHK cells. In preferred embodiments, the cell is, or is derived from, a CHO cell.

The present disclosure also provides a method for producing a cell comprising a nucleic acid(s) or vector(s) according to the present disclosure, comprising introducing a nucleic acid, a plurality of nucleic acids, a vector or a plurality of vectors according to the present disclosure into a cell. In some embodiments, introducing an isolated nucleic acid(s) or vector(s) according to the present disclosure into a cell comprises transformation, transfection, electroporation or transduction (*e.g.* retroviral transduction).

The present disclosure also provides a method for producing a cell expressing/comprising an antigen-binding molecule or polypeptide according to the present disclosure, comprising introducing a nucleic acid, a plurality of nucleic acids, a vector or a plurality of vectors according to the present disclosure in a cell. In some embodiments, the methods additionally comprise culturing the cell under conditions suitable for expression of the nucleic acid(s) or vector(s) by the cell. In some embodiments, the methods are performed *in vitro.*

The present disclosure also provides cells obtained or obtainable by the methods according to the present disclosure.

### Compositions

The present disclosure also provides compositions comprising the antigen-binding molecules, polypeptides, nucleic acids, expression vectors and cells described herein.

The antigen-binding molecules, polypeptides, nucleic acids, expression vectors and cells described herein may be formulated as pharmaceutical compositions or medicaments for clinical use and may comprise a pharmaceutically acceptable carrier, diluent, excipient or adjuvant. Thus, the present disclosure also provides a pharmaceutical composition/medicament comprising an antigen-binding molecule, polypeptide, nucleic acid/plurality, expression vector/plurality or cell described herein.

The compositions of the present disclosure may comprise one or more pharmaceutically-acceptable carriers (*e.g.* liposomes, micelles, microspheres, nanoparticles), diluents/excipients (*e.g.* starch, cellulose, a cellulose derivative, a polyol, dextrose, maltodextrin, magnesium stearate), adjuvants, fillers, buffers, preservatives (*e.g.* vitamin A, vitamin E, vitamin C, retinyl palmitate, selenium, cysteine, methionine, citric acid, sodium citrate, methyl paraben, propyl paraben), anti-oxidants (*e.g.* vitamin A, vitamin E, vitamin C, retinyl palmitate, selenium), lubricants (*e.g.* magnesium stearate, talc, silica, stearic acid, vegetable stearin), binders (*e.g.* sucrose, lactose, starch, cellulose, gelatin, polyethylene glycol (PEG), polyvinylpyrrolidone (PVP), xylitol, sorbitol, mannitol), stabilisers, solubilisers, surfactants (*e.g.,* wetting agents), masking agents or colouring agents (*e.g.* titanium oxide).

The term 'pharmaceutically-acceptable' as used herein pertains to compounds, ingredients, materials, compositions, dosage forms, *etc.,* which are, within the scope of sound medical judgment, suitable for use in contact with the tissues of the subject in question (*e.g.* a human subject) without excessive toxicity, irritation, allergic response, or other problem or complication, commensurate with a reasonable benefit/risk ratio. Each carrier, diluent, excipient, adjuvant, filler, buffer, preservative, antioxidant, lubricant, binder, stabiliser, solubiliser, surfactant, masking agent, colouring agent, flavouring agent or sweetening agent of a composition according to the present disclosure must also be 'acceptable' in the sense of being compatible with the other ingredients of the formulation. Suitable carriers, diluents, excipients, adjuvants, fillers, buffers, preservatives, anti-oxidants, lubricants, binders, stabilisers, solubilisers, surfactants, masking agents, colouring agents, flavouring agents or sweetening agents can be found in standard pharmaceutical texts, for example, Remington's 'The Science and Practice of Pharmacy' (Ed. A. Adejare), 23rd Edition (2020), Academic Press.

Compositions may be formulated for topical, parenteral, systemic, intracavitary, intravenous, intra-arterial, intramuscular, intrathecal, intraocular, intraconjunctival, intratumoral, subcutaneous, intradermal, intrathecal, oral or transdermal routes of administration. In some embodiments, a pharmaceutical composition/medicament may be formulated for administration by injection or infusion, or administration by ingestion.

Suitable formulations may comprise the relevant article in a sterile or isotonic medium. Medicaments and pharmaceutical compositions may be formulated in fluid, including gel, form. Fluid formulations may be formulated for administration by injection or infusion (*e.g.* via catheter) to a selected region of the human or animal body.

In some embodiments, the composition is formulated for injection or infusion, *e.g.* into a blood vessel, tissue/organ of interest, or a tumor.

The present disclosure also provides methods for the production of pharmaceutically-useful compositions and medicaments. Such methods may comprise one or more steps selected from: producing an antigen-binding molecule, polypeptide, nucleic acid (or plurality thereof), expression vector (or plurality thereof) or cell described herein; isolating an antigen-binding molecule, polypeptide, nucleic acid (or plurality thereof), expression vector (or plurality thereof) or cell described herein; and/or mixing an antigen-binding molecule, polypeptide, nucleic acid (or plurality thereof), expression vector (or plurality thereof) or cell described herein with a pharmaceutically-acceptable carrier, adjuvant, excipient or diluent.

For example, a further aspect of the present disclosure relates to a method of formulating or producing a medicament or pharmaceutical composition for use in the treatment of a disease/condition (*e.g.* a disease/condition described herein), the method comprising formulating a pharmaceutical composition or medicament by mixing an antigen-binding molecule, polypeptide, nucleic acid (or plurality thereof), expression vector (or plurality thereof) or cell described herein with a pharmaceutically acceptable carrier, adjuvant, excipient or diluent.

### Therapeutic and prophylactic applications

The antigen-binding molecules, polypeptides, nucleic acids, expression vectors, cells and compositions described herein find use in therapeutic and prophylactic methods.

The present disclosure provides an antigen-binding molecule, polypeptide, nucleic acid (or plurality thereof), expression vector (or plurality thereof), cell or composition described herein for use in a method of medical treatment or prophylaxis. Also provided is an antigen-binding molecule, polypeptide, nucleic acid (or plurality thereof), expression vector (or plurality thereof), cell or composition described herein for use in a method of treating or preventing a disease or condition described herein. Also provided is the use of an antigen-binding molecule, polypeptide, nucleic acid (or plurality thereof), expression vector (or plurality thereof), cell or composition described herein in the manufacture of a medicament for treating or preventing a disease or condition described herein. Also provided is a method of treating or preventing a disease or condition described herein, comprising administering to a subject a therapeutically or prophylactically effective amount of an antigen-binding molecule, polypeptide, nucleic acid (or plurality thereof), expression vector (or plurality thereof), cell or composition described herein.

The methods may be effective to reduce the development or progression of a disease/condition, alleviation of the symptoms of a disease/condition or reduction in the pathology of a disease/condition. The methods may be effective to prevent progression of the disease/condition, e.g. to prevent worsening of, or to slow the rate of development of, the disease/condition. In some embodiments, the methods may lead to an improvement in the disease/condition, *e.g.* a reduction in the symptoms of the disease/condition or reduction in some other correlate of the severity/activity of the disease/condition. In some embodiments, the methods may prevent development of the disease/condition a later stage (*e.g.* a chronic stage or metastasis).

The experimental examples of the present disclosure describe the identification and functional characterization of C1q-binding antibodies having a unique profile of functional properties as compared to known C1q-binding antibodies. In particular, the C1q-binding antibodies of the present disclosure are demonstrated to be useful to bias polarization of macrophages to away from the immunosuppressive M2 phenotype, and towards the pro-inflammatory M1 phenotype.

It will therefore be appreciated that the articles of the present disclosure find use in the treatment/prevention of essentially any disease/condition that would derive therapeutic or prophylactic benefit from a reduction in the number/proportion or activity of macrophages having an M2 or M2-like phenotype.

For example, the disease/condition may be a disease/condition in which an increased number/proportion or activity of macrophages having an M2 or M2-like phenotype is positively associated with the onset, development or progression of the disease/condition, and/or severity of one or more symptoms of the disease/condition. In some embodiments, an increased number/proportion or activity of macrophages having an M2 or M2-like phenotype may be a risk factor for the onset, development or progression of the disease/condition.

Accordingly, in some aspects and embodiments, the disease/condition to be treated/prevented in accordance with the present disclosure is a disease/condition in which M2 macrophages and/or M2-like macrophages are pathologically-implicated.

A disease/condition in which M2 macrophages and/or M2-like macrophages are pathologically-implicated may be characterized by one or more of the following:
an increase in the number/proportion/activity of M2 macrophages and/or M2-like macrophages, *e.g.* as compared to the level/number/proportion/activity in the absence of the disease/condition (*e.g.* in a healthy subject, or in equivalent non-diseased tissue);
a decrease in the number/proportion/activity of M1 macrophages and/or M1-like macrophages, *e.g.* as compared to the level/number/proportion/activity in the absence of the disease/condition (*e.g.* in a healthy subject, or in equivalent non-diseased tissue);
an increase in the number/proportion/activity of perivascular macrophages (*e.g.* perivascular macrophages having an M2 or M2-like phenotype), *e.g.* as compared to the level/number/proportion/activity in the absence of the disease/condition (*e.g.* in a healthy subject, or in equivalent non-diseased tissue);
an increase in the number/proportion/activity of IL-10-expressing macrophages, *e.g.* as compared to the level/number/proportion/activity in the absence of the disease/condition (*e.g.* in a healthy subject, or in equivalent non-diseased tissue);
an increase in the level of efferocytosis by macrophages, *e.g.* as compared to the level observed in the absence of the disease/condition (*e.g.* in a healthy subject, or in equivalent non-diseased tissue);
an increase in the number/proportion/activity of activated endothelial cells, *e.g.* as compared to the level/number/proportion/activity in the absence of the disease/condition (*e.g.* in a healthy subject, or in equivalent non-diseased tissue); and/or
the presence of tumor-associated macrophages (*e.g.* tumor-associated macrophages having an M2 or M2-like phenotype).

The development/polarization of macrophages to M2 and M2-like phenotypes is induced by factors produced by T cells having the Th2 phenotype. Th2 cells promote M2 macrophages via production of M-CSF, IL-4, IL-10 and IL-13 (see Atri etal., Int J Mol Sci. (2018) 19(6): 1801). M2 and M2-like macrophages moreover produce IL-4, IL-10 and IL-13, thereby amplifying Th2 responses. It will therefore be appreciated that the articles of the present disclosure find use in the treatment/prevention of diseases/conditions in which Th2 cells are pathologically-implicated. The role of Th2 cells in health and disease is reviewed *e.g.* in Bertschi etal., Int Arch Allergy Immunol (2021) 182 (5): 365-380, which is hereby incorporated by reference in its entirety.

In some embodiments, a disease/condition to be treated in accordance with the present disclosure is selected from: a cancer, fibrosis, a fibrotic disease (*e.g.* pulmonary fibrosis, liver fibrosis, systemic sclerosis), an inflammatory disease of the airways/lungs (*e.g.* asthma, chronic obstructive pulmonary disease), an allergic condition (*e.g.* allergic asthma, allergic rhinitis, allergic conjunctivitis, atopic dermatitis, food allergy) and a neurological disease (*e.g.* a neurological disease in which synaptic pruning is pathologically-implicated; *e.g.* Alzheimer's disease, schizophrenia, Rett syndrome or multiple sclerosis).

The role of M2 and M2-like macrophages in cancers is reviewed e.g. in Mantovani et al. (J Exp Med. (2015) 212(4):435-445), Sica et al. (Eur J Cancer. 2006;42(6):717-727) and Shapouri-Moghaddam et al. (J Cell Physiol. 2018;233(9):6425-6440), all of which are hereby incorporated by reference in their entirety.

Macrophages having an M2/M2-like phenotype contribute to the development and progression of cancers through various different mechanisms. M2 and M2-like macrophages produce cytokines and growth factors such as IL-10 and TGFβ, which promote the survival, proliferation, and migration of cancer cells, and which also suppress T cell-mediated anti-cancer immune responses, and promote the expansion of immunosuppressive Tregs. TAMs having an M2/M2-like phenotype are important effector cells for establishing the immunosuppressive tumor microenvironment (TME). Such cells inhibit the activity of anti-cancer effector T cells and NK cells, through the production of immunosuppressive soluble mediators, and expression of immune-checkpoint molecules, which promote T cell exhaustion. Macrophages having an M2/M2-like phenotype also produce VEGF, promoting angiogenesis and neovascularization and thereby promoting tumor growth and metastasis. They also secrete proteases, which along with TGFβ promote extracellular matrix remodeling, facilitating tissue invasion and metastasis. Moreover, studies of efferocytosis-mediated regulation of the TME suggest that efferocytosis represents an immune checkpoint that is exploited by tumors to evade anti-tumor immunity (Vaught et al., Cancer Cell Microenviron. 2015; 2(1): e666).

Accordingly, in some aspects and embodiments, the disease/condition to be treated/prevented in accordance with the present disclosure is a cancer. As used herein, a 'cancer' may be or comprise any unwanted cell proliferation (or any disease manifesting itself by unwanted cell proliferation), neoplasm or tumor. The cancer may be benign or malignant. The cancer may be primary or secondary (metastatic). A neoplasm or tumor may be any abnormal growth or proliferation of cells and may be located in any tissue. The cancer may be of tissues/cells derived from *e.g.* the adrenal gland, adrenal medulla, anus, appendix, bladder, blood, bone, bone marrow, brain, breast, cecum, central nervous system (including or excluding the brain) cerebellum, cervix, colon, duodenum, endometrium, epithelial cells (*e.g.* renal epithelia), gallbladder, biliary tract, oesophagus, glial cells, heart, ileum, jejunum, kidney, lacrimal glad, larynx, liver, lung, lymph, lymph node, lymphoblast, maxilla, mediastinum, mesentery, myometrium, nasopharynx, omentum, oral cavity, ovary, pancreas, parotid gland, peripheral nervous system, peritoneum, pleura, prostate, salivary gland, sigmoid colon, skin, small intestine, soft tissues, spleen, stomach, testis, thymus, thyroid gland, tongue, tonsil, trachea, uterus, vulva or white blood cells.

Tumors to be treated may be nervous or non-nervous system tumors. Nervous system tumors may originate either in the central or peripheral nervous system, *e.g.* glioma, medulloblastoma, meningioma, neurofibroma, ependymoma, Schwannoma, neurofibrosarcoma, astrocytoma and oligodendroglioma. Non-nervous system cancers/tumors may originate in any other non-nervous tissue; examples include melanoma, mesothelioma, lymphoma, myeloma, leukemia, Non-Hodgkin's lymphoma (NHL), Hodgkin's lymphoma, chronic myelogenous leukemia (CML), acute myeloid leukemia (AML), myelodysplastic syndrome (MDS), cutaneous T-cell lymphoma (CTCL), chronic lymphocytic leukemia (CLL), hepatoma, epidermoid carcinoma, prostate carcinoma, breast cancer, lung cancer, colon cancer, ovarian cancer, pancreatic cancer, thymic carcinoma, NSCLC, hematologic cancer and sarcoma.

It will be appreciated that therapeutic and prophylactic utility of the articles of the present disclosure extends to essentially all cancers, as they would reasonably derive therapeutic/prophylactic benefit from the release of anti-cancer immune effectors from suppression mediated by macrophages having an M2/M2-like phenotype.

In some embodiments, a cancer is selected from: a solid tumor, a hematological cancer, a squamous cell cancer, breast cancer, breast carcinoma, breast invasive carcinoma, ductal carcinoma, metastatic breast cancer, triple-negative breast cancer, gastric cancer, gastric carcinoma, gastric adenocarcinoma, gastrointestinal adenocarcinoma, colorectal cancer, metastatic colorectal cancer, colon cancer, colorectal carcinoma, colorectal adenocarcinoma, colon adenocarcinoma, head and neck cancer, head and neck squamous cell carcinoma (HNSCC), lung cancer, non-small cell lung cancer, lung adenocarcinoma, invasive mucinous lung adenocarcinoma, lung squamous cell carcinoma (LUSC), ovarian cancer, ovarian carcinoma, ovarian serous adenocarcinoma, ovarian serous cystadenocarcinoma, fallopian tube cancer, renal cancer, renal cell carcinoma, renal clear cell carcinoma, renal cell adenocarcinoma, renal papillary cell carcinoma, pancreatic cancer, pancreatic adenocarcinoma, pancreatic ductal adenocarcinoma, cervical cancer, cervical squamous cell carcinoma, skin cancer, melanoma, oral cavity cancer, oropharyngeal cancer, esophageal cancer, esophageal squamous cell carcinoma (ESCC), esophageal adenocarcinoma, liver cancer, hepatocellular carcinoma, cholangiocarcinoma, gallbladder cancer, uterine cancer, endometrial cancer, uterine corpus endometrial carcinoma, uterine carcinosarcoma, thyroid cancer, thyroid cancer, thyroid carcinoma, pheochromocytoma, paraganglioma, bladder cancer, bladder urothelial carcinoma, prostate cancer, prostate adenocarcinoma, retinoblastoma, sarcoma, soft tissue sarcoma, peritoneal cancer, thymoma, neuroendocrine tumor, neuroendocrine tumor of the nasopharynx, squamous cell carcinoma of the skin, astrocytoma, low grade astrocytoma, high grade astrocytoma, anaplastic astrocytoma and glioblastoma multiforme.

In some embodiments, a cancer is selected from: lung cancer (*e.g.* non-small cell lung cancer), skin cancer (*e.g.* melanoma), head and neck cancer (*e.g.* head and neck squamous cell carcinoma), liver cancer (*e.g.* hepatocellular carcinoma), glioblastoma, colorectal cancer, cervical cancer and breast cancer.

Fibrosis is a form of pathologic tissue remodeling characterized by the formation of excess connective tissue as a consequence of the excess deposition of extracellular matrix (ECM) components (including collagen). 'Excess connective tissue' refers to an amount of connective tissue at a given location (*e.g.* a given tissue/organ, or part of a given tissue/organ) which is greater than the amount of connective tissue present at that location under normal, non-pathological conditions. Similarly, 'excess deposition of ECM components' refers to a level of deposition of one or more ECM components which is greater than the level of deposition under normal, non-pathological conditions. The cellular and molecular mechanisms of fibrosis are described in Wynn, J. Pathol. (2008) 214(2): 199-210, and Wynn and Ramalingam, Nature Medicine (2012) 18:1028-1040, both of which are hereby incorporated by reference in their entirety.

The main cellular effectors of fibrosis are myofibroblasts. In response to tissue injury, damaged cells and leukocytes produce pro-fibroinflammatory factors such as TGFβ, IL-13 and PDGF, which activate fibroblasts (and other myofibroblast precursor cells) to become αSMA-expressing myofibroblasts, and recruit myofibroblasts to the site of injury. Myofibroblasts produce large amounts of extracellular matrix components such as collagen and periostin for wound contracture and closure, and also produce proinflammatory cytokines such as IL-6, and tissue remodeling factors such as MMP2 and TIMP1. Persistent/chronic infection and/or inflammation can result in the generation of too many myofibroblasts, and consequently the over-production of extracellular matrix, resulting in fibrosis. In many diseases and conditions characterized by fibrosis, a persistent inflammatory trigger is crucial to upregulation of production of growth factors, proteolytic enzymes, angiogenic factors and fibrogenic cytokines, which stimulate the deposition of connective tissue elements that progressively remodel and destroy normal tissue architecture.

The role of M2 and M2-like macrophages in fibrosis is reviewed *e.g.* in Wynn and Vannella, Immunity. (2016) 44(3):450-462, Murray and Wynn, Nat Rev Immunol. (2011) 11(11):723-737 and Distler et al., Nat Rev Rheumatol. (2019) 15(12):705-730, all of which are hereby incorporated by reference in their entirety.

Macrophages having an M2/M2-like phenotype can be activated in response to tissue injury or chronic inflammation (*e.g.* in the context of chronic infection, autoimmune disease or cancer). They release pro-fibrotic factors such TGFβ and platelet-derived growth factor (PDGF), which stimulate fibroblasts to become pro-fibrotic myofibroblasts. Myofibroblasts produce large amounts of extracellular matrix components including collagen, resulting in fibrosis. M2/M2-like macrophages also directly produce extracellular matrix components such as collagen, and also extracellular matrix-remodeling enzymes such as matrix metalloproteinases (MMPs), which further contribute to fibrosis. The immunomodulatory activity of M2/M2-like macrophages can compromise clearance elimination of the initiators of inflammation (*e.g.* infectious agents, allergens) resulting in chronic inflammation and thereby promoting fibrosis.

Accordingly, in some aspects and embodiments, the disease/condition to be treated/prevented in accordance with the present disclosure is fibrosis, or a disease/condition characterized by fibrosis.

As used herein, a disease/condition which is 'characterized by fibrosis' is a disease/condition in which fibrosis is a symptom of the disease/condition. Diseases and conditions characterized by fibrosis include, but are not limited to:
Diseases/conditions affecting the respiratory system such as pulmonary fibrosis, fibrothorax, radiation-induced lung injury, interstitial lung disease (ILD), idiopathic interstitial pneumonia (IIP), idiopathic pulmonary fibrosis (IPF), cystic fibrosis, progressive massive fibrosis, scleroderma, obliterative bronchiolitis, Hermansky-Pudlak syndrome, asbestosis, silicosis, sarcoidosis, tumor stroma in lung disease, chronic obstructive pulmonary disease (COPD), emphysema, chronic bronchitis and asthma;
Diseases/conditions affecting the liver such as chronic liver disease, liver fibrosis, bridging fibrosis, cirrhosis, non-alcoholic fatty liver disease (NAFLD), steatohepatitis, non-alcoholic steatohepatitis (NASH), alcoholic liver disease (ALD), alcoholic fatty liver (AFL), alcoholic hepatitis, alcoholic steatohepatitis (ASH), primary biliary cirrhosis (PBC), schistosomal liver disease and hepatocellular carcinoma (HCC);
Diseases/conditions affecting the cardiovascular system such as hypertrophic cardiomyopathy (HCM), dilated cardiomyopathy (DCM), fibrosis of the atrium, atrial fibrillation, fibrosis of the ventricle, ventricular fibrillation, myocardial fibrosis, interstitial fibrosis, replacement fibrosis Brugada syndrome, myocarditis, endomyocardial fibrosis, myocardial infarction, fibrotic vascular disease, hypertension, hypertensive heart disease, arrhythmogenic right ventricular cardiomyopathy (ARVC), atherosclerosis, arterial stiffness, chronic pulmonary hypertension, AIDS-associated pulmonary hypertension, heart failure with preserved ejection fraction (HFpEF), heart failure with reduced ejection fraction (HFrEF), varicose veins and cerebral infarcts;
Diseases/conditions affecting the kidneys such as tubulointerstitial fibrosis, glomerular fibrosis, renal fibrosis, nephritic syndrome, Alport's syndrome, HIV-associated nephropathy, polycystic kidney disease, Fabry's disease, diabetic nephropathy, chronic glomerulonephritis and nephritis associated with systemic lupus;
Diseases/conditions affecting the pancreas such as pancreatic fibrosis, cystic fibrosis and chronic pancreatitis;
Diseases/conditions affecting the nervous system such as gliosis, Alzheimer's disease and multiple sclerosis;
Diseases/conditions affecting the musculoskeletal system such as muscular dystrophy, Duchenne muscular dystrophy (DMD), Becker's muscular dystrophy (BMD) and fibrotic myopathy;
Diseases/conditions affecting the gastrointestinal system such as inflammatory bowel disease (IBD), Crohn's disease, microscopic colitis and primary sclerosing cholangitis (PSC);
Diseases/conditions affecting the skin such as scleroderma, nephrogenic systemic fibrosis, Dupuytren's contracture and cutis keloid;
Diseases/conditions affecting the eye such as Grave's opthalmopathy, epiretinal fibrosis, retinal fibrosis, subretinal fibrosis, subretinal fibrosis associated with macular degeneration (*e.g.* wet age-related macular degeneration (AMD)), diabetic retinopathy, glaucoma, corneal fibrosis, post-surgical fibrosis (*e.g.* of the posterior capsule following cataract surgery, or of the bleb following trabeculectomy for glaucoma), conjunctival fibrosis and subconjunctival fibrosis;
Diseases/conditions affecting the joints such as arthrofibrosis, arthritis and adhesive capsulitis;
Diseases/conditions affecting multiple tissues/organ systems, including progressive systemic sclerosis (PSS), chronic graft versus host disease (GVHD); fibrotic pre-neoplastic and fibrotic neoplastic disease, and fibrosis induced by chemical or environmental insult (*e.g.,* cancer chemotherapy, pesticides, radiation/cancer radiotherapy);
Cancers, such as hepatocellular carcinoma, gastric cancer, oesophageal cancer, head and neck cancer, colorectal cancer, pancreatic cancer, cervical cancer, and vulvar cancer;
Mediastinal fibrosis, retroperitoneal fibrosis, myelofibrosis and Peyronie's disease.

In some embodiments, the fibrosis to be treated/prevented according to the present disclosure is selected from: fibrosis of tissue of the respiratory system (*e.g.* the airways or lung) or fibrosis of the liver (*e.g.* cirrhosis). In some embodiments, the disease/condition characterized by fibrosis is selected from: pulmonary fibrosis, liver fibrosis, and systemic sclerosis.

Macrophages having an M2/M2-like phenotype are also implicated in the pathology of inflammatory airway conditions such as asthma and chronic obstructive pulmonary disease (COPD). The role of M2/M2-like macrophages in such diseases/conditions is reviewed e.g. in Lambrecht and Hammand, Nat Immunol. (2015) 16(1):45-56, Wenzel, Nat Med. (2012) 18(5):716-725, Girodet et al. Am J Respir Cell Mol Biol. (2016) 55(4):467-475, He et al., Int J Chron Obstruct Pulmon Dis. (2017) 12: 3029-3039 and Finicelli et al., Antioxidants (Basel). (2022) 11(3): 464, all of which are hereby incorporated by reference in their entirety.

M2 macrophages can infiltrate airways (*e.g.* displaying inflammation as a consequence of allergic responses, infection, *etc.*) and release pro-inflammatory cytokines and chemokines, such as interleukin-4 (IL-4) and interleukin-13 (IL-13), which recruit and activate other immune cells such as eosinophils, further promoting airway inflammation and resulting in asthma. The activity of IL-4 and IL-13 in the lung can similarly result in COPD. Their immunomodulatory activity can compromise clearance of allergens and infectious agents, resulting in chronic inflammation. These macrophages can also stimulate goblet cell hyperplasia and production of excess mucus in the airways, worsening asthma symptoms. M2/M2-like macrophages also promote tissue remodeling in the airways, which is a key pathological feature of severe asthma and COPD.

Accordingly, in some aspects and embodiments, the disease/condition to be treated/prevented in accordance with the present disclosure is selected from: and inflammatory airway condition, asthma, allergic asthma and chronic obstructive pulmonary disease (COPD).

M2/M2-like macrophages are moreover implicated in the pathology of allergic conditions such as allergic rhinitis, allergic conjunctivitis and atopic dermatitis - see Kasraie and Werfel; Mediators Inflamm. (2013) 2013: 942375, Deng etal., Immunol. (2019) 12(5):1141-1149 and Iwasaki et al., PLoS One (2021) 16(3): e0248158.

M2 and M2-like macrophages are implicated in the pathology of neurological diseases, in particular diseases characterized by excessive/aberrant synaptic pruning. Synaptic pruning refers to the removal of neurons and synapses that are not needed. Central nervous system (CNS)-resident macrophages, known as microglia, are implicated in synaptic pruning through phagocytosis of neuronal synapses (see Wang et al. CNS Neurosci Ther. (2021) 27(5): 528-539). Excessive/aberrant synaptic pruning is observed in Alzheimer's disease, schizophrenia, Rett syndrome (RTT), and multiple sclerosis (MS) - see Wang et al., CNS Neurosci Ther. (2021) 27(5): 528-539, which is hereby incorporated by reference in its entirety.

In Alzheimer's disease, greater loss of synapses is observed as the disease progresses. Excessive microglial phagocytosis/efferocytosis resulting in synapse loss is also observed in schizophrenia and multiple sclerosis, and is also thought to contribute to the pathology of Rett syndrome (RTT; see Nayak et al. Innate Immunity. 2012;18(2):350-363).

Accordingly, in some aspects and embodiments, the disease/condition to be treated/prevented in accordance with the present disclosure is selected from: a neurological disease, a neurological disease in which synaptic pruning is pathologically-implicated, a neurodegenerative disease, Alzheimer's disease, schizophrenia, Rett syndrome, and multiple sclerosis.

It will be appreciated that the articles of the present disclosure also find use in the treatment/prevention of essentially any disease/condition that would derive therapeutic or prophylactic benefit from a reduction in the level of expression or activity of C1q.

For example, the disease/condition may be a disease/condition in which C1q is pathologically-implicated, *e.g.* a disease/condition in which an increased level/activity of C1q is positively associated with the onset, development or progression of the disease/condition, and/or severity of one or more symptoms of the disease/condition. In some embodiments, an increased level/activity of C1q may be a risk factor for the onset, development or progression of the disease/condition.

Diseases and conditions in which C1q is pathologically-implicated are described *e.g.* in van de Bovenkamp et al., Mol Immunol. (2021) Dec:140:206-216, Schulz et al., Front. Immunol., (2022) Sec. Molecular Innate Immunity, 13: 2022 and Zhang etal., Front. Immunol. (2023) 14:1145649, all of which are hereby incorporated by reference in their entirety.

Elevated C1q levels can result in increased deposition of C1q in tissues, resulting in inflammation and diseases such as C1q nephropathy and cryoglobulinemia. Serum C1q levels correlate with disease activity in rheumatoid arthritis (see Holers and Banda, Front. Immunol., (2018) Sec. Molecular Innate Immunity, 9: 2018), while serum concentrations of C1q are elevated in systemic sclerosis patients relative to controls (Benbassat et al., Immunologic Research (1993) 12: 312-316).

Excessive activation of the classical complement pathway through C1q is also observed in diseases such as dermatomyositis (Li etal., Ann Clin Lab Sci. (2019) 49(2):237-241), and in obesity and metabolic syndrome (Zhang et al. American Journal of Physiology. Endocrinology and Metabolism (2007) 292 (5): E1433-E1440). High levels of C1q are also associated with poor prognosis in patients having non-small cell lung cancer, and patients with idiopathic pulmonary fibrosis (Kou et al., BMC Cancer. (2022) 22(1):110).

C1q is also thought to contribute to the pathology of neurological diseases such as Alzheimer's disease, amyotrophic lateral sclerosis (ALS), Huntington's disease, multiple sclerosis, Guillain-Barré syndrome and gliomas.

In some embodiments, the disease/condition to be treated in accordance with the present disclosure is selected from: a disease/condition characterized by an increased level of C1q, C1q nephropathy, cryoglobulinemia, rheumatoid arthritis, systemic sclerosis, dermatomyositis, obesity, metabolic syndrome, non-small cell lung cancer, idiopathic pulmonary fibrosis, a neurological disease, Alzheimer's disease, amyotrophic lateral sclerosis, Huntington's disease, multiple sclerosis, Guillain-Barré syndrome and glioma.

Administration of the articles of the present disclosure is preferably in a 'therapeutically-effective' or 'prophylactically-effective' amount, this being sufficient to show therapeutic or prophylactic benefit to the subject. The actual amount administered, and rate and time-course of administration, will depend on the nature and severity of the disease/condition and the particular article administered. Prescription of treatment, *e.g.,* decisions on dosage *etc.,* is within the responsibility of general practitioners and other medical doctors, and typically takes account of the disease/disorder to be treated, the condition of the individual subject, the site of delivery, the method of administration and other factors known to practitioners. Examples of the techniques and protocols mentioned above can be found in Remington's 'The Science and Practice of Pharmacy' (ed. A. Adejare), 23rd Edition (2020), Academic Press.

Administration of the articles of the present disclosure may be topical, parenteral, systemic, intracavitary, intravenous, intra-arterial, intramuscular, intrathecal, intraocular, intravitreal, intraconjunctival, subretinal, suprachoroidal, subcutaneous, intradermal, intrathecal, oral, nasal, or transdermal. Administration may be by injection or infusion. Where the articles of the present disclosure are employed for the treatment of a cancer, administration may be intratumoral.

In some cases, the articles of the present disclosure are formulated for targeted delivery to specific cells, a tissue, an organ and/or a tumor.

Administration of the articles of the present disclosure may be alone, or in combination with other treatments, either simultaneously or sequentially dependent upon the disease/condition to be treated. The antigen-binding molecule, cell or composition described herein and another prophylactic/therapeutic agent may be administered simultaneously or sequentially.

In some embodiments, the methods comprise additional therapeutic or prophylactic intervention. In some embodiments, the additional therapeutic or prophylactic intervention is selected from chemotherapy, immunotherapy, radiotherapy, surgery, vaccination and/or hormone therapy. In some embodiments, the additional therapeutic or prophylactic intervention comprises leukapheresis. In some embodiments, the additional therapeutic or prophylactic intervention comprises a stem cell transplant.

Simultaneous administration refers to administration of the antigen-binding molecule, polypeptide, nucleic acid (or plurality thereof), expression vector (or plurality thereof), cell or composition and therapeutic agent together, for example as a pharmaceutical composition containing both agents (combined preparation), or immediately after each other and optionally via the same route of administration, e.g., to the same artery, vein, or other blood vessel. Sequential administration refers to administration of one of the antigen-binding molecule/composition or therapeutic agent followed after a given time interval by separate administration of the other agent. It is not required that the two agents are administered by the same route, although this is the case in some embodiments. The time interval may be any time interval.

In some embodiments, treatment of cancer further comprises chemotherapy and/or radiotherapy. Chemotherapy and radiotherapy respectively refer to treatment of a cancer with a drug or with ionizing radiation (*e.g.* radiotherapy using X-rays or γ-rays). The drug may be a chemical entity, *e.g.* small molecule pharmaceutical, antibiotic, DNA intercalator, protein inhibitor (*e.g.* kinase inhibitor), or a biological agent, *e.g.* antibody, antibody fragment, aptamer, nucleic acid (*e.g.* DNA, RNA), peptide, polypeptide, or protein. The drug may be formulated as a pharmaceutical composition or medicament. The formulation may comprise one or more drugs (*e.g.* one or more active agents) together with one or more pharmaceutically acceptable diluents, excipients or carriers.

Chemotherapy may involve administration of more than one drug. A drug may be administered alone or in combination with other treatments, either simultaneously or sequentially dependent upon the condition to be treated. The chemotherapy may be administered by one or more routes of administration, *e.g.* parenteral, intravenous injection, oral, subcutaneous, intradermal or intratumoral. The chemotherapy may be administered according to a treatment regime. The treatment regime may be a pre-determined timetable, plan, scheme, or schedule of chemotherapy administration which may be prepared by a physician or medical practitioner and may be tailored to suit the patient requiring treatment. The treatment regime may indicate one or more of: the type of chemotherapy to administer to the patient; the dose of each drug or radiation; the time interval between administrations; the length of each treatment; the number and nature of any treatment holidays, if any *etc.* For a co-therapy a single treatment regime may be provided which indicates how each drug is to be administered.

Multiple doses of the antigen-binding molecule, polypeptide, nucleic acid (or plurality thereof), expression vector (or plurality thereof), cell or composition described herein may be provided. One or more, or each, of the doses may be accompanied by simultaneous or sequential administration of another therapeutic agent.

Multiple doses may be separated by a predetermined time interval, which may be selected to be one of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, or 1, 2, 3, 4, 5, or 6 months. By way of example, doses may be given once every 7, 14, 21 or 28 days (plus or minus 3, 2, or 1 days).

In accordance with various aspects of the present disclosure, a method of treating and/or preventing a disease/condition may comprise one or more of the following: inhibiting polarization of macrophages to an M2 or M2-like phenotype; inhibiting development and/or maturation of macrophages to an M2 or M2-like phenotype; reducing the number/proportion of macrophages having an M2 or M2-like phenotype (*i.e.* within a subject, or within a tissue/tumor of a subject); promoting polarization of macrophages to an M1 or M1-like phenotype; promoting development and/or maturation of macrophages to an M1 or M1-like phenotype; increasing the number/proportion of macrophages having an M1 or M1-like phenotype (*i.e.* within a subject, or within a tissue/tumor of a subject); inhibiting IL-10 expression/production by macrophages; inhibiting gene and/or protein expression of IL-10 by macrophages; inhibiting secretion of IL-10 by macrophages; reducing the number/proportion of IL-10-producing/expressing macrophages (*i.e.* within a subject, or within a tissue/tumor of a subject); inhibiting macrophage efferocytosis; inhibiting efferocytosis by macrophages of cells (*e.g.* apoptotic cells) coated with C1q; reducing the number/proportion of efferocytotic macrophages (*i.e.* within a subject, or within a tissue/tumor of a subject); inhibiting activation of the complement cascade; inhibiting the activity of the C1 complex; inhibiting binding of C1q to activated endothelial cells; inhibiting binding of C1q to a C1q receptor (*e.g.* a C1q receptor selected from cC1qR, LAIR1, LAIR2, gC1qR and CR1); inhibiting binding of C1q to cC1qR; and/or inhibiting binding of C1q to LAIR1.

The present disclosure further provides the use of an antigen-binding molecule according to the present disclosure to: inhibit polarization of macrophages to an M2 or M2-like phenotype; inhibit development and/or maturation of macrophages to an M2 or M2-like phenotype; reduce the number/proportion of macrophages having an M2 or M2-like phenotype (*i.e.* within a subject, or within a tissue/tumor of a subject); promote polarization of macrophages to an M1 or M1-like phenotype; promote development and/or maturation of macrophages to an M1 or M1-like phenotype; increase the number/proportion of macrophages having an M1 or M1-like phenotype (*i.e.* within a subject, or within a tissue/tumor of a subject); inhibit IL-10 expression/production by macrophages; inhibit gene and/or protein expression of IL-10 by macrophages; inhibit secretion of IL-10 by macrophages; reduce the number/proportion of IL-10-producing/expressing macrophages (*i.e.* within a subject, or within a tissue/tumor of a subject); inhibit macrophage efferocytosis; inhibit efferocytosis by macrophages of cells (*e.g.* apoptotic cells) coated with C1q; reduce the number/proportion of efferocytotic macrophages (*i.e.* within a subject, or within a tissue/tumor of a subject); inhibit activation of the complement cascade; inhibit the activity of the C1 complex; inhibit binding of C1q to activated endothelial cells; inhibit binding of C1q to a C1q receptor (*e.g.* a C1q receptor selected from cC1qR, LAIR1, LAIR2, gC1qR and CR1); inhibit binding of C1q to cC1qR; and/or inhibit binding of C1q to LAIR1.

The present disclosure further provides methods for: inhibiting polarization of macrophages to an M2 or M2-like phenotype; inhibiting development and/or maturation of macrophages to an M2 or M2-like phenotype; reducing the number/proportion of macrophages having an M2 or M2-like phenotype (*i.e.* within a subject, or within a tissue/tumor of a subject); promoting polarization of macrophages to an M1 or M1-like phenotype; promoting development and/or maturation of macrophages to an M1 or M1-like phenotype; increasing the number/proportion of macrophages having an M1 or M1-like phenotype (*i.e.* within a subject, or within a tissue/tumor of a subject); inhibiting IL-10 expression/production by macrophages; inhibiting gene and/or protein expression of IL-10 by macrophages; inhibiting secretion of IL-10 by macrophages; reducing the number/proportion of IL-10-producing/expressing macrophages (*i.e.* within a subject, or within a tissue/tumor of a subject); inhibiting macrophage efferocytosis; inhibiting efferocytosis by macrophages of cells (*e.g.* apoptotic cells) coated with C1q; reducing the number/proportion of efferocytotic macrophages (*i.e.* within a subject, or within a tissue/tumor of a subject); inhibiting activation of the complement cascade; inhibiting the activity of the C1 complex; inhibiting binding of C1q to activated endothelial cells; inhibiting binding of C1q to a C1q receptor (*e.g.* a C1q receptor selected from cC1qR, LAIR1, LAIR2, gC1qR and CR1); inhibiting binding of C1q to cC1qR; and/or inhibiting binding of C1q to LAIR1, using an antigen-binding molecule according to the present disclosure.

In some embodiments, the methods comprise contacting cells with an antigen-binding molecule according to the present disclosure. In some embodiments, the cells are contacted with the antigen-binding molecule *in vitro* or *ex vivo.* In some embodiments, the cells are contacted with the antigen-binding molecule *in vivo, e.g.* via administration of the antigen-binding molecule to a subject.

Accordingly, the present disclosure provides methods for: inhibiting polarization of macrophages to an M2 or M2-like phenotype; inhibiting development and/or maturation of macrophages to an M2 or M2-like phenotype; reducing the number/proportion of macrophages having an M2 or M2-like phenotype (*i.e.* within a subject, or within a tissue/tumor of a subject); promoting polarization of macrophages to an M1 or M1-like phenotype; promoting development and/or maturation of macrophages to an M1 or M1-like phenotype; increasing the number/proportion of macrophages having an M1 or M1-like phenotype (*i.e.* within a subject, or within a tissue/tumor of a subject); inhibiting IL-10 expression/production by macrophages; inhibiting gene and/or protein expression of IL-10 by macrophages; inhibiting secretion of IL-10 by macrophages; reducing the number/proportion of IL-10-producing/expressing macrophages (*i.e.* within a subject, or within a tissue/tumor of a subject); inhibiting macrophage efferocytosis; inhibiting efferocytosis by macrophages of cells (*e.g.* apoptotic cells) coated with C1q; reducing the number/proportion of efferocytotic macrophages (*i.e.* within a subject, or within a tissue/tumor of a subject); inhibiting activation of the complement cascade; inhibiting the activity of the C1 complex; inhibiting binding of C1q to activated endothelial cells; inhibiting binding of C1q to a C1q receptor (*e.g.* a C1q receptor selected from cC1qR, LAIR1, LAIR2, gC1qR and CR1); inhibiting binding of C1q to cC1qR; and/or inhibiting binding of C1q to LAIR1 in a subject, comprising administering to a subject an antigen-binding molecule according to the present disclosure.

### Methods of detection

The present disclosure also provides the articles of the present disclosure for use in methods for detecting, localizing or imaging C1q, or cells producing C1q.

The antigen-binding molecules described herein may be used in methods that involve detecting binding of the antigen-binding molecule to C1q. Such methods may involve detection of the bound complex of the antigen-binding molecule and C1q.

As such, a method is provided, comprising contacting a sample containing, or suspected to contain, C1q, and detecting the formation of a complex of the antigen-binding molecule and C1q.

Suitable method formats are well known in the art, including immunoassays such as sandwich assays, e.g. ELISA. The methods may involve labelling the antigen-binding molecule, or target(s), or both, with a detectable moiety, *e.g.* a fluorescent label, phosphorescent label, luminescent label, immuno-detectable label, radiolabel, chemical, nucleic acid or enzymatic label as described herein. Detection techniques are well known to those of skill in the art and can be selected to correspond with the labelling agent.

Methods comprising detecting C1q include methods for diagnosing/prognosing a disease/condition described herein.

Methods of this kind may be performed *in vitro* on a patient sample, or following processing of a patient sample. Once the sample is collected, the patient is not required to be present for the *in vitro* method to be performed, and therefore the method may be one which is not practiced on the human or animal body. In some embodiments, the method is performed *in vivo.*

Such methods may involve detecting or quantifying C1q, *e.g.* in a patient sample. Where the method comprises quantifying the relevant factor, the method may further comprise comparing the determined amount against a standard or reference value as part of the diagnostic or prognostic evaluation. Other diagnostic/prognostic tests may be used in conjunction with those described herein to enhance the accuracy of the diagnosis or prognosis or to confirm a result obtained by using the tests described herein.

Detection in a sample may be used for the purpose of diagnosis of a disease/condition (*e.g.* a cancer), predisposition to a disease/condition, or for providing a prognosis (prognosticating) for a disease/condition, *e.g.* a disease/condition described herein. The diagnosis or prognosis may relate to an existing (previously diagnosed) disease/condition.

A sample may be taken from any tissue or bodily fluid. The sample may comprise or may be derived from: a quantity of blood; a quantity of serum derived from the individual's blood which may comprise the fluid portion of the blood obtained after removal of the fibrin clot and blood cells; a tissue sample or biopsy; pleural fluid; cerebrospinal fluid (CSF); or cells isolated from said individual. In some embodiments, the sample may be obtained or derived from a tissue or tissues which are affected by the disease/condition (*e.g.* tissue or tissues in which symptoms of the disease manifest, or which are involved in the pathogenesis of the disease/condition).

A subject may be selected for diagnostic/prognostic evaluation based on the presence of symptoms indicative of a disease/condition described herein, or based on the subject being considered to be at risk of developing a disease/condition described herein.

The present disclosure also provides methods for selecting/stratifying a subject for treatment with a C1q-targeted agent. In some embodiments a subject is selected for treatment/prevention in accordance with the methods of the present disclosure, or is identified as a subject which would benefit from such treatment/prevention, based on detection/quantification of C1q, *e.g.* in a sample obtained from the individual.

### Subjects

A subject in accordance with the various aspects of the present disclosure may be any animal or human. Therapeutic and prophylactic applications may be in human or animals (veterinary use).

The subject to be administered with an article of the present disclosure (*e.g.* in accordance with therapeutic or prophylactic intervention) may be a subject in need of such intervention. The subject is preferably mammalian, more preferably human. The subject may be a non-human mammal, but is more preferably human. The subject may be male or female. The subject may be a patient.

A subject may have (*e.g.* may have been diagnosed with) a disease or condition described herein, may be suspected of having such a disease/condition, or may be at risk of developing/contracting such a disease/condition. In embodiments according to the present disclosure, a subject may be selected for treatment according to the methods based on characterization for one or more markers of such a disease/condition.

### Kits

The present disclosure also provides kits of parts.

In some embodiments, the kit may have at least one container having a predetermined quantity of an antigen-binding molecule, polypeptide, nucleic acid (or plurality thereof), expression vector (or plurality thereof), cell or composition described herein.

In some embodiments, the kit may comprise materials for producing an antigen-binding molecule, polypeptide, nucleic acid (or plurality thereof), expression vector (or plurality thereof), cell or composition described herein. In some embodiments, the kit of parts may comprise materials for formulating an antigen-binding molecule, polypeptide, nucleic acid (or plurality thereof), expression vector (or plurality thereof), cell or composition described herein to a pharmaceutical composition/medicament, *e.g.* in a composition further comprising a pharmaceutically-acceptable carrier, diluent, excipient or adjuvant.

The kit may provide the antigen-binding molecule, polypeptide, nucleic acid (or plurality thereof), expression vector (or plurality thereof), cell or composition together with instructions for administration to a patient in order to treat a specified disease/condition (*e.g.* a disease/condition described herein).

In some embodiments the kit may further comprise at least one container having a predetermined quantity of another therapeutic agent (*e.g.* as described herein). In such embodiments, the kit may also comprise a second medicament or pharmaceutical composition such that the two medicaments or pharmaceutical compositions may be administered simultaneously or separately such that they provide a combined treatment for the specific disease/condition.

Kits according to the present disclosure may include instructions for use, *e.g.* in the form of an instruction booklet or leaflet. The instructions may include a protocol for performing any one or more of the methods described herein.

### Sequence identity

As used herein, 'sequence identity' refers to the percent of nucleotides/amino acid residues in a subject sequence that are identical to nucleotides/amino acid residues in a reference sequence, after aligning the sequences and, if necessary, introducing gaps, to achieve the maximum percent sequence identity between the sequences. Pairwise and multiple sequence alignment for the purposes of determining percent sequence identity between two or more amino acid or nucleic acid sequences can be achieved in various ways known to a person of skill in the art, for instance, using publicly available computer software such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)) and MAFFT (Katoh and Standley 2013, Molecular Biology and Evolution, 30(4) 772-780) software. When using such software, the default parameters, *e.g.* for gap penalty and extension penalty, are preferably used.

### Sequences

| **SEQ ID NO:** | **DESCRIPTION** | **SEQUENCE** |
|---|---|---|
| 1 | Human C1qA (UniProt P02745) | |
| 2 | Human C1qA signal peptide | MEGPRGWLVLCVLAISLASMVT |
| 3 | Human C1qA N-terminal region | TEDLCRAPD |
| 4 | Human C1qA collagen-like region | GKKGEAGRPGRRGRPGLKGEQGEPGAPGIRTGIQGLKGDQGEPGPSGN PGKVGYPGPSGPLGARGIPGIKGTKGSPGNI |
| 5 | Human C1qA C-terminal globular region | KDQPRPAFSAIRRNPPMGGNVVIFDTVITNQEEPYQNHSGRFVCTVPGYY YFTFQVLSQWEICLSIVSSSRGQVRRSLGFCDTTNKGLFQVVSGGMVLQL QQGDQVWVEKDPKKGHIYQGSEADSVFSGFLIFPSA |
| 6 | Mature Human C1qA | EDLCRAPDGKKGEAGRPGRRGRPGLKGEQGEPGAPGIRTGIQGLKGDQG EPGPSGNPGKVGYPGPSGPLGARGIPGIKGTKGSPGNIKDQPRPAFSAIR RNPPMGGNVVIFDTVITNQEEPYQNHSGRFVCTVPGYYYFTFQVLSQWEI CLSIVSSSRGQVRRSLGFCDTTNKGLFQVVSGGMVLQLQQGDQVWVEKD PKKGHIYQGSEADSVFSGFLIFPSA |
| 7 | Human C1qB (UniProt P02746) | MMMKIPWGSIPVLMLLLLLGLIDISQAQLSCTGPPAIPGIPGIPGTPGPDGQP GTPGIKGEKGLPGLAGDHGEFGEKGDPGIPGNPGKVGPKGPMGPKGGPG APGAPGPKGESGDYKATQKIAFSATRTINVPLRRDQTIRFDHVITNMNNNY EPRSGKFTCKVPGLYYFTYHASSRGNLCVNLMRGRERAQKVVTFCDYAY NTFQVTTGGMVLKLEQGENVFLQATDKNSLLGMEGANSIFSGFLLFPDME A |
| 8 | Human C1qB signal peptide | MMMKIPWGSIPVLMLLLLLGLIDISQA |
| 9 | Human C1qB N-terminal region | QLSCTGPPA |
| 10 | Human C1qB collagen-like region 1 | IPGIPGIPGTPGPDGQPGTPGIKGEKGLPGLAGDHGEFGEKGDPGIPGNP |
| 11 | Human C1qB collagen-like region 2 | GEKGLPGLAGDHGEFGEKGDPGIPGNPGKVGPKGPMGPKGGPGAPGAP GPKGESG |
| 12 | Human C1qB C-terminal globular region | KATQKIAFSATRTINVPLRRDQTIRFDHVITNMNNNYEPRSGKFTCKVPGLY YFTYHASSRGNLCVNLMRGRERAQKVVTFCDYAYNTFQVTTGGMVLKLE QGENVFLQATDKNSLLGMEGANSIFSGFLLFPDMEA |
| 13 | Mature Human C1qB | QLSCTGPPAIPGIPGIPGTPGPDGQPGTPGIKGEKGLPGLAGDHGEFGEK GDPGIPGNPGKVGPKGPMGPKGGPGAPGAPGPKGESGDYKATQKIAFSA TRTINVPLRRDQTIRFDHVITNMNNNYEPRSGKFTCKVPGLYYFTYHASSR GNLCVNLMRGRERAQKVVTFCDYAYNTFQVTTGGMVLKLEQGENVFLQA TDKNSLLGMEGANSIFSGFLLFPDMEA |
| 14 | Human C1qC (UniProt P02747) | |
| 15 | Human C1qC signal peptide | MDVGPSSLPHLGLKLLLLLLLLPLRGQA |
| 16 | Human C1qC N-terminal region | NT |
| 17 | Human C1qC collagen-like region | GCYGIPGMPGLPGAPGKDGYDGLPGPKGEPGIPAIPGIRGPKGQKGEPGL PGHPGKNGPMGPPGMPGVPGPMGIPGEPGEEG |
| 18 | Human C1qC C-terminal globular region | KQKFQSVFTVTRQTHQPPAPNSLIRFNAVLTNPQGDYDTSTGKFTCKVPG LYYFVYHASHTANLCVLLYRSGVKVVTFCGHTSKTNQVNSGGVLLRLQVG EEVWLAVNDYYDMVGIQGSDSVFSGFLLFPD |
| 19 | Mature Human C1qC | NTGCYGIPGMPGLPGAPGKDGYDGLPGPKGEPGIPAIPGIRGPKGQKGEP GLPGHPGKNGPMGPPGMPGVPGPMGIPGEPGEEGRYKQKFQSVFTVTR QTHQPPAPNSLIRFNAVLTNPQGDYDTSTGKFTCKVPGLYYFVYHASHTA NLCVLLYRSGVKVVTFCGHTSKTNQVNSGGVLLRLQVGEEVWLAVNDYY DMVGIQGSDSVFSGFLLFPD |
| 20 | Human IgG1 constant region (IGHG1; UniProt:P01857-1, v1) | |
| 21 | CH1 IgG1 (positions 1-98 of P01857-1, v1) | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKV |
| 22 | Hinge IgG1 (positions 99-110 of P01857-1, v1) | EPKSCDKTHTCP |
| 23 | CH2 IgG1 (positions 111-223 of P01857-1, v1) | PCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKA LPAPIEKTISKAK |
| 24 | CH3 IgG1 (positions 224-330 of P01857-1, v1) | GQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQK SLSLSPGK |
| 25 | Human IgG1 constant region G1m3 allotype (K214R, D356E and L358M (EU numbering) relative to P01857-1) | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKRVEPK SCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKE YKCKVSNKALPAPIEKTISKAKGQPREPQVYTLPPSREEMTKNQVSLTCLV KGFYPSDIAVEWESNGQPENNYKTTPPVLDSDGSFFLYSKLTVDKSRWQQ GNVFSCSVMHEALHNHYTQKSLSLSPGK |
| 26 | CH1 IgG1 G1m3 allotype | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKRV |
| 27 | CH3 IgG1 G1m3 allotype | GQPREPQVYTLPPSREEMTKNQVSLTCLVKGFYPSDIAVEWESNGQPEN NYKTTPPVLDSDGSFFLYSKLTVDKSRWQQGNVFSCSVMHEALHNHYTQ KSLSLSPGK |
| 28 | Human IgG1 CH2-CH3 region | PCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKA LPAPIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVM HEALHNHYTQKSLSLSPGK |
| 29 | Human IgG1 G1m3 allotype CH2-CH3 region | PCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVM HEALHNHYTQKSLSLSPG |
| 30 | **Cκ CL (IGKC;** UniProt: P01834-1, v2) | |
| 31 | **Cλ CL1 (IGLC1;** UniProt: P0CG04-1, v1) | GQPKANPTVTLFPPSSEELQANKATLVCLISDFYPGAVTVAWKADGSPVKA GVETTKPSKQSNNKYAASSYLSLTPEQWKSHRSYSCQVTHEGSTVEKTVA PTECS |
| 32 | **Cλ CL2 (IGLC2;** UniProt: P0DOY2-1, v1) | GQPKAAPSVTLFPPSSEELQANKATLVCLISDFYPGAVTVAWKADSSPVKA GVETTTPSKQSNNKYAASSYLSLTPEQWKSHRSYSCQVTHEGSTVEKTVA PTECS |
| 33 | **Cλ CL3 (IGLC3;** UniProt: P0DOY3-1, v1) | GQPKAAPSVTLFPPSSEELQANKATLVCLISDFYPGAVTVAWKADSSPVKA GVETTTPSKQSNNKYAASSYLSLTPEQWKSHKSYSCQVTHEGSTVEKTVA PTECS |
| 34 | **Cλ CL6 (IGLC6;** UniProt: P0CF74-1, v1) | GQPKAAPSVTLFPPSSEELQANKATLVCLISDFYPGAVKVAWKADGSPVN TGVETTTPSKQSNNKYAASSYLSLTPEQWKSHRSYSCQVTHEGSTVEKTV APAECS |
| 35 | **Cλ CL7 (IGLC7;** UniProt: A0M8Q6-1, v3) | GQPKAAPSVTLFPPSSEELQANKATLVCLVSDFNPGAVTVAWKADGSPVK VGVETTKPSKQSNNKYAASSYLSLTPEQWKSHRSYSCRVTHEGSTVEKTV APAECS |

### Numbered statements

Statement 1. An antigen-binding molecule that binds to C1q for treating or preventing a disease in which M2 macrophages and/or M2-like macrophages are pathologically-implicated.

Statement 2. Use of an antigen-binding molecule that binds to C1q in the manufacture of a medicament for treating or preventing a disease in which M2 macrophages and/or M2-like macrophages are pathologically-implicated.

Statement 3. A method of treating or preventing a disease in which M2 macrophages and/or M2-like macrophages are pathologically-implicated, wherein the method comprises administering to a subject a therapeutically- or prophylactically-effective amount of an antigen-binding molecule that binds to C1q.

Statement 4. The antigen-binding molecule for use according to statement 1, the use according to statement 2, or the method according to statement 3, wherein the method of treating or preventing a disease in which M2 macrophages and/or M2-like macrophages are pathologically-implicated comprises inhibiting polarization of macrophages to an M2 or M2-like phenotype.

Statement 5. The antigen-binding molecule for use according to statement 1 or statement 4, the use according to statement 2 or statement 4, or the method according to statement 3 or statement 4, wherein the disease in which M2 macrophages and/or M2-like macrophages are pathologically-implicated is selected from: a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome and multiple sclerosis.

Statement 6. The antigen-binding molecule for use according to any one of statements 1, 4 or 5, the use according to any one of statements 2, 4 or 5, or the method according to any one of statements 3 to 5, wherein the antigen-binding molecule inhibits polarization of macrophages to an M2 or M2-like phenotype, inhibits IL-10 production by macrophages and/or inhibits macrophage efferocytosis.

Statement 7. The antigen-binding molecule for use according to any one of statements 1 or 4 to 6, the use according to any one of statements 2 or 4 to 6, or the method according to any one of statements 3 to 6, wherein the antigen-binding molecule does not substantially inhibit activation of the complement cascade.

Statement 8. An antigen-binding molecule that binds to C1q.

Statement 9. An antigen-binding molecule that binds to C1q, wherein the antigen-binding molecule inhibits polarization of macrophages to an M2 or M2-like phenotype.

Statement 10. An antigen-binding molecule that binds to C1q, wherein the antigen-binding molecule inhibits IL-10 production by macrophages.

Statement 11. An antigen-binding molecule that binds to C1q, wherein the antigen-binding molecule inhibits macrophage efferocytosis.

Statement 12. An antigen-binding molecule that binds to C1q, wherein the antigen-binding molecule does not substantially inhibit activation of the complement cascade.

Statement 13. An antigen-binding molecule that binds to C1q, wherein the antigen-binding molecule inhibits binding of C1q to activated endothelial cells.

Statement 14. An antigen-binding molecule that binds to C1q, wherein the antigen-binding molecule inhibits C1q binding to a C1q receptor (e.g. cC1qR and/or LAIR1).

Statement 15. A chimeric antigen receptor (CAR) comprising an antigen-binding molecule according to any one of statements 8 to 14.

Statement 16. A nucleic acid, or a plurality of nucleic acids, optionally isolated, encoding an antigen-binding molecule according to any one of statements 8 to 14, or a CAR according to statement 15.

Statement 17. An expression vector, or a plurality of expression vectors, comprising a nucleic acid or a plurality of nucleic acids according to statement 17.

Statement 18. A cell comprising an antigen-binding molecule according to any one of statements 8 to 14, a CAR according to statement 15, a nucleic acid or a plurality of nucleic acids according to statement 16, or an expression vector or a plurality of expression vectors according to statement 17.

Statement 19. A method comprising culturing a cell according to statement 18 under conditions suitable for expression of an antigen-binding molecule or CAR by the cell.

Statement 20. A composition comprising an antigen-binding molecule according to any one of statements 8 to 14, a CAR according to statement 15, a nucleic acid or a plurality of nucleic acids according to statement 16, an expression vector or a plurality of expression vectors according to statement 17, or a cell according to statement 18, and a pharmaceutically acceptable carrier, diluent, excipient or adjuvant.

Statement 21. An antigen-binding molecule according to any one of statements 8 to 14, a CAR according to statement 15, a nucleic acid or a plurality of nucleic acids according to statement 16, an expression vector or a plurality of expression vectors according to statement 17, a cell according to statement 18, or a composition according to statement 20, for use in a method of medical treatment or prophylaxis.

Statement 22. An antigen-binding molecule according to any one of statements 8 to 14, a CAR according to statement 15, a nucleic acid or a plurality of nucleic acids according to statement 16, an expression vector or a plurality of expression vectors according to statement 17, a cell according to statement 18, or a composition according to statement 20, for treating or preventing: a disease/condition characterized by an increased level of C1q, C1q nephropathy, cryoglobulinemia, rheumatoid arthritis, systemic sclerosis, dermatomyositis, obesity, metabolic syndrome, non-small cell lung cancer, idiopathic pulmonary fibrosis, a neurological disease, Alzheimer's disease, amyotrophic lateral sclerosis, Huntington's disease, multiple sclerosis, Guillain-Barré syndrome, glioma, a disease in which M2 macrophages and/or M2-like macrophages are pathologically-implicated, a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome or multiple sclerosis.

Statement 23. Use of an antigen-binding molecule according to any one of statements 8 to 14, a CAR according to statement 15, a nucleic acid or a plurality of nucleic acids according to statement 16, an expression vector or a plurality of expression vectors according to statement 17, a cell according to statement 18, or a composition according to statement 20, in the manufacture of a medicament for treating or preventing: a disease/condition characterized by an increased level of C1q, C1q nephropathy, cryoglobulinemia, rheumatoid arthritis, systemic sclerosis, dermatomyositis, obesity, metabolic syndrome, non-small cell lung cancer, idiopathic pulmonary fibrosis, a neurological disease, Alzheimer's disease, amyotrophic lateral sclerosis, Huntington's disease, multiple sclerosis, Guillain-Barré syndrome, glioma, a disease in which M2 macrophages and/or M2-like macrophages are pathologically-implicated, a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome or multiple sclerosis.

Statement 24. A method of treating or preventing a disease/condition characterized by an increased level of C1q, C1q nephropathy, cryoglobulinemia, rheumatoid arthritis, systemic sclerosis, dermatomyositis, obesity, metabolic syndrome, non-small cell lung cancer, idiopathic pulmonary fibrosis, a neurological disease, Alzheimer's disease, amyotrophic lateral sclerosis, Huntington's disease, multiple sclerosis, Guillain-Barré syndrome, glioma, a disease in which M2 macrophages and/or M2-like macrophages are pathologically-implicated, a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome or multiple sclerosis, wherein the method comprises administering to a subject a therapeutically- or prophylactically-effective amount of an antigen-binding molecule according to any one of statements 8 to 14, a CAR according to statement 15, a nucleic acid or a plurality of nucleic acids according to statement 16, an expression vector or a plurality of expression vectors according to statement 17, a cell according to statement 18, or a composition according to statement 20.

Statement 25. An in vitro complex, optionally isolated, comprising an antigen-binding molecule according to any one of statements 8 to 14 bound to C1q.

Statement 26. A method for detecting C1q in a sample, comprising contacting a sample containing, or suspected to contain, C1q with an antigen-binding molecule according to any one of statements 8 to 14, and detecting the formation of a complex of the antigen-binding molecule with C1q.

Statement 27. Use of an antigen-binding molecule according to any one of statements 8 to 14 as an in vitro or in vivo diagnostic or prognostic agent.

Statement 28. A composition comprising:
(i) an antigen-binding molecule according to any one of statements 8 to 14; and
(ii) a population of cells.

Statement 29. The composition according to statement 28, wherein the population of cells comprises a macrophage cell.

The present disclosure includes the combination of the aspects and preferred features described except where such a combination is clearly impermissible or expressly avoided.

The section headings used herein are for organizational purposes only and are not to be construed as limiting the subject matter described.

Aspects and embodiments of the present disclosure will now be illustrated, by way of example, with reference to the accompanying figures. Further aspects and embodiments will be apparent to those skilled in the art. All documents mentioned in this text are incorporated herein by reference.

Throughout this specification, including the claims which follow, unless the context requires otherwise, the word 'comprise,' and variations such as 'comprises' and 'comprising,' will be understood to imply the inclusion of a stated integer or step or group of integers or steps but not the exclusion of any other integer or step or group of integers or steps.

As used herein, an amino acid sequence, or a region of a polypeptide which 'corresponds' to a specified reference amino acid sequence or region of a polypeptide has at least 60%, e.g. one of at least 65%, 70%, 75%, 80%, 85%, 90%, 91%, 92%, 93%, 94%, 95%, 96%, 97%, 98%, 99% or 100% sequence identity to the amino acid sequence of the amino acid sequence/polypeptide/region. An amino acid sequence/region/position of a polypeptide/amino acid sequence which 'corresponds' to a specified reference amino acid sequence/region/position of a polypeptide/amino acid sequence can be identified by sequence alignment of the subject sequence to the reference sequence, e.g. using sequence alignment software such as ClustalOmega (Söding, J. 2005, Bioinformatics 21, 951-960).

It must be noted that, as used in the specification and the appended claims, the singular forms 'a,' 'an,' and 'the' include plural referents unless the context clearly dictates otherwise. Ranges may be expressed herein as from 'about' one particular value, and/or to 'about' another particular value. When such a range is expressed, another embodiment includes from the one particular value and/or to the other particular value. Similarly, when values are expressed as approximations, by the use of the antecedent 'about,' it will be understood that the particular value forms another embodiment.

Where a nucleic acid sequence is disclosed herein, the reverse complement thereof is also expressly contemplated.

Methods described herein may preferably be performed *in vitro.* The term *'in vitro'* is intended to encompass procedures performed with cells in culture whereas the term *'in vivo'* is intended to encompass procedures with/on intact multi-cellular organisms.

Antigen-binding molecules, peptides/polypeptides, peptide/polypeptide complexes, nucleic acids/polynucleotides, vectors, compositions or cells according to the present disclosure may optionally be provided in isolated or purified form. For example, articles according to the present disclosure may be isolated/purified from naturally-occurring biological material.

### Brief description of the drawings

For a further understanding of the various described implementations, reference should be made to the detailed description below, in conjunction with the following drawings in which like reference numerals refer to corresponding parts throughout the figures.

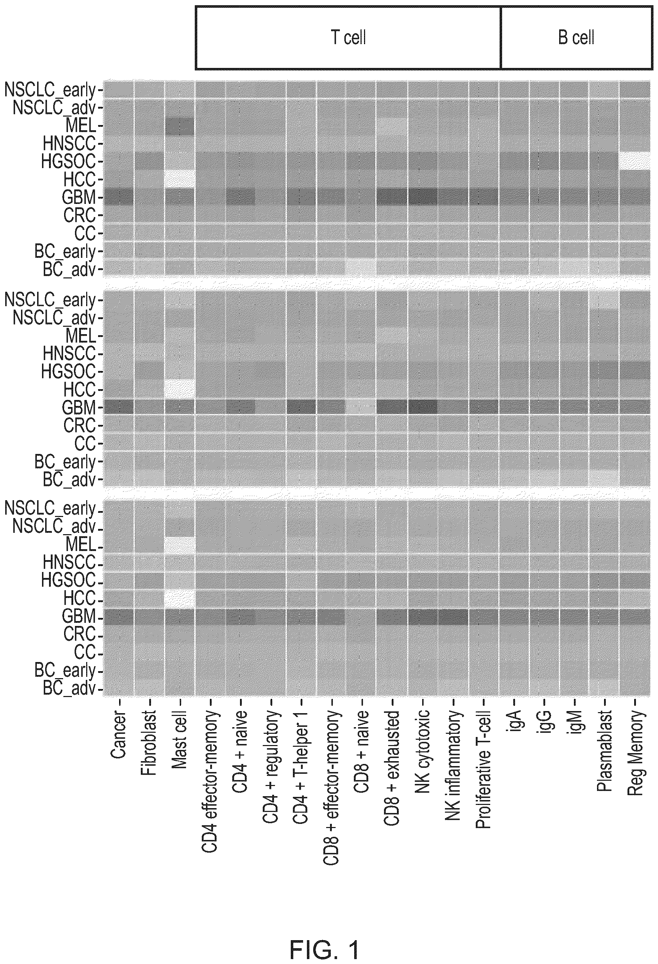

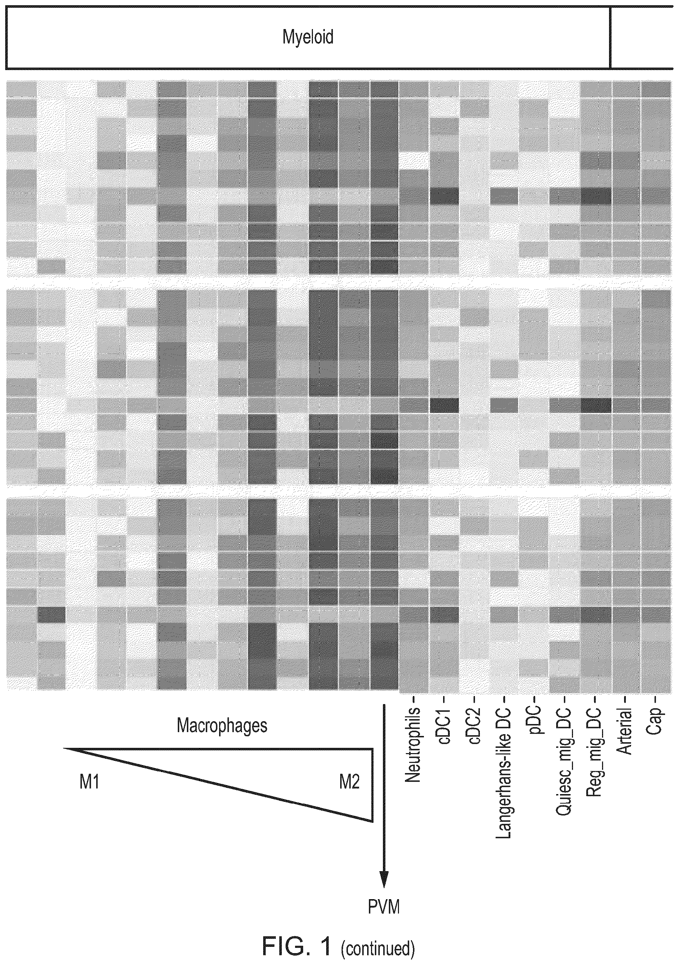

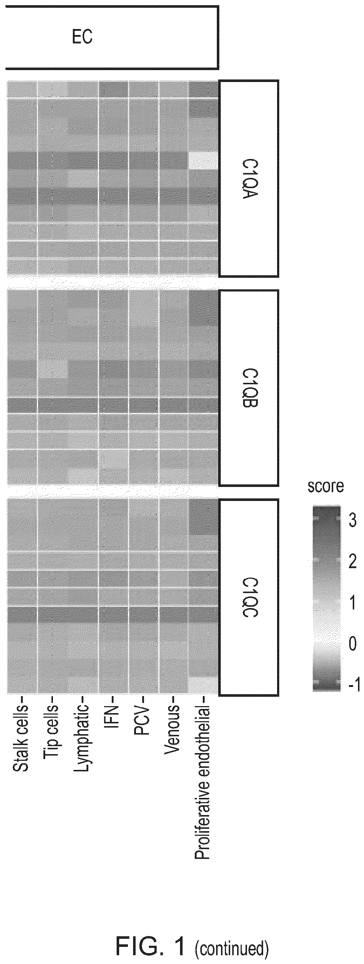

**Figure 1****.** Image showing the results of single-cell expression analysis of *C1QA, C1QB* and *C1QC* by immune cells of different types obtained from non-small cell lung cancer (NSCLC), melanoma (MEL), head and neck squamous cell carcinoma (HNSCC), hepatocellular carcinoma (HCC), glioblastoma (GBM), colorectal cancer (CRC), cervical cancer (CC) and breast cancer (BC) samples. PVM = perivascular macrophage.

### Examples

### Example 1: Single-cell analysis of C1q expression

Single-cell gene expression analysis was performed to assess the gene expression of *C1QA, C1QB* and *C1QC* by T cells, B cells, myeloid cells and endothelial cells obtained from various different cancerous tissue samples, including samples of non-small cell lung cancer (NSCLC), melanoma (MEL), head and neck squamous cell carcinomas (HNSCC), hepatocellular carcinoma (HCC), glioblastoma (GBM), colorectal cancer (CRC), cervical cancer (CC), breast cancer (BC).

All tumors were treatment-naive, reflected different disease stages (*e.g.,* stage I-IV lung cancer) or different histopathologies (*e.g.,* adenocarcinoma versus squamous lung cancer). Tumor samples were gently dissociated using a standardized uniform dissociation protocol to allow annotation and analysis of all stromal cellular phenotypes across the different cancer types. A unique feature of the scRNA-seq dataset is therefore that each stromal cell has been annotated as a specific cellular phenotype, resulting in a phenotypic blueprint of each tumor and its microenvironment. The blueprint includes, 10 myeloid and 9 endothelial pan-cancer cellular phenotypes. The macrophage subtypes include Macro_LYVE1 (Perivascular Macrophages), Macro_CXCL10, Macro_MMP9, Macro_MT1G, Macro_SLC2A1, Macro_CCL18, Macro_CCL2_CCL18, Macro_CCL2, Macro_CX3CR1, Macro_CCR2_CX3CR1, Macro_CCR2, and proliferating macrophages (Macro_prolif).

The endothelial cell subtypes include Arterial, PCVs (Post capillary venules), Capillaries, Stalk cells, Tip cells, IFN EC, Venous ECs, and Proliferative ECs.

The dataset was analyzed using a standard Seurat pipeline. Through unsupervised clustering and differential expression, detailed labels for myeloid, endothelial, and T-cells were obtained.

The results of the analysis are shown in Figure 1. The expression of genes encoding the constituent polypeptides of C1q was found to be elevated among tumor-associated macrophages. These genes were found to be particularly highly-expressed by perivascular macrophages (PVMs), and macrophages having an M2 (or M2-like) phenotype, while expression by macrophages having an M1 (or M1-like) phenotype was much lower. This pattern of *C1QA, C1QB* and *C1QC* expression was observed in tissue samples from cancers of diverse origin.

### Example 2: Production of human anti-C1q antibodies

Fully-human antibody clones that bind to human C1q were identified via screening library. The antibody screening process focused on the identification of anti-C1q antibodies from a fully human IgG library.

A total of 111 unique C1q binders were identified. Two binders were further characterized.

A known C1q-binding antibody was used as a control in the Examples (*e.g.,* Examples 3 to 7). The control antibody comprised murine heavy and light chain variable regions of the anti-C1q antibody "M1" (Annexon Biosciences). The control antibody comprises hIgG1 and kappa constant regions, and was expressed as a chimeric antibody. The anti-C1q antibody "M1", is described, for example, in US 2021/0277096 A1. This known C1q-binding antibody is referred to in the Examples herein, for example, as a "control antibody", "anti-C1q control IgG", "anti-C1q competitor IgG", and/or "Competitor IgG".

### Example 3: Inhibition of macrophage efferocytosis

The inventors investigated the ability of two antibodies identified according to Example 2 to inhibit engulfment (*i.e.* efferocytosis) of apoptotic Jurkat cells by human monocyte-derived macrophages (HMDMs), in the presence of C1q.

HMDMs were generated by culturing peripheral blood mononuclear cells (PBMCs) obtained from healthy donor subjects in the presence of human monocyte colony stimulating factor (M-CSF), at a concentration of 25 ng/ml. Wells of 96-well, flat-bottomed, black plates were coated with 30 nM C1q in PBS (50 µl volume) for 2 h at room temperature. After washing once with PBS, the two antibodies identified according to Example 2, anti-C1q control IgG, and an isotype-matched control antibody (isotype IgG) were added to the coated plates at a concentration of 100 nM in full RPMI (RPMI 1640 medium, supplemented with 10%FBS, 2mM L-glutamine and 1% Pen-strep). After 1 h incubation at 37°C, CMFDA-labeled HMDMs (50 µl volume, 0.2 x 10⁶ cells/mL) were introduced to the wells. The cells were then incubated for 48 h at 37°C. Subsequently, apoptotic Jurkat cells labeled with pHrodo dye (50 µl volume, 0.5 x 10⁶ cells/mL) were added to the wells. The apoptotic Jurkat cells had been generated via treatment for 16 h with camptothecin, at a concentration of 5 µM.

Engulfment of the apoptotic Jurkat cells by the HMDMs was subsequently analyzed using the Incucyte system, by measuring pHrodo fluorescence. This dye is not fluorescent outside cells at neutral pH, but fluoresces brightly in acidic, intracellular environments such in the endosomes and lysosomes. Engulfment was calculated as the total red fluorescence integrated intensity per well, normalized to the number of HMDMs (positive fluorescent signal).

The two antibodies identified according to Example 2 were both found to inhibit efferocytosis (by >95% and 40%, respectively). The anti-C1q control IgG was found not to inhibit efferocytosis.

### Example 4: Inhibition of macrophage polarization

The inventors next investigated the ability of two antibodies identified according to Example 2 to inhibit C1q-induced polarization of macrophages to an M2/M2-like phenotype. The level of IL-10 secreted in the cell culture medium was evaluated as a correlate of an M2/M2-like phenotype.

HMDMs were generated as described in Example 3 above. Wells of 96-well, flat-bottomed, transparent plates were coated with 30 nM C1q in PBS (50 µl volume) for 2 h at room temperature. After washing once with PBS, the two antibodies identified according to Example 2, anti-C1q control IgG, and an isotype-matched control antibody (isotype IgG) were added to the coated plates at a concentration of 100 nM in full RPMI (RPMI 1640 medium, supplemented with 10%FBS, 2mM L-glutamine and 1% Pen-strep). After 1 h incubation at 37°C, HMDMs (50µl volume, 0.2 x 10⁶ cells/mL) were introduced to the wells. The cells were then cultured for 48 h at 37°C. To stimulate cytokine secretion, LPS (10 µl volume, final concentration of 100 ng/mL) was added to the wells. After 24 hours, cell culture supernatants were collected for subsequent analysis of the level of IL-10 by ELISA (using the DuoSet IL-10 ELISA kit from R&D Systems, in accordance with the manufacturer's instructions, and using the Spectramax i3x plate reader). The number of HMDMs per well was determined by capturing images using the Incucyte system. Calculated levels of IL-10 levels (pg/ml) were normalized based on the number of HMDMs per well.

The two antibodies identified according to Example 2 were both found to inhibit IL-10 secretion by HMDMs (by >95% and 50%, respectively). The anti-C1q control IgG was found not to inhibit IL-10 secretion.

### Example 5: Inhibition of complement activity

The inventors next investigated the ability of two antibodies identified according to Example 2 to inhibit C1q-mediated activation of the complement cascade in human serum.

Activation of the complement cascade was measured via ELISA-based analysis of the level of the C5b-9 terminal complement complex in sera obtained from healthy human subjects. Wells of 96-well plates were blocked with 200 µl/well of 1% PBS-BSA, and incubated at room temperature for 2 h with gentle shaking at 350 rpm. Normal human serum (Complement Tech, Cat. NHS), stored at -80°C, was thawed and activated using by adding 1µl of CaCl₂ (0.3M) and 1µl of MgCl₂ (1M) solutions per 100µL of NHS for 5 minutes at room temperature. The activated serum was then diluted 1:100 in GVB++ buffer (2x serum solution). The two antibodies identified according to Example 2, anti-C1q control IgG and an isotype-matched control antibody (isotype IgG) were separately diluted in GVB++ buffer to a concentration of 200 nM (double the final concentration at which they were to be evaluated in the assay; 2x antibody solution). The serum and antibody solutions were then added to the wells of the assay plate in duplicate, and wells were incubated for 1 h at 37°C. A HRP-conjugated anti-C5b-9 antibody (Novus Biologicals, Cat. NBP1-0520H) added to the wells at a dilution of 1:10000 in DPBS (ThermoFisher Scientific, Cat. 14190094), and plates were incubated at room temperature for 30 min. After three washes with 300µL of DPBS+Tween0.05% , plates were developed by addition of TMB substrate solution, and incubation for 10 min at RT. Development was stopped using 100µl of the ELISA Stop solution (ThermoFisher Scientific, Cat. SS04). Finally, absorbance readings were taken at λ= 450 nm using the SpectraMax^{®} i3x spectrometer from Molecular Devices. OD values were used to calculate percentage inhibition relative to signal obtained with isotype IgG.

One antibody which was identified through the method of Example 2 and the anti-C1q control IgG were found to completely suppress activation of the complement cascade by C1q. By contrast, the other antibody identified according to Example 2 was found not to inhibit complement activation.

### Example 6: Inhibition of binding of C1q to activated endothelial cells

The inventors next investigated the ability of two antibodies identified according to Example 2 to inhibit binding of C1q to activated endothelial cells.

Briefly, 96-well cell culture plates were coated with 0.1% gelatin and incubated for 15 min. After washing with PBS, HUVECs were added to the wells (10,000 cells/well, in 100 µL volume in full EGM2 medium (Lonza, Cat. CC-3162). The cells were allowed to settle for 15 min at room temperature. Plates were then incubated at 37°C with 5% CO₂ for 24 h. On Day 2, 50 µL of EGM2 supplemented with TNFα (to a final achieved concentration of 10 ng/mL) was added to the wells to induce endothelial cell activation. After 24 h incubation at 37°C with 5% CO₂, the cell culture medium was removed, and 100 µL of basal EBM2 (endothelial basal medium, without serum or growth factors) was added to the wells and incubated for 10 minutes at room temperature.

Then, 50 µL of C1q in EBM was added (to a final concentration of 50 nM), in the presence or absence of two antibodies identified according to Example 2, anti-C1q control IgG or an isotype-matched control antibody (isotype IgG), to a final concentration of 100 nM. The plates were then incubated at 37°C for 1 h, and then the cell culture medium was removed, and cells were washed once with EBM2 and fixed with 4% PFA in PBS for 10 min at room temperature. Following blocking with 1% BSA in PBS for 1 h, a biotinylated polyclonal anti-C1q antibody was added as a biotinylated rabbit polyclonal C1q detection antibody (Abcam, ab48341) was added in PBS-1%FBS (dilution 1/1000) and incubated for 1 h at 37°C. After washing twice with 100 µl PBS+0.05% Tween20, Pierce^{™} Streptavidin Poly-HRP (Thermo Fisher, Cat. 21140) was added (at 1:1000 dilution factor), followed by Tyramide-working solution.

Finally, cells were imaged using the Incucyte instrument (Phase/Green, 20X objective, 4 images/well). C1q binding calculated be measuring the total red fluorescence integrated intensity per well, normalized to the number of endothelial cells per well and then to values obtained for the isotype IgG conditions.

The two antibodies identified according to Example 2 and the anti-C1q control IgG were found to inhibit binding of C1q to activated endothelial cells. One antibody which was identified according to Example 2, and the anti-C1q control IgG, strongly inhibited binding of C1q to activated endothelial cells (displaying 100% and 94% inhibition, respectively) while the other antibody identified according to Example 2 displayed a -24% inhibition of binding of C1q to activated endothelial cells.

### Example 7: Inhibition of binding of C1q to cC1qR and LAIR1

The inventors next investigated the ability of two antibodies identified according to Example 2 to inhibit binding of C1q to its receptors cC1qR and LAIR1.

Recombinant human cC1qR (Sinobiological, cat. HPLC-13539-H08H) or LAIR1 (Bio Techne, cat. 2664-LR) proteins were diluted in PBS to a final concentration of 2.0 µg/mL, and 50 µL was added to wells of flat-bottomed, 96-well MaxiSorp plates and allowed to coat onto the surface of the well overnight at 4°C. Subsequently, the coating solution was removed and the wells were washed three times with 250 µL of PBS+0.05% Tween20, using a microplate washer. To prevent nonspecific binding, plate blocking was performed by adding 250 µL/well of PBS+4% BSA, followed by a 2 h incubation at room temperature in an orbital shaker at 150 rpm.

Biotinylated human C1q (0.5 nM) was premixed with two antibodies identified according to Example 2, anti-C1q control IgG, or an isotype-matched control antibody (isotype IgG), to a final concentration of 100 nM, and incubated for 1 h at room temperature on an orbital shaker at 150 rpm. The solution containing biotinylated human C1q and antibodies was then added to the wells of the 96 well plate (after the blocking solution had been removed), and the plates were incubated for 1 h at room temperature on the orbital shaker at 150 rpm.

Wells were then washed with 250 µL of PBS+0.05% Tween20, using a microplate washer. Streptavidin solution comprising a streptavidin-HRP (Abcam, cat. Ab7403) dilution ratio of 1/50000 in PBS-1%BSA was then added to wells at a volume of 100 µL/well, and plates were incubated for an hour at room temperature on the orbital shaker at 150 rpm. After a further wash with 250 µL of PBS+0.05% Tween20, the HRP-reaction was induced by addition of 100 µL/well of TMB, and the wells were incubated at room temperature until the solution turned blue. The reaction was stopped by adding 100 µL/well of ELISA Stop Solution. Finally, absorbance readings were taken at λ= 450 nm using the SpectraMax^{®} i3x spectrometer from Molecular Devices. OD values were used to calculate percentage inhibition relative to signal obtained with isotype IgG.

The two antibodies identified according to Example 2 and the anti-C1q control IgG were found to strongly inhibit interaction between C1q and LAIR1. While one antibody identified according to Example 2 and the anti-C1q control IgG were found to strongly inhibit interaction between C1q and cC1qR, the other antibody identified according to Example 2 only weakly inhibited interaction between C1q and cC1qR.

## Claims

1. An antigen-binding molecule that binds to C1q for treating or preventing a disease in which M2 macrophages and/or M2-like macrophages are pathologically-implicated.

2. The antigen-binding molecule for use according to claim 1, wherein the method of treating or preventing a disease in which M2 macrophages and/or M2-like macrophages are pathologically-implicated comprises inhibiting polarization of macrophages to an M2 or M2-like phenotype.

3. The antigen-binding molecule for use according to claim 1 or claim 2, wherein the disease in which M2 macrophages and/or M2-like macrophages are pathologically-implicated is selected from: a cancer, fibrosis, a fibrotic disease, pulmonary fibrosis, liver fibrosis, systemic sclerosis, an inflammatory disease of the airways/lungs, asthma, chronic obstructive pulmonary disease, an allergic condition, allergic asthma, allergic rhinitis, allergic conjunctivitis, atopic dermatitis, food allergy, a neurological disease, a neurological disease in which synaptic pruning is pathologically-implicated, Alzheimer's disease, schizophrenia, Rett syndrome and multiple sclerosis.

4. The antigen-binding molecule for use according to any one of claims 1 to 3, wherein the antigen-binding molecule inhibits polarization of macrophages to an M2 or M2-like phenotype.

5. The antigen-binding molecule for use according to any one of claims 1 to 4, wherein the antigen-binding molecule inhibits IL-10 production by macrophages.

6. The antigen-binding molecule for use according to any one of claims 1 to 5, wherein the antigen-binding molecule inhibits macrophage efferocytosis.

7. The antigen-binding molecule for use according to any one of claims 1 to 6, wherein the antigen-binding molecule does not substantially inhibit activation of the complement cascade.

8. An in vitro complex, optionally isolated, comprising an antigen-binding molecule bound to C1q, wherein the C1q is present on a macrophage cell.

9. A method for detecting C1q in a sample, comprising contacting a sample with an antigen-binding molecule that binds to C1q, and detecting the formation of a complex of the antigen-binding molecule with C1q, wherein the sample contains a macrophage, or is suspected to contain a macrophage.

10. Use of an antigen-binding molecule that binds to C1q as an *in vitro* diagnostic or prognostic agent, for the diagnosis or prognosis of a disease in which M2 macrophages and/or M2-like macrophages are pathologically-implicated.
